# EUROPEAN PATENT APPLICATION

(11) **EP 2 905 279 A1**
(43) Date of publication of application: **12.08.2015**
(21) Application number: 13844175.3
(22) Date of filing: 01.10.2013
(51) Int. Cl.: C07D 233/64, A61K 31/4164, A61K 31/4439, A61K 31/454, A61K 31/506, A61P 5/00, A61P 21/00, A61P 25/00, A61P 25/04, A61P 25/28, A61P 25/30, A61P 43/00, C07D 401/04, C07D 401/14, C07D 405/12, C07D 405/14, C07D 409/14

(54) **IMIDAZOLE DERIVATIVE**

(30) Priority: 02.10.2012 JP 2012219934
(71) Applicant: Sumitomo Dainippon Pharma Co., Ltd., Osaka-shi Osaka 541-8524 (JP)
(72) Inventor: SUETSUGU, Satoshi, Osaka-shi, Osaka 554-0022 (JP); SAWAYAMA, Yusuke, Osaka-shi, Osaka 554-0022 (JP); NAKAI, Yoko, Osaka-shi, Osaka 554-0022 (JP); SAWAYAMA, Yusuke, Osaka-shi, Osaka 554-0022 (JP)
(74) Representative: Webster, Jeremy Mark
(86) International application number: PCT/JP2013/076694
(87) International publication number: WO 2014/054635

(57) **Abstract**

Provided is a therapeutic agent for diseases associated with a cholinergic property of the central nervous system (CNS) and / or the peripheral nervous system (PNS), diseases associated with the contraction of smooth muscle, incretion disease, diseases associated with neuronal degeneration and the like, said therapeutic agent comprising a compound represented by Formula (I): 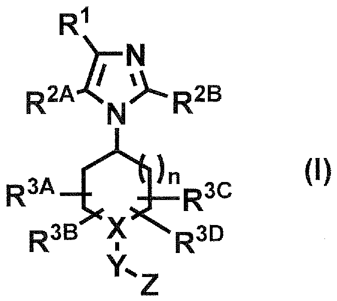 [wherein X-Y-Z represents N-CO-NR^{4A}R^{4B} or the like; R¹ represents a phenyl group or the like; R^{2A} and R^{2B} may be the same as or different from each other and independently represent a hydrogen atom or the like; R^{3A} to R^{3D} and R⁶ may be the same as or different from one another and independently represent a hydrogen atom or the like; R^{4A} and R^{4B} may be the same as or different from each other and independently represent an aryl group or the like; and n represents 1 or 2]
or a pharmaceutically acceptable salt thereof and having a potent activity of regulating an α7 nicotinic acetylcholine receptor (an α7 nAChR).

## Description

### TECHNICAL FIELD

The present invention relates to a novel imidazole derivative which is a modulator of α7 nicotinic acetylcholine receptor (α7 nAChR). On the basis of such pharmacological properties, the present compound can be useful for treating, for example, diseases related to cholinergic properties in the central nervous system (CNS) and/or peripheral nervous system (PNS), diseases associated with smooth muscle contraction, endocrine disorders, neurodegenerative disorders, diseases such as inflammation and pain, and diseases associated with withdrawal symptoms caused by addictive drug abuse.

### BACKGROUND ART

Recently, potential neuroprotective-effects of nicotine have been shown, and meanwhile various neurodegenerative-models in animals and cultured cells suffering from excitotoxic injury, athrepsia, ischemia, injury, neuronal cell death induced by amyloid beta (Aβ) or neurodegeneration induced by protein aggregation have been proposed. In many cases where nicotine shows neuroprotective effects, it has been found that nicotinic acetylcholine receptors containing α7 subtype are activated. These findings suggest that nicotine is useful in providing neuroprotective effects, and indicate that receptors containing α7-subtype are directly related with the effects. These data suggest that α7 nicotinic acetylcholine receptor is typically a suitable molecular-target for neuroprotection. In other words, the neuroprotection may be accomplished by developing an active agonist/positive modulator (i.e. positive allosteric modulator: PAM) of the receptor. In fact, α7 nicotinic acetylcholine receptor agonist has already been identified, and is expected to provide a possible clue to the development of neuroprotective drugs. In addition, it has recently been reported that α7 nicotinic acetylcholine receptor is also involved in inflammation. Thus, the development of a novel modulator of the receptor is expected to lead to a novel treatment for nervous system diseases, psychiatric diseases and inflammatory diseases.

In the past, there were some disclosures about modulators of α7 nicotinic acetylcholine receptor (α7 nAChR), but the chemical structures thereof are different from that of the present compound (see, Patent Reference 1 and Patent Reference 2).

### CONVENTIONAL ART REFERENCES

### PATENT REFERENCES

[Patent reference 1] WO 2003/093250
[Patent reference 2] WO 2006/138510

### SUMMARY OF INVENTION

### PROBLEMS TO BE SOLVED BY THE INVENTION

A problem to be solved by the present invention is to provide a novel compound which has potent modulatory-effects on the activity of α7 nicotinic acetylcholine receptor (α7 nAChR), and can be useful as a novel medicament for treating and/or preventing nervous system diseases, psychiatric diseases and inflammatory diseases.

In addition, WO 2012/133509 and WO 2012/176763 are applications related to the present application, which have already been published. The compounds therein have similar but different structures from that of the present compound. Further, the priority date of the present application is earlier than the published dates of the related applications, and they are not thus conventional art references for the present application.

### MEANS OF SOLVING THE PROBLEMS

The present inventors have extensively studied to solve the problem and then have found that a novel compound of the following Formula (I) exhibits potent modulatory-effects on the activity of α7 nicotinic acetylcholine receptor (α7 nAChR). On the basis of the new findings, the present invention has been completed. The present invention provides an imidazole derivative of the following Formula (I) or a pharmaceutically acceptable salt thereof (hereinafter, optionally referred to as "the present compound").
[Item 1] A compound of Formula (I): 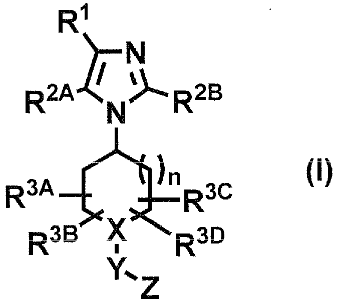
   wherein X-Y-Z is N-CO-NR^{4A}R^{4B}, N-COR⁵, CR⁶-CO-NR^{4A}R^{4B}, CR⁶-NR⁷-COR⁵, CR⁶-NR⁷-CONR^{4A}R^{4B} or CR⁶-NR⁷-Q;
   R¹ is phenyl or heteroaryl in which the phenyl and the heteroaryl may be each optionally substituted with 1 to 5 substituents independently selected from the group consisting of halogen, hydroxyl group, C₁₋₆ alkyl which may be optionally substituted with 1 to 5 fluorine atoms, C₁₋₆ alkoxy which may be optionally substituted with 1 to 5 fluorine atoms, cyano, - NR⁸R⁹, -COOR⁸, -CONR⁸R⁹ and -NR⁸COR⁹,
   R^{2A} and R^{2B} are the same or different and are hydrogen atom; halogen; cyano; - COOR¹⁰; -CONR¹⁰R¹¹; -NR¹⁰R¹¹; -NR¹⁰COR¹¹; C₁₋₆ alkyl which may be optionally substituted with 1 to 5 substituents independently selected from the group consisting of halogen, hydroxyl group, C₁₋₆ alkyl, C₃₋₁₀ cycloalkyl which may be optionally substituted with 1 to 5 fluorine atoms, C₁₋₆ alkoxy, 4- to 10-membered saturated heterocycle, cyano, -NR¹⁰R¹¹, -COOR¹⁰,-CONR¹⁰R¹¹ and -NR¹⁰COR¹¹; or C₃₋₁₀ cycloalkyl which may be optionally substituted with 1 to 5 substituents independently selected from the group consisting of halogen, hydroxyl group, C₁₋₆ alkyl, C₁₋₆ alkoxy, cyano, -NR¹⁰R¹¹, -COOR¹⁰, -CONR¹⁰R¹¹ and -NR¹⁰COR¹¹; provided that when X-Y-Z is N-CO-NHEt and n = 1, R^{2A} is C₁₋₄ alkyl which may be optionally substituted with the above substituents,
   R^{3A}, R^{3B}, R^{3C}, R^{3D} and R⁶ are the same or different and are hydrogen atom; fluorine atom; hydroxyl group; C₁₋₆ alkoxy which may be optionally substituted with 1 to 5 fluorine atoms; or C₁₋₆ alkyl which may be optionally substituted with 1 to 5 fluorine atoms; provided that when any two of R^{3A}, R^{3B}, R^{3C}, R^{3D} and R⁶ are independently selected from C₁₋₆ alkyl which may be optionally substituted with 1 to 5 fluorine atoms, the two alkyl groups may be combined each other together with the ring to which the alkyl groups attach to form another ring,
   R^{4A}, R^{4B}, R⁵ and R⁷ are the same or different and are C₁₋₆ alkyl which may be optionally substituted with 1 to 5 substituents independently selected from the group consisting of aryl or heteroaryl (in which the aryl and the heteroaryl may be optionally substituted with 1 to 5 substituents independently selected from the group consisting of halogen, hydroxyl group, C₁₋₆ alkyl which may be optionally substituted with 1 to 5 fluorine atoms, and C₁₋₆ alkoxy which may be optionally substituted with 1 to 5 fluorine atoms), halogen, hydroxyl group, C₁₋₆ alkoxy, 4- to 10-membered saturated heterocycle, C₃₋₁₀ cycloalkyl and -NR¹²R¹³; C₃₋₁₀ cycloalkyl which may be optionally substituted with 1 to 5 substituents independently selected from the group consisting of aryl or heteroaryl (in which the aryl and the heteroaryl may be optionally substituted with 1 to 5 substituents independently selected from the group consisting of halogen, hydroxyl group, C₁₋₆ alkyl which may be optionally substituted with 1 to 5 fluorine atoms, and C₁₋₆ alkoxy which may be optionally substituted with 1 to 5 fluorine atoms), halogen, hydroxyl group, C₁₋₆ alkoxy, C₁₋₆ alkyl and -NR¹²R¹³; 4- to 10-membered saturated heterocycle which may be optionally substituted with C₁₋₆ alkyl; aryl or heteroaryl in which the aryl and the heteroaryl may be each optionally substituted with 1 to 5 substituents independently selected from the group consisting of halogen, C₁₋₆ alkyl which may be optionally substituted with 1 to 5 fluorine atoms, and C₁₋₆ alkoxy which may be optionally substituted with 1 to 5 fluorine atoms; or hydrogen atom; provided that R⁵ is not hydrogen atom, and R^{4A} and R^{4B} are not concurrently hydrogen atom, and that when both R^{4A} and R^{4B} are independently selected from C₁₋₆ alkyl, they may be combined each other to form 4- to 10-membered nitrogen-containing saturated heterocycle which may be optionally substituted with 1 to 5 substituents independently selected from the group consisting of fluorine atom, C₁₋₆ alkyl and C₁₋₆ alkoxy,
   Q is 6-membered heteroaryl containing one or two nitrogen atoms [in which the heteroaryl may be optionally substituted with 1 to 3 substituents independently selected from the group consisting of C₁₋₆ alkyl which may be optionally substituted with 1 to 5 substituents independently selected from the group consisting of fluorine atom, hydroxyl group, C₁₋₆ alkoxy, C₃₋₆ cycloalkyl, -NR¹⁰R¹¹, -CONR¹⁰R¹¹ and -NR¹⁰COR¹¹; C₃₋₁₀ cycloalkyl, C₃₋₁₀ cycloalkoxy or 4- to 10-membered saturated heterocycle (in which the cycloalkyl, the cycloalkoxy and the saturated heterocycle may be each optionally substituted with 1 to 5 substituents independently selected from the group consisting of fluorine atom, hydroxyl group, C₁₋₆ alkyl, C₁₋₆ alkoxy, - NR¹⁴R¹⁵, -CONR¹⁴R¹⁵ and -NR¹⁴COR¹⁵); C₁₋₆ alkoxy which may be optionally substituted with 1 to 5 substituents independently selected from the group consisting of fluorine atom, hydroxyl group, C₁₋₆ alkoxy, -NR¹⁴R¹⁵, -CONR¹⁴R¹⁵ and -NR¹⁴COR¹⁵; halogen; cyano; -CONR¹⁴R¹⁵; - NR¹⁴COR¹⁵ ; and -NR¹⁴R¹⁵],
   R⁸ to R¹⁵ are the same or different, and independent each other when the same substituent symbol exists plurally, and are hydrogen atom, or C₁₋₆ alkyl which may be optionally substituted with 1 to 5 fluorine atoms; provided that in each combination of R⁸ and R⁹, R¹⁰ and R¹¹, R¹² and R¹³, or R¹⁴ and R¹⁵, (1) when one is hydrogen atom, the other is not hydrogen atom, and (2) when both of them are independently selected from C₁₋₆ alkyl, they may be combined each other to form 4- to 10-membered nitrogen-containing saturated heterocycle, and
   n is 1 or 2,
   or a pharmaceutically acceptable salt thereof.
[Item 2] A compound of Formula (I): 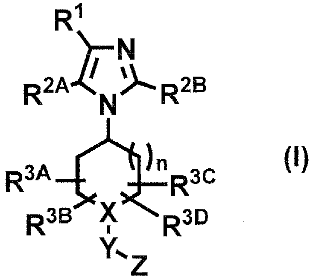
   wherein X-Y-Z is N-CO-NR^{4A}R^{4B}, N-COR⁵, CR⁶-CO-NR^{4A}R^{4B}, CR⁶-NR⁷-COR⁵, CR⁶-NR⁷-CONR^{4A}R^{4B} or CR⁶-NR⁷-Q,
   R¹ is phenyl or monocyclic heteroaryl in which the phenyl and the monocyclic heteroaryl may be each optionally substituted with 1 to 5 substituents independently selected from the group consisting of halogen, hydroxyl group, C₁₋₆ alkyl which may be optionally substituted with 1 to 5 fluorine atoms, C₁₋₆ alkoxy which may be optionally substituted with 1 to 5 fluorine atoms, cyano, -NR⁸R⁹, -COOR⁸, -CONR⁸R⁹ and -NR⁸COR⁹,
   R^{2A} and R^{2B} are the same or different and are hydrogen atom; halogen; cyano; - COOR¹⁰; -CONR¹⁰R¹¹; -NR¹⁰R¹¹; -NR¹⁰COR¹¹; C₁₋₆ alkyl which may be optionally substituted with 1 to 5 substituents independently selected from the group consisting of halogen, hydroxyl group, C₁₋₆ alkyl, C₃₋₁₀ cycloalkyl, which may be optionally substituted with 1 to 5 fluorine atoms, C₁₋₆ alkoxy, 4- to 10-membered saturated heterocycle, cyano, -NR¹⁰R¹¹, -COOR¹⁰, - CONR¹⁰R¹¹, and -NR¹⁰COR¹¹; or C₃₋₁₀ cycloalkyl which may be optionally substituted with 1 to 5 substituents independently selected from the group consisting of halogen, hydroxyl group, C₁₋₆ alkyl, C₁₋₆ alkoxy, cyano, -NR¹⁰R¹¹, -COOR¹⁰, -CONR¹⁰R¹¹ and -NR¹⁰COR¹¹; provided that when X-Y-Z is N-CO-NHEt and n = 1, R^{2A} is hydrogen atom; halogen; cyano: or C₁₋₄ alkyl which may be optionally substituted with the above substituents,
   R^{3A}, R^{3B}, R^{3C}, R^{3D} and R⁶ are the same or different and are hydrogen atom; fluorine atom; hydroxyl group; C₁₋₆ alkoxy which may be optionally substituted with 1 to 5 fluorine atoms; or C₁₋₆ alkyl which may be optionally substituted with 1 to 5 fluorine atoms; provided that when any two of R^{3A}, R^{3B}, R^{3C}, R^{3D} and R⁶ are independently selected from C₁₋₆ alkyl which may be optionally substituted with 1 to 5 fluorine atoms, the two alkyl groups may be combined each other together with the ring to which the alkyl groups attach to form another ring,
   R^{4A}, R^{4B}, R⁵ and R⁷ are the same or different and are C₁₋₆ alkyl which may be optionally substituted with 1 to 5 substituents independently selected from the group consisting of aryl or heteroaryl (in which the aryl and the heteroaryl may be optionally substituted with 1 to 5 substituents independently selected from the group consisting of halogen, hydroxyl group, C₁₋₆ alkyl which may be optionally substituted with 1 to 5 fluorine atoms, and C₁₋₆ alkoxy which may be optionally substituted with 1 to 5 fluorine atoms), halogen, hydroxyl group, C₁₋₆ alkoxy, 4- to 10-membered saturated heterocycle, C₃₋₁₀ cycloalkyl, and -NR¹²R¹³; C₃₋₁₀ cycloalkyl which may be optionally substituted with 1 to 5 substituents independently selected from the group consisting of aryl or heteroaryl (in which the aryl and the heteroaryl may be optionally substituted with 1 to 5 substituents independently selected from the group consisting of halogen, hydroxyl group, C₁₋₆ alkyl which may be optionally substituted with 1 to 5 fluorine atoms, and C₁₋₆ alkoxy which may be optionally substituted with 1 to 5 fluorine atoms), halogen, hydroxyl group, C₁₋₆ alkoxy, C₁₋₆ alkyl, and -NR¹²R¹³; 4 to 10-membered saturated heterocycle which may be optionally substituted with C₁₋₆ alkyl; aryl or heteroaryl in which the aryl and the heteroaryl may be each optionally substituted with 1 to 5 substituents independently selected from the group consisting of halogen, C₁₋₆ alkyl which may be optionally substituted with 1 to 5 fluorine atoms, and C₁₋₆ alkoxy which may be optionally substituted with 1 to 5 fluorine atoms; or hydrogen atom; provided that R⁵ is not hydrogen atom and R^{4A} and R^{4B} are not concurrently hydrogen atom, and that when both R^{4A} and R^{4B} are independently selected from C₁₋₆ alkyl, they may be combined each other to form 4- to 10-membered nitrogen-containing saturated heterocycle which may be optionally substituted with 1 to 5 substituents independently selected from the group consisting of fluorine atom, C₁₋₆ alkyl and C₁₋₆ alkoxy,
   Q is 6-membered heteroaryl containing one or two nitrogen atoms [in which the heteroaryl may be optionally substituted with 1 to 3 substituents independently selected from the group consisting of C₁₋₆ alkyl which may be optionally substituted with 1 to 5 substituents independently selected from the group consisting of fluorine atom, hydroxyl group, C₁₋₆ alkoxy, C₃₋₆ cycloalkyl, -NR¹⁰R¹¹, -CONR¹⁰R¹¹ and -NR¹⁰COR¹¹; C₃₋₁₀ cycloalkyl, C₃₋₁₀ cycloalkoxy or 4- to 10-membered saturated heterocycle (in which the cycloalkyl, the cycloalkoxy and the saturated heterocycle may be each optionally substituted with 1 to 5 substituents independently selected from the group consisting of fluorine atom, hydroxyl group, C₁₋₆ alkyl, C₁₋₆ alkoxy, - NR¹⁴R¹⁵, -CONR¹⁴R¹⁵ and -NR¹⁴COR¹⁵); C₁₋₆ alkoxy which may be optionally substituted with 1 to 5 substituents independently selected from the group consisting of fluorine atom, hydroxyl group, C₁₋₆ alkoxy, -NR¹⁴R¹⁵, -CONR¹⁴R¹⁵ and -NR¹⁴COR¹⁵; halogen; cyano; -CONR¹⁴R¹⁵; - NR¹⁴COR¹⁵; and -NR¹⁴R¹⁵],
   R⁸ to R¹⁵ are the same or different, and independent each other when the same substituent symbol exists plurally, and are hydrogen atom, or C₁₋₆ alkyl which may be optionally substituted with 1 to 5 fluorine atoms; provided that in each combination of R⁸ and R⁹, R¹⁰ and R¹¹, R¹² and R¹³, or R¹⁴ and R¹⁵, (1) when one is hydrogen atom, the other is not hydrogen atom, and (2) when both of them are independently selected from C₁₋₆ alkyl, they may be combined each other to form 4- to 10-membered nitrogen-containing saturated heterocycle, and
   n is 1 or 2,
   or a pharmaceutically acceptable salt thereof.
[Item 3] The compound of Item 1 or 2 wherein n is 1, or a pharmaceutically acceptable salt thereof.
[Item 4] The compound of any one of Items 1 to 3 wherein X-Y-Z is N-CO-NR^{4A}R^{4B}, N-COR⁵, CR⁶-CO-NR^{4A}R^{4R} or CR⁶-NR⁷-COR⁵, or a pharmaceutically acceptable salt thereof.
[Item 5] The compound of any one of Items 1 to 4 wherein R¹ is phenyl or monocyclic heteroaryl in which the phenyl and the monocyclic heteroaryl may be each optionally substituted with 1 to 5 substituents independently selected from the group consisting of halogen, hydroxyl group, C₁₋₆ alkyl which may be optionally substituted with 1 to 5 fluorine atoms, C₁₋₆ alkoxy which may be optionally substituted with 1 to 5 fluorine atoms, and cyano, or a pharmaceutically acceptable salt thereof.
[Item 6] The compound of any one of Items 1 to 4 wherein R¹ is phenyl which may be optionally substituted with 1 to 5 substituents independently selected from the group consisting of halogen, hydroxyl group, C₁₋₆ alkyl which may be optionally substituted with 1 to 5 fluorine atoms, C₁₋₆ alkoxy which may be optionally substituted with 1 to 5 fluorine atoms, cyano, - NR⁸R⁹, -COOR⁸, -CONR⁸R⁹, and -NR⁸COR⁹, or a pharmaceutically acceptable salt thereof.
[Item 7] The compound of any one of Items 1 to 4 wherein R¹ is phenyl which may be optionally substituted with 1 to 5 substituents independently selected from the group consisting of halogen, C₁₋₆ alkyl which may be optionally substituted with 1 to 5 fluorine atoms, and C₁₋₆ alkoxy which may be optionally substituted with 1 to 5 fluorine atoms, or a pharmaceutically acceptable salt thereof.
[Item 8] The compound of any one of Items 1 to 7 wherein R^{2A} and R^{2B} are the same or different and are hydrogen atom; halogen; cyano; C₁₋₆ alkyl which may be optionally substituted with 1 to 5 substituents independently selected from the group consisting of halogen, hydroxyl group, C₁₋₆ alkyl, C₃₋₁₀ cycloalkyl which may be optionally substituted with 1 to 5 fluorine atoms, C₁₋₆ alkoxy, 4- to 10-membered saturated heterocycle, cyano, -NR¹⁰R¹¹,-COOR¹⁰, -CONR¹⁰R¹¹, and -NR¹⁰COR¹¹; or C₃₋₁₀ cycloalkyl which may be optionally substituted with 1 to 5 substituents independently selected from the group consisting of halogen, hydroxyl group, C₁₋₆ alkyl, C₁₋₆ alkoxy, cyano, -NR¹⁰R¹¹, -COOR¹⁰, -CONR¹⁰R¹¹ and -NR¹⁰COR¹¹; provided that when X-Y-Z is N-CO-NHEt and n = 1, R^{2A} is hydrogen atom, halogen, cyano, or C₁₋₄ alkyl which may be optionally substituted with the above substituents, or a pharmaceutically acceptable salt thereof.
[Item 9] The compound of any one of Items 1 to 7 wherein R^{2A} and R^{2B} are the same or different and are hydrogen atom; halogen; cyano; or C₁₋₆ alkyl which may be optionally substituted with 1 to 5 substituents independently selected from the group consisting of halogen, hydroxyl group, C₁₋₆ alkyl, C₃₋₁₀ cycloalkyl which may be optionally substituted with 1 to 5 fluorine atoms, C₁₋₆ alkoxy and 4- to 10-membered saturated heterocycle; provided that when X-Y-Z is N-CO-NHEt and n = 1 R^{2A} is hydrogen atom, halogen, cyano, or C₁₋₄ alkyl which may be optionally substituted with the above substituents, or a pharmaceutically acceptable salt thereof.
[Item 10] The compound of any one of Items 1 to 7 wherein R^{2A} and R^{2B} are the same or different and are hydrogen atom, halogen or cyano, or a pharmaceutically acceptable salt thereof.
[Item 11] The compound of any one of Items 1 to 7 wherein R^{2A} is halogen or cyano, and R^{2B} is hydrogen atom, or a pharmaceutically acceptable salt thereof.
[Item 12] The compound of any one of Items 1 to 10 wherein R^{2B} is hydrogen atom, or a pharmaceutically acceptable salt thereof.
[Item 13] The compound of any one of Items 1 to 12 wherein R^{3A}, R^{3B}, R^{3C}, R^{3D} and R⁶ are the same or different and are hydrogen atom, or C₁₋₆ alkyl; provided that when any two of R^{3A}, R^{3B}, R^{3C} and R^{3D} are independently selected from C₁₋₆ alkyl, the two alkyl groups may be combined each other together with the carbon atoms to which the alkyl groups attach or the ring containing the carbon atoms to form another ring, or a pharmaceutically acceptable salt thereof.
[Item 14] The compound of any one of Items 1 to 12 wherein all of R^{3A}, R^{3B}, R^{3C}, R^{3D} and R⁶ are hydrogen atom, or a pharmaceutically acceptable salt thereof.
[Item 15] The compound of any one of Items 1 to 14 wherein R^{4A}, R^{4B}, R⁵ and R⁷ are the same or different and are C₁₋₆ alkyl which may be optionally substituted with 1 to 5 substituents independently selected from the group consisting of aryl or heteroaryl (in which the aryl and the heteroaryl may be optionally substituted with 1 to 5 substituents independently selected from the group consisting of halogen, hydroxyl group, C₁₋₆ alkyl which may be optionally substituted with 1 to 5 fluorine atoms, and C₁₋₆ alkoxy which may be optionally substituted with 1 to 5 fluorine atoms), halogen, hydroxyl group, C₁₋₆ alkoxy, 4- to 10-membered saturated heterocycle, C₃₋₁₀ cycloalkyl and -NR¹²R¹³ ; C₃₋₁₀ cycloalkyl which may be optionally substituted with 1 to 5 substituents independently selected from the group consisting of aryl or heteroaryl (in which the aryl and the heteroaryl may be optionally substituted with 1 to 5 substituents independently selected from the group consisting of halogen, hydroxyl group, C₁₋₆ alkyl which may be optionally substituted with 1 to 5 fluorine atoms, and C₁₋₆ alkoxy which may be optionally substituted with 1 to 5 fluorine atoms), halogen, hydroxyl group, C₁₋₆ alkoxy, C₁₋₆ alkyl and -NR¹²R¹³; or hydrogen atom; provided that R⁵ is not hydrogen atom and both R^{4A} and R^{4B} are not concurrently hydrogen atom, and that when both R^{4A} and R^{4B} are independently selected from C₁₋₆ alkyl, they may be combined with each other to form 4- to 10-membered nitrogen-containing saturated heterocycle which may be optionally substituted with 1 to 5 substituents independently selected from the group consisting of fluorine atom, C₁₋₆ alkyl and C₁₋₆ alkoxy, or a pharmaceutically acceptable salt thereof.
[Item 16] The compound of any one of Items 1 to 14 wherein R^{4A}, R^{4B}, R⁵ and R⁷ are the same or different and are C₁₋₆ alkyl which may be optionally substituted with 1 to 5 substituents independently selected from the group consisting of halogen, hydroxyl group, C₁₋₆ alkoxy, 4- to 10-membered saturated heterocycle, C₃₋₁₀ cycloalkyl and -NR¹²R¹³; C₃₋₁₀ cycloalkyl which may be optionally substituted with 1 to 5 substituents independently selected from the group consisting of halogen, hydroxyl group, C₁₋₆ alkoxy, C₁₋₆ alkyl and -NR¹²R¹³; aryl or heteroaryl (in which the aryl and the heteroaryl may be each optionally substituted with 1 to 5 substituents independently selected from the group consisting of halogen, C₁₋₆ alkyl which may be optionally substituted with 1 to 5 fluorine atoms, and C₁₋₆ alkoxy which may be optionally substituted with 1 to 5 fluorine atoms); or hydrogen atom; provided that R⁵ is not hydrogen atom, and that when both R^{4A} and R^{4B} are independently selected from C₁₋₆ alkyl, they may be combined each other to form 4- to 10-membered nitrogen-containing saturated heterocycle which may be optionally substituted with 1 to 5 substituents independently selected from the group consisting of fluorine atom, C₁₋₆ alkyl and C₁₋₆ alkoxy, or a pharmaceutically acceptable salt thereof.
[Item 17] The compound of any one of Items 1 to 14 wherein R^{4A}, R^{4B}, R⁵ and R⁷ are the same or different and are C₁₋₆ alkyl, which may be optionally substituted with 1 to 5 substituents independently selected from the group consisting of halogen, hydroxyl group, C₁₋₆ alkoxy, 4- to 10-membered saturated heterocycle, C₃₋₁₀ cycloalkyl and -NR¹²R¹³; C₃₋₁₀ cycloalkyl which may be optionally substituted with 1 to 5 substituents independently selected from the group consisting of halogen, hydroxyl group, C₁₋₆ alkoxy, C₁₋₆ alkyl or -NR¹²R¹³; or hydrogen atom; provided that R⁵ is not hydrogen atom, or a pharmaceutically acceptable salt thereof.
[Item 18] The compound of any one of Items 1 to 17 wherein R^{4B} and R⁷ are hydrogen atom, or a pharmaceutically acceptable salt thereof.
[Item 19] The compound of any one of Items 1 to 18 wherein X-Y-Z is N-CO-NR^{4A}R^{4B}, or a pharmaceutically acceptable salt thereof.
[Item 20] The compound of any one of Items 1 to 18 wherein X-Y-Z is N-COR⁵, or a pharmaceutically acceptable salt thereof.
[Item 21] The compound of any one of Items 1 to 18 wherein X-Y-Z is CR⁶-CO-NR^{4A}R^{4B}, or a pharmaceutically acceptable salt thereof.
[Item 22] The compound of any one of Items 1 to 18 wherein X-Y-Z is CR⁶-NR⁷-COR⁵, or a pharmaceutically acceptable salt thereof.
[Item 23] The compound of any one of Items 1 to 18 wherein X-Y-Z is CR⁶-NR⁷-Q, or a pharmaceutically acceptable salt thereof.
[Item 24] The compound of any one of Items 1, 2, or 4 to 18, wherein X-Y-Z is CR⁶-NR⁷-Q, and Q is pyrimidinyl in which the pyrimidinyl may be optionally substituted with 1 to 3 substituents independently selected from the group consisting of halogen, C₁₋₆ alkyl which may be optionally substituted with 1 to 5 fluorine atoms, and C₁₋₆ alkoxy which may be optionally substituted with 1 to 5 fluorine atoms, or a pharmaceutically acceptable salt thereof.
[Item 25] The compound of Item 1 selected from the following compounds:
   N-cyclohexyl-4-[4-(3-fluorophenyl)-1H-imidazol-1-yl]piperidine-1-carboxamide (Example 1),
   1-{1-[(4,4-difluorocyclohexyl)carbonyl]piperidin-4-yl}-4-phenyl-1H-imidazole-5-carbonitrile (Example 7),
   N-cyclohexyl-4-[4-(2-fluorophenyl)-1H-imidazol-1-yl]piperidine-1-carboxamide (Example 66),
   4-{5-chloro-4-[3-(trifluoromethyl)phenyl]-1H-imidazol-1-yl}-N-(tetrahydro-2H-pyran-4-yl)piperidine-1-carboxamide (Example 94),
   N-(4,4-difluorocyclohexyl)-4-(5-methyl-4-phenyl-1H-imidazol-1-yl)piperidine-1-carboxamide (Example 105),
   1-{1-[(4,4-difluorocyclohexyl)carbonyl]piperidin-4-yl}-4-(4-fluorophenyl)-1H-imidazole-5-carbonitrile (Example 154),
   1-{1-[(4,4-difluorocyclohexyl)carbonyl]piperidin-4-yl}-4-(2-fluorophenyl)-1H-imidazole-5-carbonitrile (Example 156),
   N-{cis-4-[5-chloro-4-(4-fluorophenyl)-1H-imidazol-1-yl]cyclohexyl}-2-fluoro-2-methylpropanamide (Example 183),
   N-[cis-4-(5-cyano-4-phenyl-1H-imidazol-1-yl)cyclohexyl]-2-fluoro-2-methylpropanamide (Example 197),
   N-(cis-4-{ 5-cyano-4-[4-(trifluoromethyl)phenyl]-1H-imidazol-1-yl}cyclohexyl)-2-fluoro-2-methylpropanamide (Example 201),
   cis-4-{5-chloro-4-[4-(trifluoromethyl)phenyl]-1H-imidazol-1-yl}-N-(tetrahydro-2H-pyran-4-yl)cyclohexanecarboxamide (Example 224),
   N-{cis-4-[4-(4-chlorophenyl)-1H-imidazol-1-yl]cyclohexyl}-2-fluoro-2-methylpropanamide (Example 280),
   N-{cis-4-[4-(4-chloro-2-fluorophenyl)-1H-imidazol-1-yl]cyclohexyl}-2-fluoro-2-methylpropanamide (Example 283),
   N-{cis-4-[4-(2,4-difluorophenyl)-1H-imidazol-1-yl]cyclohexyl}-2-fluoro-2-methylpropanamide (Example 300),
   N-{cis-4-[5-cyano-4-(4-fluorophenyl)-1H-imidazol-1-yl]cyclohexyl}-2-fluoro-2-methylpropanamide (Example 332),
   cis-4-[5-chloro-4-(3,4-difluorophenyl)-1H-imidazol-1-yl]-N-(tetrahydro-2H-pyran-4-yl)cyclohexanecarboxamide (Example 370), and
   {(3-exo)-3-[5-chloro-4-(4-fluorophenyl)-1H-imidazol-1-yl]-8-azabicyclo[3.2.1]oct-8-yl}(4,4-difluorocyclohexyl)methanone (Example 452),
   or a pharmaceutically acceptable salt thereof.
[Item 26] The compound of Item 1 selected from the following compounds:
   N-cyclohexyl-4-[4-(3-fluorophenyl)-1H-imidazol-1-yl]piperidine-1-carboxamide (Example 1), 1-{1-[(4,4-difluorocyclohexyl)carbonyl]piperidin-4-yl}-4-phenyl-1H-imidazole-5-carbonitrile (Example 7),
   N-cyclohexyl-4-[4-(2-fluorophenyl)-1H-imidazol-1-yl]piperidine-1-carboxamide (Example 66), 1-{1-[(4,4-difluorocyclohexyl)carbonyl]piperidin-4-yl}-4-(4-fluorophenyl)-1H-imidazole-5-carbonitrile (Example 154),
   N-{cis-4-[5-chloro-4-(4-fluorophenyl)-1H-imidazol-1-yl]cyclohexyl}-2-fluoro-2-methylpropanamide (Example 183),
   cis-4-[5-chloro-4-(3,4-difluorophenyl)-1H-imidazol-1-yl]-N-(tetrahydro-2H-pyran-4-yl)cyclohexanecarboxamide (Example 370), and
   {(3-exo)-3-[5-chloro-4-(4-fluorophenyl)-1H-imidazol-1-yl]-8-azabicyclo[3.2.1]oct-8-yl}(4,4-difluorocyclohexyl)methanone (Example 452),
   or a pharmaceutically acceptable salt thereof.
[Item 27] A pharmaceutical composition comprising the compound of any one of Items 1 to 26 or a pharmaceutically acceptable salt thereof.
[Item 28] A medicament for treating a disease due to an abnormality of the intracellular signaling mediated by acetylcholine, comprising as an active ingredient the compound of any one of Items 1 to 26 or a pharmaceutically acceptable salt thereof.
[Item 29] The medicament of Item 28 wherein the disease due to an abnormality of the intracellular signaling mediated by acetylcholine is CIAS (cognitive impairment associated with schizophrenia), Alzheimer's disease, Down's syndrome, cognitive disorder, mild cognitive disorder, memory disorder / learning disorder, attention deficit / hyperactivity disorder or cerebral angiopathy.
[Item 30] A drug comprising the combination use of the compound of any one of Items 1 to 26 or a pharmaceutically acceptable salt thereof and at least one drug selected from atypical antipsychotics.
[Item 31] A method for treating a disease due to an abnormality of the intracellular signaling mediated by acetylcholine, comprising administering a therapeutically effective amount of the compound of any one of Items 1 to 26 or a pharmaceutically acceptable salt thereof to a patient in need thereof.
[Item 32] Use of the compound of any one of Items 1 to 26 or a pharmaceutically acceptable salt thereof in the manufacture of a medicament for treating a disease due to an abnormality of the intracellular signaling mediated by acetylcholine.
[Item 33] A pharmaceutical composition comprising the compound of any one of Items 1 to 26 or a pharmaceutically acceptable salt thereof for use in the treatment of a disease due to an abnormality of the intracellular signaling mediated by acetylcholine.

### EFFECT OF THE INVENTION

The present compound is useful as a novel medicament for treating nervous system disease, psychiatric disease, and inflammatory disease (e.g. senile dementia, attentional deficit disorder, Alzheimer's disease, and schizophrenia). The present compound is also useful as a combination drug with an atypical antipsychotic for treating nervous system disease, psychiatric disease such as schizophrenia.

### DESCRIPTION OF EMBODIMENTS

The present compound may exist in a form of hydrates and/or solvates, and thus such hydrates and/or solvates are also included in the present compound.

Since the compound of Formula (I) may contain one or possibly more asymmetric carbon atoms, or may have geometrical isomerism or an axial chirality, the compound may exist as several stereoisomers. Such stereoisomers, mixtures thereof, and racemates are also included in the present compound of Formula (I).

The compound of Formula (I) wherein one or more of ¹H are substituted with ²H (D) (i.e. its deuterated form) is also included in the present compound of Formula (I).

In the present invention, hydrates and solvates such as an ethanolate of the compound of Formula (I) or a pharmaceutically acceptable salt thereof are also included in the present compound of Formula (I).

The terms used herein are explained hereinafter.

The term "alkyl" refers to a straight or branched chain saturated hydrocarbon group, and for example, "C₁₋₄ alkyl" or "C₁₋₆ alkyl" refers to an alkyl wherein the number of the carbon atoms is 1 to 4, or 1 to 6, respectively. In the case where the alkyl is "C₁₋₄ alkyl", its specific example includes, for example, methyl, ethyl, propyl, isopropyl, butyl, isobutyl, sec-butyl, tert-butyl, etc. In the case where the alkyl is "C₁₋₆ alkyl", it includes, for example, pentyl, isopentyl, neopentyl, hexyl, etc. in addition to those mentioned above.

The term "cycloalkyl" refers to a monocyclic or polycyclic saturated hydrocarbon, and for example, "C₃₋₁₀ cycloalkyl" refers to a cyclic alkyl wherein the number of the carbon atoms is 3 to 10, and also includes a group which has a partially-cross-linked structure or forms a fused ring with aryl or heteroaryl. In the case where the cycloalkyl is "C₃₋₁₀ cycloalkyl", its specific example includes, for example, cyclopropyl, cyclobutyl, cyclopentyl, cyclohexyl, cycloheptyl, cyclooctyl, adamantyl, etc.

The term "alkoxy" refers to a group wherein its straight or branched chain saturated hydrocarbon group attaches through its oxygen atom to a parent molecular moiety, and for example, "C₁₋₆ alkoxy" refers to an alkoxy wherein the number of the carbon atoms is 1 to 6. In the case where the alkoxy is "C₁₋₆ alkoxy", its specific example includes, for example, methoxy, ethoxy, propoxy, isopropoxy, butyloxy, pentyloxy, isopentyloxy, neopentyloxy, hexyloxy, etc.

The term "halogen" refers to fluorine atom, chlorine atom, bromine atom or iodine atom. The preferable one among them is fluorine atom or chlorine atom.

The term "aryl" specifically includes, for example, phenyl, 1-naphthyl, 2-naphthyl, anthryl, etc. The preferable one among them includes phenyl.

The term "heteroaryl" includes a monocyclic 5- to 7-membered ring aromatic heterocyclic group, a bicyclic 8- to 11-membered aromatic heterocyclic group or a tricyclic 12-to 16-membered aromatic heterocyclic group, containing 1 to 4 atoms independently selected from the group consisting of nitrogen atom, oxygen atom and sulfur atom. It includes, for example, pyridyl, pyridazinyl, isothiazolyl, pyrrolyl, furyl, thienyl, thiazolyl, imidazolyl, pyrimidinyl, thiadiazolyl, pyrazolyl, oxazolyl, isooxazolyl, pyrazinyl, triazinyl, triazolyl, imidazolidinyl, oxadiazolyl, triazolyl, tetrazolyl, indolyl, indazolyl, chromenyl, quinolyl, isoquinolyl, benzofuranyl, benzothienyl, benzooxazolyl, benzothiazolyl, benzisooxazolyl, benzisothiazolyl, benzotriazolyl, benzimidazolyl, thioxanthene, 6,11-dihydrodibenzo[B,E]thiepinyl, etc. The preferable heteroaryl includes pyridyl, pyrimidinyl, quinolyl, and isoquinolyl.

The term "monocyclic heteroaryl" includes, for example, pyridyl, pyridazinyl, isothiazolyl, pyrrolyl, furyl, thienyl, thiazolyl, imidazolyl, pyrimidinyl, thiadiazolyl, pyrazolyl, oxazolyl, isooxazolyl, pyrazinyl, triazinyl, triazolyl, imidazolidinyl, oxadiazolyl, triazolyl, tetrazolyl, etc. The preferable one among them includes pyridyl, pyridazinyl, thienyl, imidazolyl, pyrimidinyl, etc. The most preferable one includes pyridyl and thienyl.

The term "6-membered heteroaryl which contains 1 or 2 nitrogen atoms" includes, for example, pyridyl, pyrimidinyl, pyrazinyl, pyridazinyl, etc. The preferable one among them includes pyrimidinyl.

The term "4- to 10-membered saturated heterocycle" refers to a saturated heterocycle consisting of 4 to 10 atoms comprising 1 to 2 atoms independently selected from the group consisting of nitrogen atom, oxygen atom and sulfur atom as well as carbon atoms. For example, it includes azetidine, pyrrolidine, piperidine, piperazine, morpholine, homopiperidine, tetrahydrofuran, tetrahydropyran, etc.

The term "4- to 10-membered nitrogen-containing saturated heterocycle" refers to a saturated heterocycle consisting of 4 to 10 atoms comprising at least 1 to 2 nitrogen atoms as well as carbon atoms. For example, it includes azetidine, pyrrolidine, piperidine, piperazine, homopiperidine, etc.

In the present compound of Formula (I), X-Y-Z, Q, R¹, R^{2A}, R^{2B}, R^{3A} to R^{3D}, R^{4A}, R^{4B}, R⁵ to R¹⁵, and n are preferably those shown below, but the technical scope of the present invention should not be limited to the following compounds.

X-Y-Z preferably includes N-CO-NR^{4A}R^{4B}, N-COR⁵, CR⁶-CO-NR^{4A}R^{4B}, CR⁶-NR⁷-COR⁵.

Q preferably includes 6-membered heteroaryl which contains 1 or 2 nitrogen atoms [in which the heteroaryl may be optionally substituted with 1 to 3 substituents independently selected from the group consisting of C₁₋₆ alkyl which may be optionally substituted with 1 to 5 substituents independently selected from the group consisting of fluorine, hydroxyl group and C₁₋₆ alkoxy; C₃₋₈ cycloalkyl or C₃₋₈ cycloalkoxy (in which the cycloalkyl and the cycloalkoxy may be each optionally substituted with 1 to 5 substituents independently selected from the group consisting of fluorine atom, hydroxyl group, C₁₋₆ alkyl and C₁₋₆ alkoxy); C₁₋₆ alkoxy which may be optionally substituted with 1 to 5 substituents independently selected from the group consisting of fluorine atom, hydroxyl group and C₁₋₆ alkoxy; or halogen]. It more preferably includes 6-membered heteroaryl which contains two nitrogen atoms in which the heteroaryl may be optionally substituted with 1 to 3 substituents independently selected from the group consisting of C₁₋₆ alkyl which may be optionally substituted with 1 to 5 substituents independently selected from the group consisting of fluorine atom and C₁₋₆ alkoxy; C₃₋₈ cycloalkyl which may be optionally substituted with 1 to 5 fluorine atoms; C₃₋₈ cycloalkoxy; C₁₋₆ alkoxy which may be optionally substituted with 1 to 5 fluorine atoms; chlorine atom; or fluorine atom. It furthermore preferably includes pyrimidinyl in which the pyrimidinyl may be optionally substituted with 1 to 3 substituents independently selected from the group consisting of C₁₋₆ alkyl which may be optionally substituted with 1 to 5 substituents independently selected from the group consisting of fluorine atom and C₁₋₆ alkoxy; C₃₋₈ cycloalkyl which may be optionally substituted with 1 to 5 fluorine atoms; C₃₋₈ cycloalkoxy; C₁₋₆ alkoxy which may be optionally substituted with 1 to 5 fluorine atoms; chlorine atom; or fluorine atom. It furthermore preferably includes pyrimidinyl in which the pyrimidinyl may be optionally substituted with 1 to 3 substituents independently selected from the group consisting of C₁₋₆ alkyl which may be optionally substituted with 1 to 5 fluorine atoms; or C₁₋₆ alkoxy which may be optionally substituted with 1 to 5 fluorine atoms.

R¹ preferably includes phenyl or monocyclic heteroaryl in which the phenyl and the monocyclic heteroaryl may be each optionally substituted with 1 to 5 substituents independenly selected from the group consisting of halogen, hydroxyl group, C₁₋₆ alkyl which may be optionally substituted with 1 to 5 fluorine atoms, C₁₋₆ alkoxy which may be optionally substituted with 1 to 5 fluorine atoms. It more preferably includes phenyl which may be optionally substituted with 1 to 5 substituents independently selected from the group consisting of halogen, C₁₋₆ alkyl which may be optionally substituted with 1 to 5 fluorine atoms, C₁₋₆ alkoxy which may be optionally substituted with 1 to 5 fluorine atoms. It furthermore preferably includes phenyl which may be optionally substituted with 1 to 5 substituents independently selected from the group consisting of fluorine atom, chlorine atom, C₁₋₆ alkyl which may be optionally substituted with 1 to 3 fluorine atoms, and C₁₋₆ alkoxy. It most preferably includes phenyl which may be optionally substituted with 1 to 5 substituents independently selected from the group consisting of fluorine atom, and C₁₋₆ alkoxy.

R^{2A} and R^{2B} are the same or different and preferably include hydrogen atom; halogen; cyano; C₁₋₆ alkyl which may be optionally substituted with 1 to 5 substituents independently selected from the group consisting of halogen, hydroxyl group, C₁₋₆ alkyl, C₃₋₁₀ cycloalkyl which may be optionally substituted with 1 to 5 fluorine atoms, C₁₋₆ alkoxy or 4- to 10-membered saturated heterocycle. More preferably, R^{2A} and R^{2B} are the same or different and include hydrogen atom, halogen or cyano. Furthermore preferably, R^{2A} includes halogen or cyano, and R^{2B} includes hydrogen atom. Most preferably, R^{2A} includes chlorine atom or cyano, and R^{2B} includes hydrogen atom.

R^{3A}, R^{3B}, R^{3C}, R^{3D} and R⁶ are the same or different and preferably include hydrogen atom, fluorine atom, hydroxyl group or C₁₋₆ alkyl. More preferable ones include hydrogen atom.

In the case where any two of R^{3A}, R^{3B}, R^{3C}, R^{3D} and R⁶ are indepedently selected from C₁₋₆ alkyl, the two alkyl groups may be combined each other together with the ring to which the alkyl groups attach to form another ring, which in particular includes the following rings. The carbon atoms on the newly formed ring may be optionally substituted with 1 to 5 fluorine atoms. It more preferably includes r³-1 and r³-2.

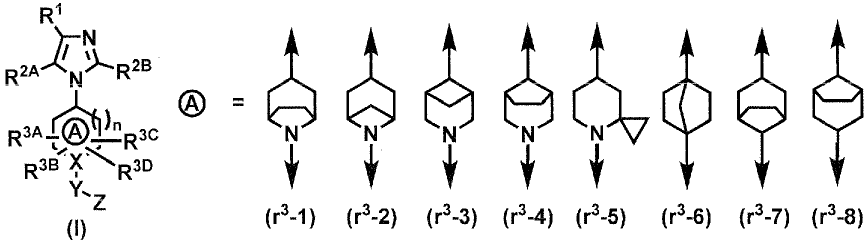

R^{4A}, R^{4B}, R⁵ and R⁷ are the same or different and preferably include C₁₋₆ alkyl which may be optionally substituted with 1 to 5 substituents independently selected from the group consisting of halogen, hydroxyl group, C₁₋₆ alkoxy, 4- to 10-membered saturated heterocycle, C₃₋₁₀ cycloalkyl and -NR¹²R¹³; C₃₋₁₀ cycloalkyl which may be optionally substituted with 1 to 5 substituents independently selected from the group consisting of halogen, hydroxyl group, C₁₋₆ alkoxy, C₁₋₆ alkyl and -NR¹²R¹³; 4- to 10-membered saturated heterocycle which may be optionally substituted with C₁₋₆ alkyl; or hydrogen atom. More preferably, they are the same or different and include C₁₋₆ alkyl which may be optionally substituted with 1 to 5 substituents independently selected from the group consisting of halogen, C₁₋₆ alkoxy, 4 to 10-membered saturated heterocycle and C₃₋₁₀ cycloalkyl; C₃₋₁₀ cycloalkyl which may be optionally substituted with 1 to 5 substituents independently selected from the group consisting of halogen, C₁₋₆ alkoxy, C₁₋₆ alkyl; or hydrogen atom. Furthermore preferably, they are the same or different and include C₁₋₆ alkyl which may be optionally substituted with 1 to 5 substituents independently selected from the group consisting of fluorine atom, 4- to 10-membered saturated heterocycle and C₃₋₁₀ cycloalkyl; C₃₋₁₀ cycloalkyl which may be optionally substituted with 1 to 5 substituents independently selected from the group consisting of fluorine atom, C₁₋₆ alkoxy and C₁₋₆ alkyl; or hydrogen atom. Most preferably, they are the same or different and include C₁₋₆ alkyl which may be optionally substituted with 1 to 5 substituents independently selected from the group consisting of fluorine atom and 4- to 10-membered saturated heterocycle; C₃₋₁₀ cycloalkyl which may be optionally substituted with 1 to 5 fluorine atoms; or hydrogen atom.

R⁸ to R¹⁵ are the same or different and preferably include hydrogen atom or C₁₋₆ alkyl. More preferable ones include C₁₋₆ alkyl.

n includes 1 or 2, preferably 1.

The pharmaceutically acceptable salt of the compound of Formula (I) refers to a salt which is formed with the compound of Formula (I) and a pharmceutically acceptable acid or base. The present compound of Formula (I) which has a basic functional group such as an amino group may form salts with various kinds of acids. Specific examples of the acid addition salt include an inorganic acid salt such as hydrochloride, hydrobromide, hydroiodide, hydrosulfate, perchlorate, and phosphate; an organic acid salt such as oxalate, malonate, maleate, fumarate, lactate, malate, citrate, tartrate, benzoate, trifluoroacetate, acetate, methanesulfonate, p-toluenesulfonate, trifluoromethanesulfonate; and an amino acid salt such as glutamate, aspartate.

The present compound of Formula (I) which has an acidic functional group such as a carboxyl group may form salts with various kinds of bases. Such pharmaceutically acceptable salts include an alkali metal salt such as sodium and potassium salt, an alkaline earth metal salt such as calcium salt, and ammonium salt. These salts can be prepared by mixing the present compound of Formula (I) with the above-mentioned base, followed by isolating it according to conventional methods such as recrystallization.

For the purpose of simplifying expressions, the following abbreviations may be used herein. o-: ortho-, m-: meta-, p-: para-, t-: tert-, s-: sec-, THF: tetrahydrofuran, DMF: N,N-dimethylformamide, NMP: N-methylpyrrolidone, DMSO: dimethylsulfoxide, d₆-DMSO: deuterated dimethylsulfoxide, HEPES: N-2-hydroxyethylpiperazin-N'-2-ethanesulfonic acid, BSA: bovine serum albumin, FDSS: Functional Drug Screening System, Boc: tert-butoxycarbonyl, c-Hex: cyclohexyl, EDCI: 1-ethyl-3-(3-dimethylaminopropyl)carbodiimide, HOBt: 1-hydroxybenzotriazole, HBTU: 2-(1H-7-benzotriazol-1-yl)-1,1,3,3-tetramethyluronium hexafluorophosphate, TFA: trifluoroacetic acid

Hereinafter, processes for preparing the present compound are explained. The present compound of Formula (I) can be prepared by, for example, the following Preparation Processes AtoG.

### Preparation Process A (Process for preparing synthetic intermediates)

Synthetic intermediates a3 to a5 for the compound of Formula (I) can be prepared by, for example, the following processes. 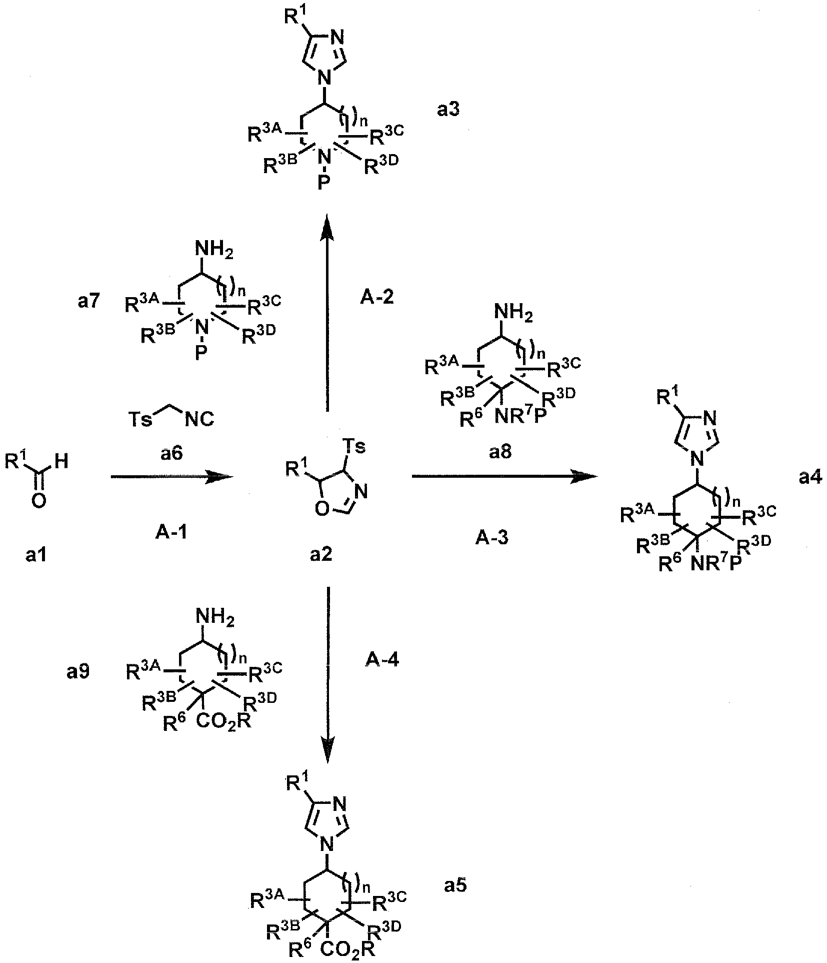 (In the scheme, R¹, R^{3A} to R^{3D}, R⁶, R⁷ and n are as defined in Item 1, P is a protecting group for the amino group, and R is alkyl or phenyl.)

Compound a1 can be synthesized by known methods such as an oxidation reaction of the corresponding alcohol and a reduction reaction of the corresponding ester, or is commercially available.

### [Step A-1]

In this step, Compound a1 is reacted with Compound a6 to give Compound a2. The solvent used in this step is selected from the solvents as illustrated hereinafter, and is preferably ethanol or tetrahydrofuran. The reaction temperature herein is preferably -78°C to 100°C, and the reaction time herein is preferably several minutes to several days. The process as described in, for example, Heterocycles, 1994, Vol. 39, 139-154 is known as a similar reaction and can similarly give the compound.

### [Step A-2]

In this step, Compound a2 which is obtained in the above Step A-1 can be reacted with Compound a7 to give Compound a3. The solvent used in this step is selected from the solvents as illustrated hereinafter, and is preferably xylene or toluene. The reaction temperature herein is preferably room temperature to 150°C, and the reaction time herein is preferably several minutes to several days. The process as described in, for example, Heterocycles, 1994, Vol. 39, 139-154 is known as a similar reaction and can similarly give the compound.

### [Step A-3]

In this step, Compound a2 which is obtained in the above Step A-1 is reacted with Compound a8 according to the above Step A-2 to give Compound a4.

### [Step A-4]

In this step, Compound a2 which is obtained in the above Step A-1 is reacted with Compound a9 according to the above Step A-2 to give Compound a5.

### Preparation Process B (Process for preparing synthetic intermediates)

Synthetic intermediates b2 to b4 for the compound of Formula (I) can be prepared by, for example, the following processes. 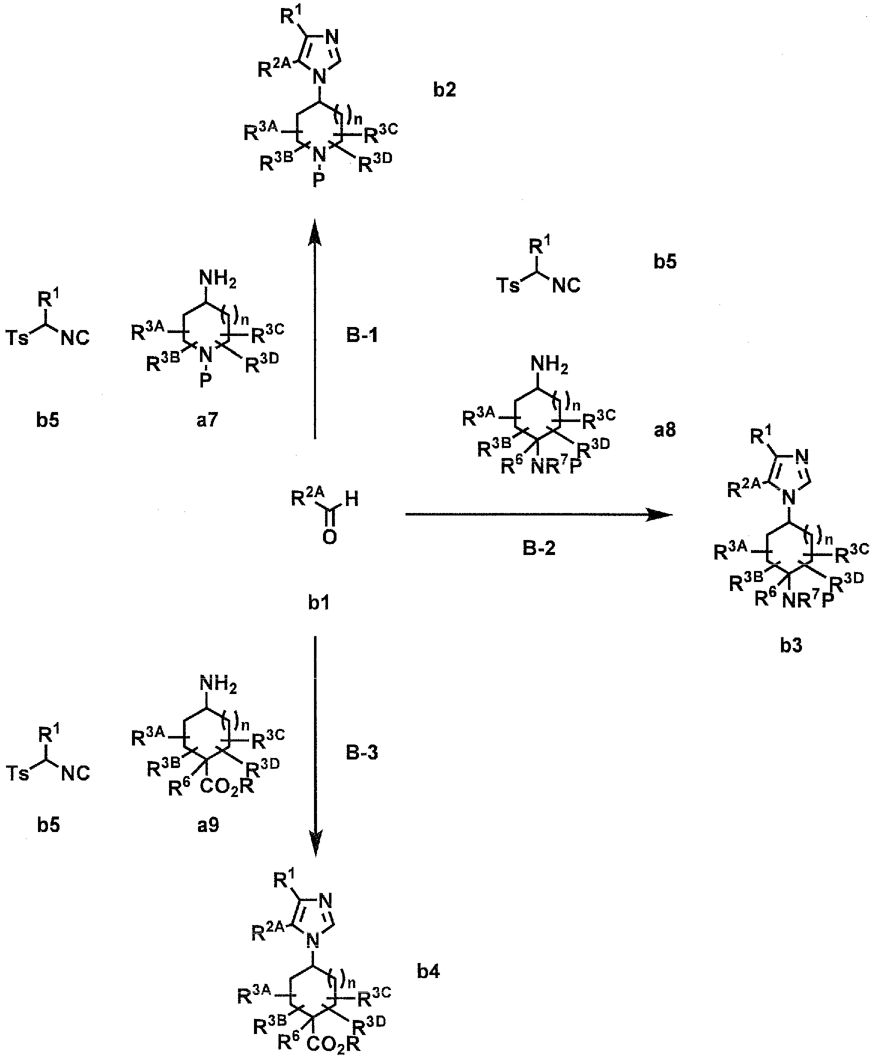 (In the scheme, R¹, R^{2A}, R^{3A} to R^{3D}, R⁶, R⁷ and n are as defined in Item 1, P is a protecting group for the amino group, and R is alkyl or phenyl.)

Compound b1 can be synthesized by known methods such as an oxidation reaction of the corresponding alcohol and a reduction reaction of the corresponding ester, or is commercially available.

Compound b5 can be synthesized by the method as desribed in, for example, Tetrahedron. Lett. 1996, 37, 8113-8116, Organic Synthesis, 2000, 77, 198, or is commercially available.

### [Step B-1]

In this step, Compound b1 can be reacted with Compound a7 and Compound b5 in an appropriate solvent in the presence of an appropriate base to give Compound b2. The base used in this step is selected from the bases as illustrated hereinafter, and is preferably potassium carbonate or piperazine. The solvent used in this step is selected from the solvents as illustrated hereinafter, and is preferably dimethylformamide or tetrahydrofuran. The reaction temperature herein is preferably -78°C to 150°C, and the reaction time herein is preferably several minutes to several days. The process as described in, for example, J. Org. Chem. 2000, 65, 1516-1524 is known as a similar reaction and can similarly give the compound.

### [Step B-2]

In this step, Compound b1 is reacted with Compound a8 and Compound b5 according to the above Step B-1 to give Compound b3.

### [Step B-3]

In this step, Compound b1 is reacted with Compound a9 and Compound b5 according to the above Step B-1 to give Compound b4.

### Preparation Process C

Among the compounds of Formula (I), compounds of formulae [C1], [C2] and [C3] wherein X-Y-Z is N-CO-NR^{4A}R^{4B} (also referred to hereinafter as Compound C1, C2, and C3, respectively) can be prepared by, for example, the following processes. 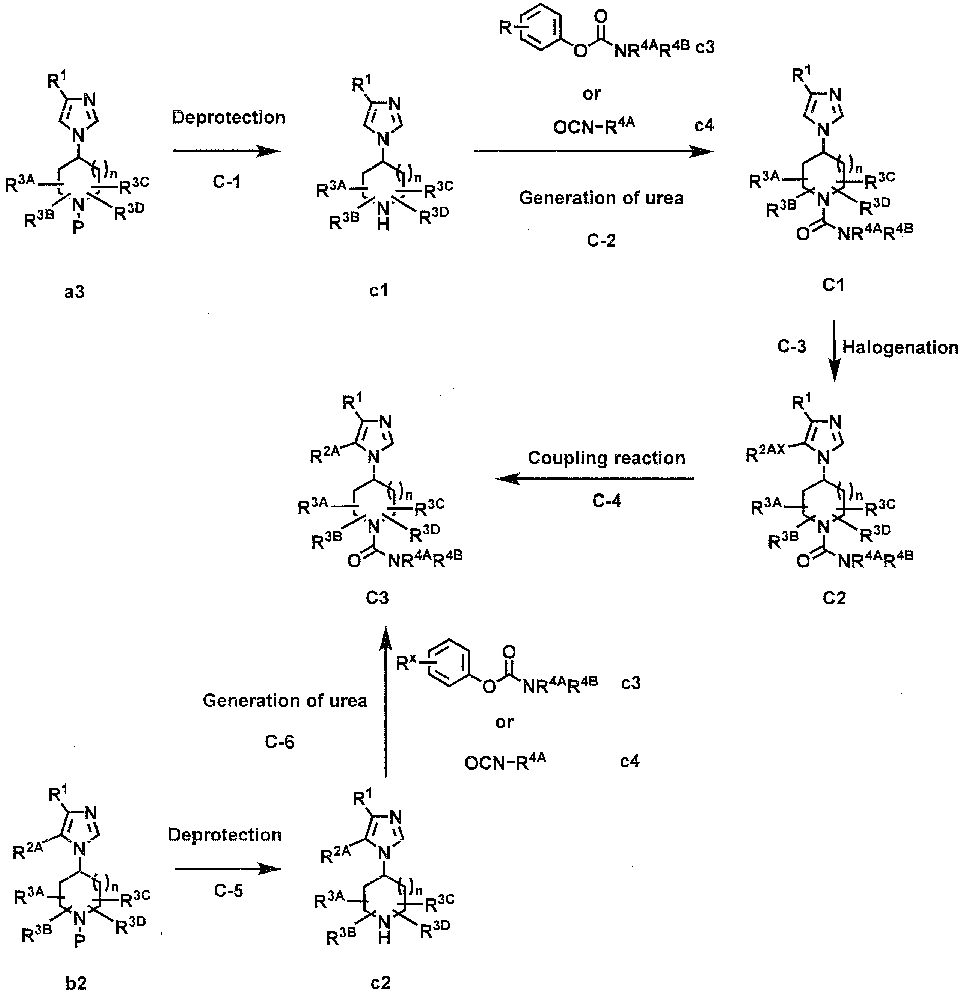 (In the scheme, R¹, R^{2A}, R^{3A} to R^{3D}, R^{4A}, R^{4B} and n are as defined in Item 1, R and R^{x} are hydrogen atom, nitro, fluorine atom or trifluoromethyl, R^{2AX} is chlorine atom, bromine atom or iodine atom, and P is a protecting group of the amino group.)

### [Step C-1]

In this step, a protecting group, P, of the amino group in Compound a3 which is obtained in the above Preparation Process A is deprotected to give Compound c1 This step can be carried out according to the process described in Protective Groups in Organic Synthesis (Theodora W. Greene, Peter G. M. Wuts, John Wiley & Sons, Inc., 1999), etc.

### [Step C-2]

In this step, Compound c1 which is obtained in the above Step C-1 is reacted with Compound c3 or c4 in the presence of an appropriate base in an appropriate solvent to give Compound C1. The base used in this step is selected from the bases as illustrated hereinafter, and is preferably diisopropylethylamine or triethylamine. The solvent used in this step is selected from the solvents as illustrated hereinafter, and is preferably tetrahydrofuran or methylene chloride. The reaction temperature herein is preferably -78°C to 100°C, and the reaction time is preferably several minutes to several days. The processes as described in, for example, J. Org. Chem. 1995, 60(25), 8262-8266, Bioorg. Med. Chem. Lett. 2004, 14(3), 727-779, Tetrahedron Lett.2001, 42(8), 1445-1447, etc. are known as a similar reaction and can similarly give the compound.

### [Step C-3]

In this step, Compound C1 which is obtained in the above Step C-2 is reacted with various halogenating agents in an appropriate solvent in the presence of an appropriate acid to give Compound C2. The halogenating agent used in this step is preferably N-chlorosuccinimide, N-bromosuccinimide, N-iodosuccinimide. The solvent used in this step is selected from the solvents as illustrated hereinafter, and is preferably methylene chloride or dichloroethane. The acid used in this step is selected from the acids as illustrated hereinafter, and is preferably trifluoroacetic acid or hydrochloric acid. The reaction temperature herein is preferably -78°C to 100°C, and the reaction time is preferably several minutes to several days. The processes as described in, for example, Bioorg. Med. Chem. Lett. 2008, 18(5), 1702-1707, J. Org. Chem. 2002, 67(17), 5913-5918, etc. are known as a similar reaction and can similarly give the compound.

### [Step C-4]

In this step, Compound C2 which is obtained in the above Step C-3 is reacted in an appropriate solvent in the presence of an appropriate metal reagent to give Compound C3. The reaction temperature herein is preferably -78°C to 150°C, and the reaction time is preferably several minutes to several days. The processes as described in, for example, Tetrahedron Lett. 2003, 44(7), 1379-1382, J. Med. Chem. 2009, 52(14), 4370-4379, Bioorg. Med. Chem. Lett. 2012, 20(9), 3009-3015, J. Org. Chem. 2002, 67(10), 3365-3373, Tetrahedron Lett. 2007, 48(13), 2339-2343, etc. are known as a similar reaction and can similarly give the compound.

### [Step C-5]

In this step, Compound b2 which is obtained in the above Preparation Process B is reacted under the condition according to the above Step C-1 to give Compound c2.

### [Step C-6]

In this step, Compound c2 which is obtained in the above Step C-5 is reacted with Compound c3 or c4 under the condition according to the above Step C-2 to give Compound C3.

### Preparation Process D

Among the compounds of Formula (I), compounds of formulae [D1], [D2], and [D3] wherein X-Y-Z is N-COR⁵ (also referred to hereinafter as Compound D1, D2, and D3, respectively) can be prepared by, for example, the following processes. 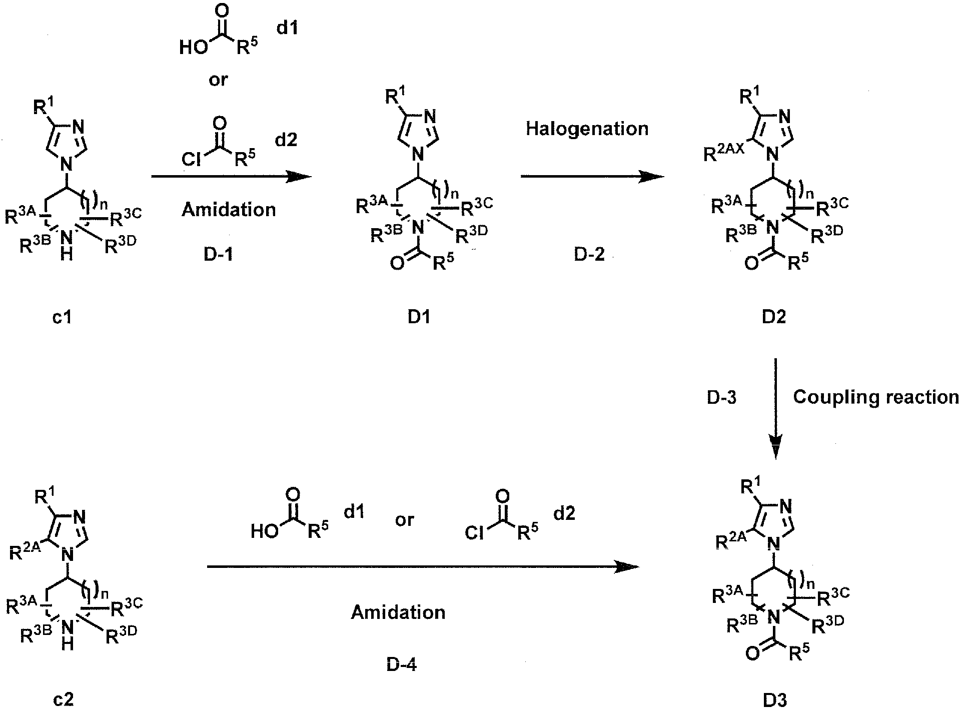 (In the scheme, R¹, R^{2A}, R^{3A} to R^{3D}, R⁵, and n are as defined in Item 1, and R^{2AX} is chlorine atom, bromine atom or iodine atom.)

### [Step D-1]

In this step, Compound c1 which is obtained in the Preparation Process C is reacted with Compound d1 or d2 in the presence or absence of an appropriate condensing agent in the presence of an appropriate base in an appropriate solvent to give Compound D1. The condensing agent used in this step is preferably EDCI (including its hydrochloride) or HBTU. The base used in this step is selected from the bases as illustrated hereinafter, and is preferably diisopropylethylamine or triethylamine. The solvent used in this step is selected from the solvents as illustrated hereinafter, and is preferably dimethylformamide, tetrahydrofuran or methylene chloride. The reaction temperature herein is preferably -78°C to 100°C, and the reaction time is preferably several minutes to several days.

### [Step D-2]

In this step, Compound D1 which is obtained in the above Step D-1 is reacted under the condition according to the above Step C-3 to give Compound D2.

### [Step D-3]

In this step, Compound D2 which is obtained in the above Step D-2 is reacted under the condition according to the above Step C-4 to give Compound D3.

### [Step D-4]

In this step, Compound c2 which is obtained in the Preparation Process C is reacted with Compound d1 or d2 under the condition according to the above Step D-1 to give Compound D3.

### Preparation Process E

Among the compounds of Formula (I), compounds of formulae [E1], [E2], and [E3] wherein X-Y-Z is CR⁶-NR⁷-COR⁵ or CR⁶-NR⁷-Q (also referred to hereinafter as Compound E1, E2, and E3, respectively) can be prepared by, for example, the following processes. 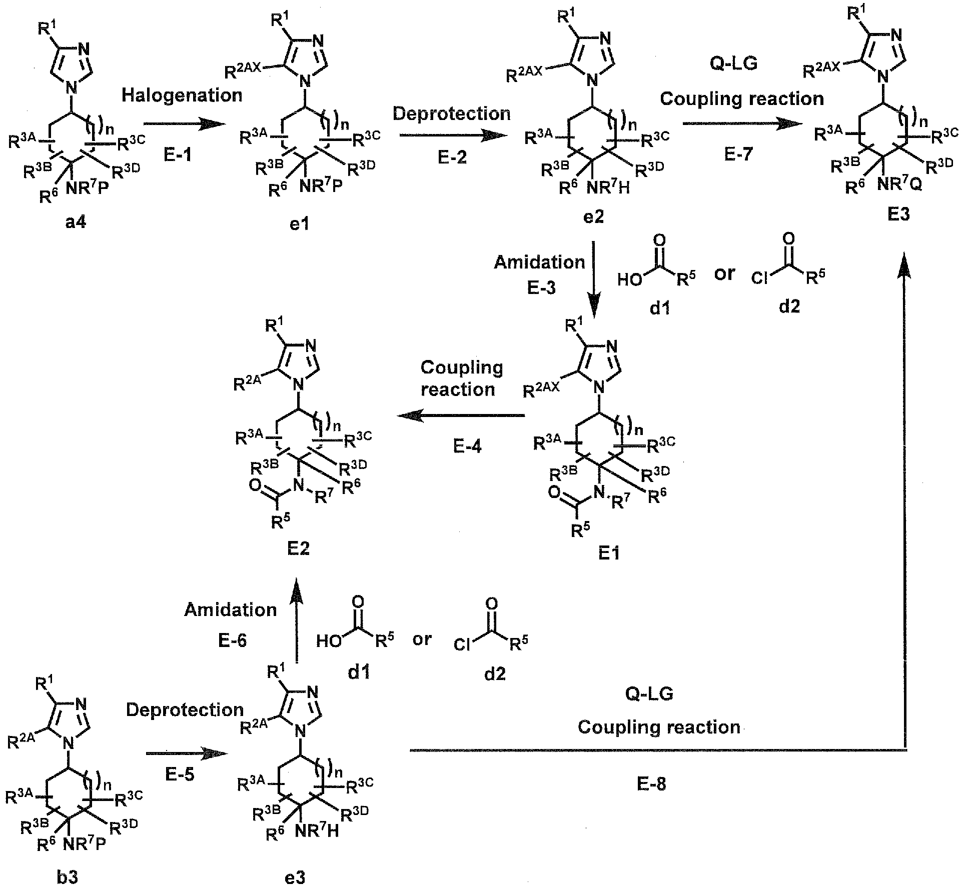 (In the scheme, Q, R¹, R^{2A}, R^{3A} to R^{3D}, R⁵, R⁶, R⁷, and n are as defined in Item 1, and R^{2AX} is chlorine atom, bromine atom or iodine atom, P is a protecting group of the amino group, and LG is a leaving group such as halogen.)

Compound Q-LG can be prepared by the processes described in, for example, EP1333029 (A1), European Journal of Organic Chemistry, 6, 1593-1598 (2006), US2004/2507 A1 2004, Tetrahedron Letters, 46, 3977-3979 (2005), WO 2011/063272 pamphlet, etc., or is commercially available.

### [Step E-1]

In this step, Compound a4 which is obtained in the Preparation Procee A is reacted under the condition according to the above Step C-3 to give Compound e1.

### [Step E-2]

In this step, Compound e1 which is obtained in the above Step E-1 is reacted under the condition according to the above Step C-1 to give Compound e2.

### [Step E-3]

In this step, Compound e2 which is obtained in the above Step E-2 is reacted with Compound d1 or d2 under the condition according to the above Step D-1 to give Compound E1.

### [Step E-4]

In this step, Compound E1 which is obtained in the above Step E-3 is reacted under the condition according to the above Step C-4 to give Compound E2.

### [Step E-5]

In this step, Compound b3 which is obtained in the Preparation Process B is reacted under the condition according to the above Step C-1 to give Compound e3.

### [Step E-6]

In this step, Compound e3 which is obtained in the above Step E-5 is reacted with Compound d1 or d2 under the condition according to the above Step D-1 to give Compound E2.

### [Step E-7]

In this step, Compound e2 which is obtained in the above Step E-2 is coupled with Compound Q-LG in the presence or absence of a catalyst in the presence of a base, under neat or in an appropriate solvent, to give Compound E3. The catalyst used herein includes a transition metal (e.g. palladium) or its salt, a complex thereof, or those which are supported on a carrier such as polymer. The base used in this step is selected from the bases as illustrated hereinafter, and is preferably diisopropylethylamine, triethylamine or potassium carbonate. The solvent used in this step should be selected depending on the types of starting compounds, etc., and includes, for example, N,N-dimethylformamide, 1-methylpyrrolidin-2-one, dimethylsulfoxide, tetrahydrofuran, dioxane, ethylene glycol dimethyl ether, methylene chloride, ethyl acetate, acetone, acetonitrile or water. These solvents each may be used alone, or in combination with two or more of them. The reaction temperature is preferably room temperature to 200°C, the reaction time is preferably several minutes to several days, and a reaction under microwave exposure can be also carried out.

### [Step E-8]

In this step, Compound e3 which is obtained in the above Step E-5 is reacted with Compound Q-LG under the condition according to the above Step E-7 to give Compound E3.

### Preparation Process F

Among the compounds of Formula (I), compounds of formulae [F1], and [F2] wherein X-Y-Z is CR⁶-CO-NR^{4A}R^{4B} (also referred to hereinafter as Compounds F1 and F2, respectively) can be prepared by, for example, the following processes. 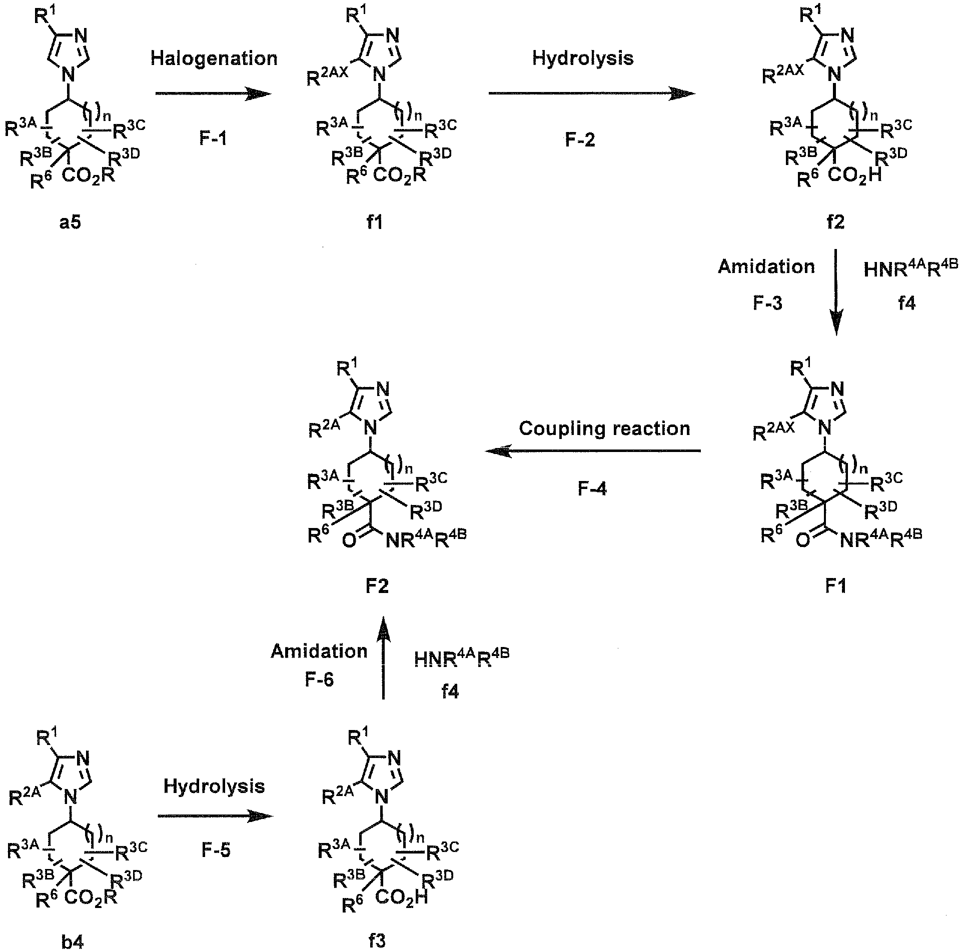 (In the scheme, R¹, R^{2A}, R^{3A} to R^{3D}, R^{4A}, R^{4B}, R⁶, and n are as defined in Item 1, R^{2AX} is chlorine atom, bromine atom or iodine atom, and R is alkyl, phenyl or benzyl.)

### [Step F-1]

In this step, Compound a5 which is obtained in the Preparation Process A is reacted under the condition according to the above Step C-3 to give Compound f1.

### [Step F-2]

In this step, the ester compound f1 which is obtained in the above Step F-1 is converted into the corresponding carboxylic acid compound f2. This step can be carried out according to the process described in Protective Groups in Organic Synthesis (Theodora W. Greene, Peter G. M. Wuts, John Wiley & Sons, Inc., 1999), etc.

### [Step F-3]

In this step, Compound f2 which is obtained in the above Step F-2 is reacted with Compound f4 in the presence of an appropriate condensing agent and an appropriate base in an appropriate solvent to give Compound F1. The condensing agent used in this step is preferably EDCI (including its hydrochloride) or HBTU. The base used in this step is selected from the bases as illustrated hereinafter, and is preferably diisopropylethylamine or triethylamine. The solvent used in this step is selected from the solvents as illustrated hereinafter, and is preferably dimethylformamide, tetrahydrofuran or methylene chloride. The reaction temperature herein is preferably -78°C to 100°C, and the reaction time is preferably several minutes to several days.

### [Step F-4]

In this step, Compound F1 which is obtained in the above Step F-3 is reacted under the condition according to the above Step C-4 to give Compound F2.

### [Step F-5]

In this step, Compound b4 which is obtained in the Preparation Process B is reacted under the condition according to the above Step F-2 to give Compound f3.

### [Step F-6]

In this step, Compound f3 which is obtained in the above Step F-5 is reacted with Compound f4 under the condition according to the above Step F-3 to give Compound F2.

### Preparation Process G

Among the compounds of Formula (I), the compounds of formulae [G1] and [G2] wherein X-Y-Z is CR⁶-NR⁷-CONR^{4A}R^{4B} (also referred to hereinafter as Compound G1 and G2, respectively) can be prepared by, for example, the following processes. 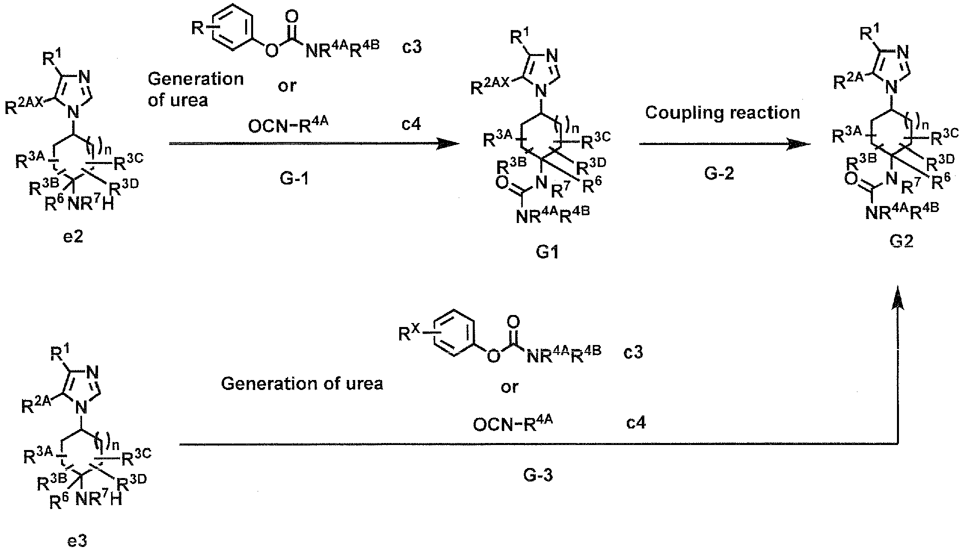 (In the scheme, R¹, R^{2A}, R^{3A} to R^{3D}, R^{4A}, R^{4B}, R⁶, R⁷, and n are as defined in Item 1, R and R^{x} are hydrogen atom, nitro, fluorine atom or trifluoromethyl, and R^{2AX} is chlorine atom, bromine atom or iodine atom.)

### [Step G-1]

In this step, Compound e2 which is obtained in the Preparation Process E is reacted with Compound c3 or c4 under the condition according to the above Step C-2 to give Compound G1.

### [Step G-2]

In this step, Compound G1 which is obtained in the above Step G-1 is reacted under the condition according to the above Step C-4 to give Compound G2.

### [Step G-3]

In this step, Compound e3 which is obtained in the Preparation Process E is reacted with Compound c3 or c4 under the condition according to the above Step C-2 to give Compound G2.

The imidazole derivatives wherein R^{2B} is hydrogen atom prepared in the Preparation Processes A to G can be subject to a conventional nucleophilic substitution reaction to give compounds of Formula (I) wherein R^{2B} has a substituent other than hydrogen atom.

The base used in each step in the above processes can be optionally selected depending on the type of reactions and starting compounds, etc.; and includes, for example, alkaline bicarbonates such as sodium bicarbonate and potassium bicarbonate; alkaline carbonates such as sodium carbonate and potassium carbonate; metal hydrides such as sodium hydride and potassium hydride; alkali metal hydroxides such as sodium hydroxide and potassium hydroxide; alkali metal alkoxides such as sodium methoxide and sodium t-butoxide; organometallic bases such as butyllithium and lithium diisopropylamide; and organic bases such as triethylamine, diisopropylethylamine, pyridine, 4-dimethylaminopyridine (DMAP) and 1,8-diazabicyclo[5.4.0]-7-undecene (DBU).

The solvent used in each step in the above processes can be optionally selected depending on the type of reactions and starting compounds, etc.; and includes, for example, alcohols such as methanol, ethanol and isopropanol; ketones such as acetone and methyl ketone; halogenated hydrocarbons such as methylene chloride and chloroform; ethers such as tetrahydrofuran (THF) and dioxane; aromatic hydrocarbons such as toluene and benzene; aliphatic hydrocarbons such as hexane and heptane; esters such as ethyl acetate and propyl acetate; amides such as N,N-dimethylformamide (DMF) and N-methyl-2-pyrrolidone; sulfoxides such as dimethylsulfoxide (DMSO); and nitriles such as acetonitrile. These solvents can be used alone or in combination with two or more of them. In addition, organic bases may be also used as the solvent depending on the type of reactions.

The present compound of Formula (I) or an intermediate thereof can be isolated and purified by well-known methods for one skilled in the art. For example, such methods include extraction, partition, reprecipitation, column chromatography (e.g. silica gel column chromatography, ion exchange column chromatography or preparative liquid chromatography) or recrystallization, etc. The recrystallization solvent used herein includes, for example, alcohol solvents such as methanol, ethanol or 2-propanol; ether solvents such as diethyl ether; ester solvents such as ethyl acetate; aromatic hydrocarbon solvents such as benzene and toluene; ketone solvents such as acetone; halogen solvents such as dichloromethane and chloroform; hydrocarbon solvents such as hexane; aprotic solvents such as dimethylformamide and acetonitrile; water, or a mixed solvent of the above-listed solvents. Other purification methods including, for example, those described in Experimental Chemistry Textbook Vol. 1 (the Chemical Society of Japan, ed., Maruzen) can be also used herein. The molecular structure of the present compound can be readily determined by spectrographic methods such as nuclear magnetic resonance method, infrared absorption spectroscopy, or circular dichroism spectroscopy, and mass spectrometry in view of each structure derived from each starting compound.

The present compound of Formula (I) or a pharmaceutically acceptable salt thereof may exhibit chirality or contain a substituent having an asymmetric carbon, which can exist as optical isomers. The present compound includes a mixture of each of the isomers and a single isomer isolated therefrom, which can be prepared according to a conventional method. Such a conventional method includes, for example, using a starting material having an asymmetric center, or introducing chirality during the process. For example, in order to obtain an optical isomer, it can be prepared by using optically active compounds as a starting material, or carrying out an optical resolution at an appropriate stage during the process. In the case where the compound of Formula (I) or an intermediate thereof has a basic functional group, the optical resolution method includes, for example, a diastereomeric method which forms a salt using an optically active acid (e.g. a monocarboxylic acid such as mandelic acid, N-benzyloxyalanine or lactic acid, a dicarboxylic acid such as tartaric acid, o-diisopropylidene tartaric acid or malic acid, a sulfonic acid such as camphor sulfonic acid or bromocamphor sulfonic acid) in an inert solvent (e.g. an alcoholic solvent such as methanol, ethanol and 2-propanol; an ether solvent such as diethylether; an ester solvent such as ethyl acetate; a hydrocarbon solvent such as toluene; an aprotic solvent such as acetonitrile; or a mixed solvent thereof). In the case where the compound of Formula (I) or an intermediate thereof has an acidic functional group such as carboxyl group, the optical resolution method can be also carried out by using an optically active amine (e.g. an organic amine such as 1-phenylethylamine, kinin, quinidine, cinchonidine, cinchonine and strychnine) to form its salt.

The temperature for forming the salt is selected from the range of -50°C to a boiling point of a solvent as used, more preferably the range of room temperature to a boiling point of the solvent. In order to improve the optical purity, it is desirable that the temperature is once raised to around a boiling point of the solvent. When a precipitated salt is collected on a filter, the filtration may be, if necessary, carried out under a cooled condition to improve the yield. The appropriate amount of an optically active acid or amine used herein is about 0.5 to about 2.0 equivalents, preferably about 1 equivalent, per mole of the reactant. If necessary, the crystal may be recrystallized from an inert solvent (e.g. an alcoholic solvent such as methanol, ethanol and 2-propanol; an ether solvent such as diethylether; an ester solvent such as ethyl acetate; a hydrocarbon solvent such as toluene; an aprotic solvent such as acetonitrile; or a mixed solvent thereof) to give the optically active salt in a high purity. In addition, if necessary, it is also possible to treat the optically-resolved salt with an acid or a base by a conventional method to give a free form thereof.

Alternatively, in the case where the compound of Formula (I) or an intermediate thereof has carboxyl group, the optical resolution method can be also carried out by using an optically active amine (e.g. 1-phenylethylamine, etc.) to form its amide.

The present compound can be a novel medicament for treating and / or preventing a disease due to an abnormality of the intracellular signaling mediated by acetylcholine, in particular, CIAS (cognitive impairment associated with schizophrenia), Alzheimer's disease, Down's syndrome, cognitive disorder, mild cognitive disorder, memory disorder/learning disorder, attention deficit/hyperactivity disorder or cerebral angiopathy, for example.

The present compound can be also a novel medicament for treating nervous system disease, psychiatric disease, and inflammatory disease (e.g. senile dementia, attentional deficit disorder, Alzheimer's disease, and schizophrenia). The administration route of the present compound may be any of oral, parenteral or rectal administration; and the daily dosage thereof may vary depending on the type of the compound, the administration method, conditions / age of the patient, and other factors. For example, in the case of an oral administration, the present compound can be administered to human beings or mammals at typically about 0.01 to 1000 mg and more preferably about 0.1 to 500 mg per kg of body weight as a single or multiple doses. In the case of a parenteral administration such as an intravenous injection, the present compound can be administered to human beings or mammals at typically about 0.01 mg to 300 mg and more preferably about 1 mg to 100 mg per kg of body weight. The term "treating" used herein also includes a prophylactic administration.

The dosage forms of the present compound include, for example, tablets, capsules, granules, powders, syrups, suspensions, injections, suppositories, eye drops, ointments, embrocations, adhesive skin patches, and inhalants. These formulations can be prepared according to conventional methods. In addition, liquid formulations may be in a form wherein the present compound is dissolved or suspended in water, appropriate aqueous solutions, or other appropriate media at the time of use. Tablets and granules may be coated according to known methods. Furthermore, these formulations may comprise additional ingredients which are useful for the treatment.

The present compound can be used in combination with an atypical antipsychotic drug. The atypical antipsychotic drug includes, for example, olanzapine, risperidone, paliperidone, quetiapine, ziprasidone, aripiprazole, asenapine, iloperidone, clozapine, sertindole, blonanserin and lurasidone.

### EXAMPLES

Hereinafter, details are further explained in particular in Reference Examples, Examples and Test Examples, but the present invention is not intended to be limited thereto. In addition, compounds were identified by, for example, elementary analysis, mass spectra, high performance liquid chromatograph-mass spectrometer, LCMS, IR spectra, NMR spectra, and high performance liquid chromatography (HPLC).

For the purpose of simplifying expressions herein, the following abbreviations may be optionally used in Reference Examples, Examples and the tables in Examples. When referring to substituents in abbreviation, Me is methyl group, Et is ethyl group, Ph is phenyl group, Ts is tosyl group. TFA is trifluoroacetic acid. The following abbreviations are used in NMR data: s: singlet; d: doublet; dd: doublet of doublet; t: triplet; td: triplet of doublet; q: quartet; m: multiplet; br: broad; brs: broad singlet; brd: broad doublet; brt: broad triplet; and J: a coupling constant.

High performance liquid chromatograph-mass spectrometer: The measurement conditions of LCMS are shown below; the observed value of mass spectrometry [MS(m/z)] is shown as MH+, and the retention time is shown as Rt (minutes, min). In addition, the conditions used in measuring each of observed values are shown as A to G.

**Measurement Condition A**

| | |
|---|---|
| Detector: | Waters ACQUITY UPLC |
| Column: | ACQUITY UPLC BEH C18 1.7 µm 2.1 × 50 mm column |
| Solvent: | Solution A: 0.05% HCOOH/H₂O, Solution B: CH₃CN |
| Gradient Condition: | |
| | 0.0-1.3 min; A/B = 90:10 to 1:99 (linear gradient) |
| | 1.35-1.5 min; A/B = 1:99 |
| | 1.5-2 min; A/B = 90:10 |
| Flow Rate: | 0.75 mL/min |
| UV: | 220 nm, 254 nm |
| Column temperature: | 50°C |

**Measurement Condition B**

| | |
|---|---|
| Detector: | Shimadzu LCMS-2020 |
| Column: | Phenomenex Kinetex 1.7 µm C18 2.1 mm × 50 mm |
| Solvent: | Solution A: MeOH, Solution B: 0.05% TFA/H₂O |
| Gradient condition: | |
| | 0 min: A/B = 30:70 |
| | 0-1.90 min: A/B = 99:1 |
| | 1.91-3.00 min: A/B = 30:70 |
| Flow Rate: | 0.5 ml/min. |
| UV: | 220 nm |
| Column temperature: | 40°C |

**Measurement Condition C**

| | |
|---|---|
| Detector: | A series of Agilent 1100 for a series of API (manufactured by Applied Biosystems) |
| HPLC: | API 150EX LC/MS system (manufactured by Applied Biosystems) |
| Column: | YMC CombiScreen Hydrosphere C18 (S-5 µM, 12 nm, 4.6 × 50 mm) |
| Solvent: | Solution A: 0.05 % TFA/H₂O, Solution B: 0.05 % TFA/MeOH |
| Gradient Condition: | 0.0-6.0 min; A/B = 75:25 to 1:99 (linear gradient) |
| Flow rate: | 3.5 mL/min |
| UV: | 254 nm |

**Measurement Condition D**

| | |
|---|---|
| Detector: | Shimadzu, LC: 20A, MS: 2010 |
| Column: | Xtimate C18 2.1*30 mm, 3 µm |
| Solvent: | Solution A: 1.5 mL/4L TFA/H₂O, Solution B: 0.75 mL/4L TFA/MeCN |
| Gradient Condition: | Using the elution gradient 10%-80% (solvent B) over 2.2 minutes and holding at 80% for 0.3 minutes |
| Flow rate: | 0.8 mL/min |
| UV: | 220 nm |

**Measurement Condition E**

| | |
|---|---|
| Detector: | Shimadzu, LC: 20A, MS: 2010 |
| Column: | Xtimate C18 2.1*30 mm, 3 µm |
| Solvent: | Solution A: 1.5 mL/4L TFA/H₂O, Solution B: 0.75 mL/4 L TFA/MeCN |
| Gradient Condition: | Using the elution gradient 30%-90% (solvent B) over 2.2 minutes and holding at 90% for 0.3 minutes |
| Flow rate: | 0.8 mL/min |
| UV: | 220 nm |

**Measurement Condition F**

| | |
|---|---|
| Detector: | Shimadzu, LC: 20A, MS: 2010 |
| Column: | Xtimate C18 2.1*30 mm, 3 µm |
| Solvent: | Solution A: 1.5 mL/4 L TFA/H₂O, Solution B: 0.75 mL/4 L TFA/MeCN |
| Gradient Condition: | Using the elution gradient 0%-60% (solvent B) over 2.2 minutes and holding at 60% for 0.3 minutes |
| Flow rate: | 0.8 mL/min |
| UV : | 220 nm |

**Measurement Condition G**

| | |
|---|---|
| Detector: | Agilent, LC: 1200, MS: 6110 |
| Column: | Xbrige RP-18 2.1*50 mm, 5 µm |
| Solvent: | Solution A: 0.5 mL/1 L NH₃ • H₂O/H₂O, Solution B: MeCN |
| Gradient Condition: | Using the elution gradient 10%-80% (solvent B) over 2.0 minutes and holding at 80% for 0.5 minutes |
| Flow rate: | 1.0 mL/min |
| UV: | 220 nm |

### Reference Example 1

### tert-Butyl 4-[4-(3-fluorophenyl)-1H-imidazol-1-yl]piperidine-1-carboxylate (Reference Example 1)

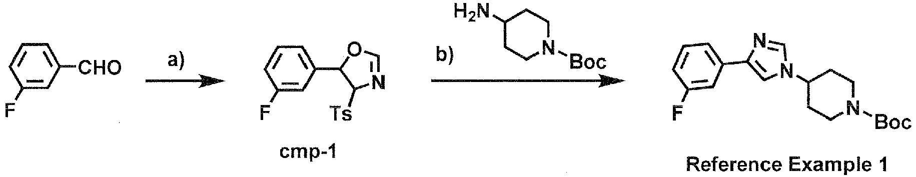

### a) Prepration of 5-(3-fluorophenyl)-4-[(4-methylphenyl)sulfonyl]-4,5-dihydro-1,3-oxazole (Compound cmp-1)

To a solution of 3-fluorobenzaldehyde (6.68 g) in ethanol (200 ml) and tetrahydrofuran (60 ml) was added p-toluenesulfonylmethylisocyanide (10 g) at room temperature, and sodium cyanide (252 mg) dissolved in a small amount of water was added dropwise thereto, and then the mixture was stirred at room temperature for 3 hours. The reaction solution was concentrated under reduced pressure, and then to the resulting residue was added ethyl acetate. The mixture was dried over anhydrous magnesium sulfate, and then concentrated under reduced pressure to give Compound cmp-1 (15.8 g).
LCMS; [M+H]⁺ / Rt (min): Measurement Condition (320 / 1.02: A)

### b) Preparation of tert-butyl 4-[4-(3-fluorophenyl)-1H-imidazol-1-yl]piperidine-1-carboxylate (Reference Example 1)

To Compound cmp-1 (15.8 g) were added tert-butyl 4-aminopiperidine-1-carboxylate (15.4 g) and xylene (100 ml) at room temperature, and the mixture was stirred under nitrogen atmosphere for 13 hours with heating at 135°C. The reaction solution was concentrated under reduced pressure, and then the resulting residue was purified by silica gel column chromatography to give Reference Example 1 (5.63 g).
LCMS; [M+H]⁺ / Rt (min): Measurement Condition (346 / 0.70: A)

### Reference Example 2

### 4-(5-Bromo-4-phenyl-1H-imidazol-1-yl)piperidine (Reference Example 2)

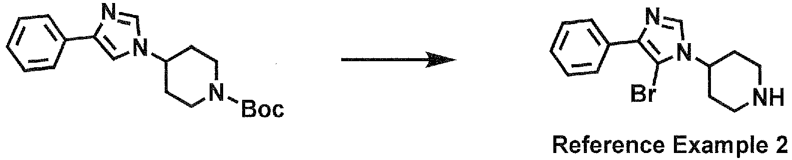

To a solution of tert-butyl 4-(4-phenyl-1H-imidazol-1-yl)piperidine-1-carboxylate (1 g) obtained in a similar manner to Reference Example 1 in methylene chloride (10 ml) were added N-bromosuccinimide (816 mg) and trifluoroacetic acid (1.18 ml) at room temperature, and then the mixture was heated to reflux at 50°C for 3 hours. The reaction was quenched by adding aqueous saturated sodium hydrogen carbonate to the reaction solution under ice cooling, which was then extracted with chloroform. The organic layer was dried over sodium sulfate and the solvent was removed under reduced pressure, and then the resulting residue was purified by silica gel column chromatography (Eluting solvent; chloroform:methanol = 100:0 to 90:10) to give Reference Example 2 (900 mg).
LCMS; [M+H]⁺ / Rt (min): Measurement Condition (306 / 0.18: A)

### Reference Example 3

### tert-Butyl 4-(5-cyano-4-phenyl-1H-imidazol-1-yl)piperidine-1-carboxylate (Reference Example 3)

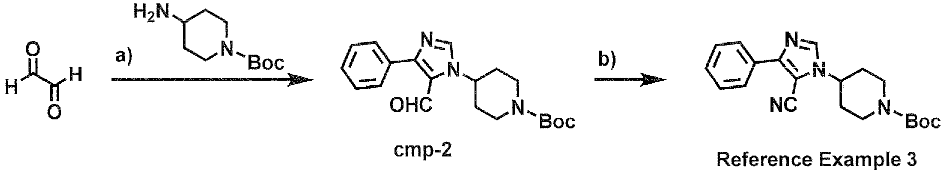

### a) Preparation of tert-butyl 4-(5-formyl-4-phenyl-1H-imidazol-1-yl)piperidine-1-carboxylate (Compound cmp-2)

To a solution of 40% aqueous glyoxal solution (1.52 g) in dimethylformamide (50 ml) was added tert-butyl 4-aminopiperidine-1-carboxylate (2.8 g) at room temperature, and the mixture was stirred at room temperature for 8 hours, and then thereto were added (1-phenyl-1-tosyl)methylisocyanide (2 g) and potassium carbonate (2.41 g), and the mixture was stirred at room temperature for 18 hours. The reaction was quenched by adding 1 mol/L hydrochloric acid to the reaction solution, which was then extracted with chloroform. The organic layer was dried over sodium sulfate and the solvent was removed under reduced pressure, and then the resulting residue was purified by silica gel column chromatography (Eluting solvent; chloroform:methanol = 100:0 to 90:10) to give Compound cmp-2 (549 mg).
LCMS; [M+H]⁺ / Rt (min): Measurement Condition (356 / 1.58: B)

### b) Preparation of tert-butyl 4-(5-cyano-4-phenyl-1H-imidazol-1-yl)piperidine-1-carboxylate (Reference Example 3)

To an aqueous solution (10 ml) of hydroxylamine hydrochloride (754 mg) was added sodium hydrogen carbonate (916 mg) at room temperature, and then thereto was added a solution of Compound cmp-2 (549 mg) in ethanol (5 ml), and the mixture was stirred at room temperature for 15 hours. Then, the precipitated solid was collected on a filter and washed with water, and then dried at 60°C to give a solid (298 mg). Then, thereto was added acetic anhydride (15 ml) at room temperature, and the mixture was stirred under reflux for 15 hours. The reaction solution was concentrated under reduced pressure, and then extracted with chloroform, and the organic layer was dried over sodium sulfate and the solvent was removed under reduced pressure. The resulting residue was purified by silica gel column chromatography (Eluting solvent; chloroform:methanol = 100:0 to 90:10) to give Reference Example 3 (254 mg).
¹H-NMR (300 MHz, CDCl₃) δ: 8.03-7.99 (m, 2H), 7.69 (s, 1H), 7.50-7.40 (m, 3H), 4.38-4.24 (m, 3H), 2.94-2.86 (m, 2H), 2.24-2.20 (m, 2H), 2.01-1.88 (m, 2H), 1.49 (s, 9H).

### Reference Example 4

### tert-Butyl 4-(5-fluoro-4-phenyl-1H-imidazol-1-yl)piperidine-1-carboxylate (Reference Example 4)

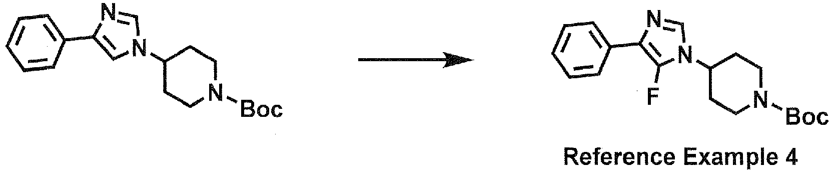

A solution of tert-butyl 4-(4-phenyl-1H-imidazol-1-yl)piperidine-1-carboxylate (250 mg) obtained in a similar manner to Reference Example 1 in tetrahydrofuran (5 ml) was cooled to -78°C, and then thereto was added n-butyllithium/hexane solution (2.69 mol/l: 0.30 ml), and the mixture was stirred at -78°C for 20 minutes. Then, to the reaction mixture was added a solution of tert-butyldimethylchlorosilane (121 mg) in tetrahydrofuran (5 ml) dropwise at-78°C, and then the mixture was warmed to room temperature and stirred for 4 hours. The mixture was cooled again to -78°C, and then thereto was added n-butyllithium/hexane solution (2.69 mol/l: 0.30 ml), and the mixture was stirred at -78°C for 1 hour. Then, to the reaction mixture was added a solution of N-fluorobenzenesulfonimide (252 mg) in tetrahydrofuran (5 ml) at -78°C, and then the mixture was stirred at -78°C for 1 hour and at room temperature for 1 hour. The reaction was quenched by adding 1 mol/L hydrochloric acid to the reaction solution under ice cooling, which was then extracted with ethyl acetate. The organic layer was dried over sodium sulfate and the solvent was removed under reduced pressure, and then the resulting residue was purified by silica gel column chromatography (Eluting solvent; chloroform:methanol = 100:0 to 90:10) to give Reference Example 4 (18 mg).
¹H-NMR (300 MHz, CDCl₃) δ: 7.78-7.76 (m, 2H), 7.43-7.38 (m, 2H), 7.30 (s, 1H), 7.27-7.22 (m, 1H), 4.35-4.31 (m, 2H), 4.14-4.03 (m, 1H), 2.90-2.82 (m, 2H), 2.13-2.08 (m, 2H), 1.96-1.83 (m, 2H), 1.49 (s, 9H).

### Reference Example 5

### tert-Butyl 4-(2-fluoro-4-phenyl-1H-imidazol-1-yl)piperidine-1-carboxylate (Reference Example 5)

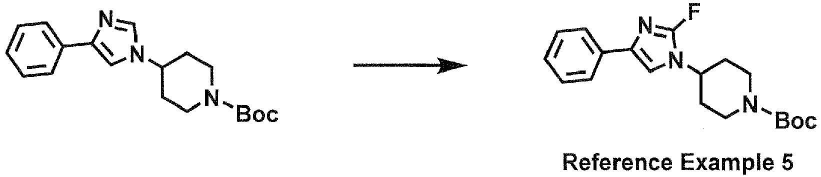

A solution of tert-butyl 4-(4-phenyl-1H-imidazol-1-yl)piperidine-1-carboxylate (160 mg) obtained in a similar manner to Reference Example 1 in tetrahydrofuran (5 ml) was cooled to -78°C, and then n-butyllithium/hexane solution (2.69 mol/l: 0.19 ml) was added thereto, and the mixture was stirred at -78°C for 30 minutes. Then, to the mixture was added a solution of N-fluorobenzenesulfonimide (161 mg) in tetrahydrofuran (5 ml) dropwise at -78°C, and then the mixture was stirred at -78°C for 1 hour and at room temperature for 15 hours. The reaction was quenched by adding aqueous saturated ammonium chloride to the reaction solution under ice cooling, which was then extracted with ethyl acetate. The organic layer was dried over sodium sulfate and the solvent was removed under reduced pressure, and then the resulting residue was purified by silica gel column chromatography (Eluting solvent; chloroform:methanol = 100:0 to 90:10) to give Reference Example 5 (10 mg).
¹H-NMR (300 MHz, CDCl₃) δ: 7.90-7.76 (m, 2H), 7.42-7.36 (m, 3H), 7.23 (s, 1H), 4.35-4.31 (m, 2H), 4.13-4.02 (m, 1H), 2.90-2.82 (m, 2H), 2.13-2.09 (m, 2H), 1.96-1.82 (m, 2H), 1.49 (s, 9H).

### Reference Example 6

### cis-4-(4-Phenyl-1H-imidazol-1-yl)cyclohexanecarboxylic acid (Reference Example 6)

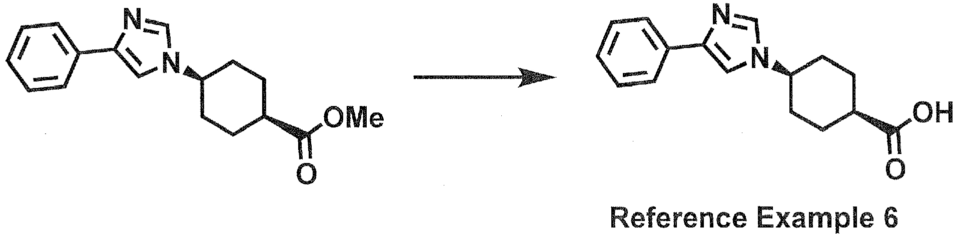

To a solution of methyl cis-4-(4-phenyl-1H-imidazol-1-yl)cyclohexanecarboxylate (280 mg) obtained in a similar manner to Reference Example 1 in methanol/tetrahydrofuran (2.5 ml/5 ml) was added 2 mol/l aqueous sodium hydroxide (2.5 ml) at room temperature, and then the mixture was stirred at room temperature for 3 hours. The mixture was concentrated under reduced pressure, and then to the resulting residue were added chloroform and 1 mol/l hydrochloric acid. The mixture was extracted with chloroform five times, and then the combined organic layer was dried over sodium sulfate and concentraterd under reduced pressure to give Reference Example 6 (146 mg).
LCMS; [M+H]⁺ / Rt (min): Measurement Condition (271 / 0.39: B)

### Example 1

### N-Cyclohexyl-4-[4-(3-fluorophenyl)-1H-imidazol-1-yl]piperidine-1-carboxamide (Example 1)

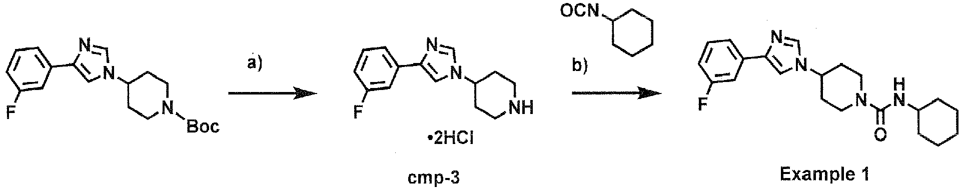

### a) Preparation of 4-[4-(3-fluorophenyl)-1H-imidazol-1-yl]piperidine hydrochloride (Compound cmp-3)

To Reference Example 1 (5.63 g) was added 4 mol/L hydrogen chloride/ethyl acetate solution (50 ml) at room temperature, and the mixture was stirred at room temperature for 30 minutes. The reaction solution was concentrated under reduced pressure to give Compound cmp-3 (4.06 g).
LCMS; [M+H]⁺ / Rt (min): Measurement Condition (246 / 0.18: A)

### b) Preparation of N-cyclohexyl-4-[4-(3-fluorophenyl)-1H-imidazol-1-yl]piperidine-1-carboxamide (Example 1)

To a solution of Compound cmp-3 (1 g) in tetrahydrofuran (15 ml) were added cyclohexyl isocyanate (0.48 ml) and triethylamine (1.3 ml) at room temperature, and the mixture was stirred at room temperature for 18 hours. The reaction solution was concentrated under reduced pressure, and then the resulting residue was purified by silica gel column chromatography (Eluting solvent; chloroform:methanol = 100:0 to 90:10) to give Example 1 (960 mg).
LCMS; [M+H]⁺ / Rt (min): Measurement Condition (371 / 0.63: A)
¹H-NMR (400 MHz, MeOD) δ : 7.62 (s, 1H), 7.52-7.45 (m, 2H), 7.33 (s, 1H), 6.93 (s, 1H), 6.73 (s, 1H), 4.36 (s, 1H), 4.15-4.11 (m, 3H), 3.67 (s, 1H), 2.96-2.89 (m, 2H), 2.15-2.13 (m, 2H), 1.97-1.90 (m, 2H), 1.71-1.62 (m, 4H), 1.40-1.37 (m, 2H), 1.14-1.11 (m, 4H)

### Example 2

### N-(Bicyclo[2.2.1]hept-2-yl)-4-[4-(3-fluorophenyl)-1H-imidazol-1-yl]piperidine-1-carboxamide (Example 2)

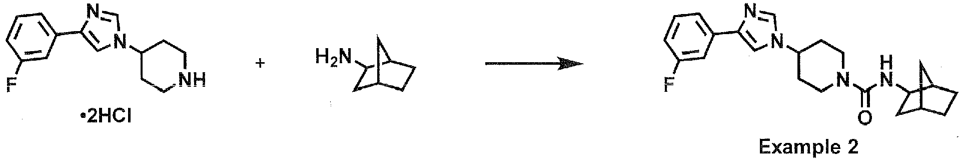

To a solution of exo-2-aminonorbornene (0.027 ml) in tetrahydrofuran (0.5 ml) were added triphosgene (27 mg) and triethylamine (0.064 ml) under ice cooling, and the mixture was stirred for 1 hour under ice cooling. Then, to the reaction mixture were added a solution of Compound cmp-3 (53 mg) in tetrahydrofuran (0.5 ml) and triethylamine (0.082 ml) under ice cooling, followed by adding a small amount of water thereto so as to be homogenized, and then the mixture was stirred at room temperature for 2 hours. The reaction solution was concentrated under reduced pressure, and then the resulting residue was purified by silica gel column chromatography to give Example 2 (14 mg).
LCMS; [M+H]⁺ / Rt (min): Measurement Condition (383 / 0.65: A)

### Example 3

### 4-[4-(3-Fluorophenyl)-1H-imidazol-1-yl]-N-[trans-4-(trifluoromethyl)cyclohexyl]piperidine-1-carboxamide (Example 3)

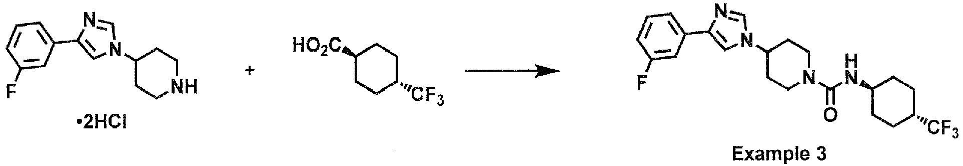

To a solution of trans-4-trifluoromethylcyclohexanecarboxylic acid (0.045 ml) in toluene (5 ml) were added diphenylphosphoryl azide (0.137 ml) and triethylamine (0.096 ml), and the mixture was stirred for 3 hours with heating at 95°C. Then, the mixture was cooled to room temperature, and thereto were added a solution of Compound cmp-3 (53 mg) in tetrahydrofuran and triethylamine (0.082 ml), followed by adding a small amount of water so as to be homogenized, and then the mixture was stirred at room temperature for 2 hours. The reaction solution was concentrated under reduced pressure, and then the resulting residue was purified by silica gel column chromatography to give Example 3 (22 mg).
LCMS; [M+H]⁺ / Rt (min): Measurement Condition (439 / 0.70: A)

### Example 4

### N-(trans-4-Methoxycyclohexyl)-4-{4-[4-(trifluoromethyl)phenyl]-1H-imidazol-1-yl}piperidine-1-carboxamide (Example 4)

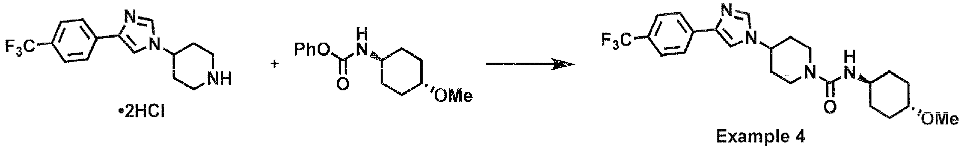

To a solution of 4-{4-[4-(trifluoromethyl)phenyl]-1H-imidazol-1-yl}piperidine hydrochloride (200 mg) obtained in a similar manner to Reference Example 1, Example 1a) in dimethylformamide (6 ml) were added diisopropylethylamine (0.47 ml) and phenyl(trans-4-methoxycyclohexyl)carbamate (136 mg) at room temperature, and the mixture was stirred for 60 hours with heating at 70°C. The reaction solution was concentrated under reduced pressure, and then the resulting residue was purified by silica gel column chromatography (Eluting solvent; chloroform:methanol = 100:0 to 90:10) to give Example 4 (11 mg).
LCMS; [M+H]⁺ / Rt (min): Measurement Condition (451 / 1.27: B)

### Example 5

### N-tert-Butyl-4-[5-chloro-4-(3-fluorophenyl)-1H-imidazol-1-yl]piperidine-1-carboxamide (Example 5)

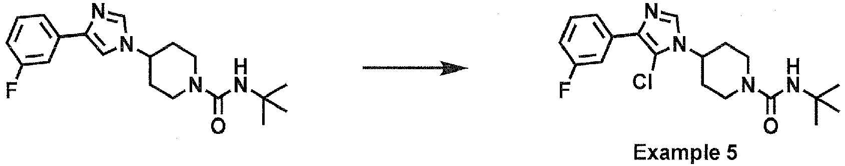

To a solution of N-tert-butyl-4-[4-(3-fluorophenyl)-1H-imidazol-1-yl]piperidine-1-carboxamide (48 mg) obtained in a similar manner to Example 1 in methylene chloride (3 ml) were added N-chlorosuccinimide (29 mg) and trifluoroacetic acid (0.06 ml) at room temperature, and then the mixture was heated to reflux at 50°C for 40 hours. The reaction was quenched by adding aqueous saturated sodium hydrogen carbonate to the reaction solution under ice cooling, which was then extracted with chloroform. The organic layer was dried over sodium sulfate and the solvent was removed under reduced pressure, and then the resulting residue was purified by silica gel column chromatography (Eluting solvent; chloroform:methanol = 100:0 to 90:10) to give Example 5 (9 mg).
LCMS; [M+H]⁺ / Rt (min): Measurement Condition (379 / 1.59: B)
¹H-NMR (300 MHz, CDCl₃) δ: 7.76-7.73 (m, 1H), 7.70-7.65 (m, 1H), 7.61 (s, 1H), 7.41-7.34 (m, 1H), 7.02-6.98 (m, 1H), 4.37 (brs, 1H), 4.26-4.09 (m, 3H), 2.96-2.87 (m, 2H), 2.18-2.13 (m, 2H), 1.96-1.83 (m, 2H), 1.38 (s, 9H).

### Example 6

### [4-(4-Phenyl-1H-imidazol-1-yl)piperidin-1-yl](4,4-difluorocyclohexyl)methanone (Example 6)

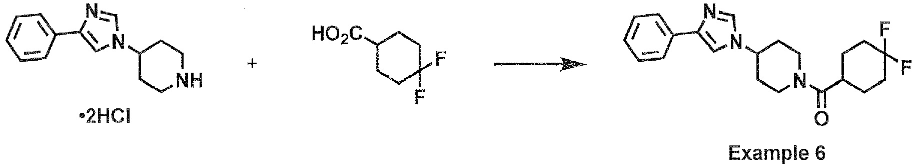

To a solution of 4-(4-phenyl-1H-imidazol-1-yl)piperidine dihydrochloride (300 mg) obtained in a similar manner to Reference Example 1, Example 1a) in methylene chloride (10 ml) were added 4,4-difluorocyclohexylcarboxylic acid (246 mg) and diisopropylethylamine (0.87 ml), HBTU (569 mg) at room temperature, and the mixture was stirred at room temperature for 5 hours. The reaction solution was concentrated under reduced pressure, and then the resulting residue was purified by silica gel column chromatography (Eluting solvent; chloroform:methanol = 100:0 to 90:10) to give Example 6 (370 mg).
LCMS; [M+H]⁺ / Rt (min): Measurement Condition (374 / 0.54: A)

### Example 7

### 1-{1-[(4,4-Difluorocyclohexyl)carbonyl]piperidin-4-yl}-4-phenyl-1H-imidazole-5-carbonitrile (Example 7)

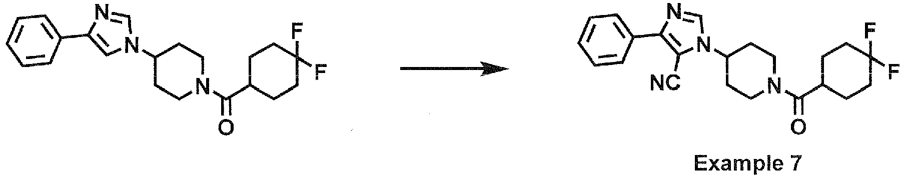

To a solution of Example 6 (370 mg) in methylene chloride (12 ml) were added N-iodosuccinimide (391 mg) and trifluoroacetic acid (0.52 ml) at room temperature, and then the mixture was stirred at room temperature under protecting from light for 18 hours. The reaction was quenched by adding aqueous sodium thiosulfate to the reaction solution, which was then extracted with chloroform. The organic layer was dried over sodium sulfate and the solvent was removed under reduced pressure, and then to the residue were added dimethylformamide (6 ml), copper iodide (67 mg), and potassium cyanide (113 mg) at room temperature, and the mixture was stirred for 15 hours with heating at 150°C. The reaction solution was concentrated under reduced pressure, and then the resulting residue was purified by silica gel column chromatography (Eluting solvent; chloroform:methanol = 100:0 to 90:10) to give Example 7 (269 mg).
LCMS; [M+H]⁺ / Rt (min): Measurement Condition (399 / 1.49: B) ¹H-NMR (300 MHz, CDCl₃) δ: 8.03-7.99 (m, 2H), 7.68 (s, 1H), 7.50-7.38 (m, 3H), 4.92-4.89 (m, 1H), 4.41-4.36 (m, 1H), 4.11-4.07 (m, 1H), 3.30-3.26 (m, 1H), 2.69-2.60 (m, 2H), 2.30-1.67 (m, 12H).

### Example 8

### 2-Fluoro-N-{cis-4-[4-(4-fluorophenyl)-1H-imidazol-1-yl]cyclohexyl}-2-methylpropanamide (Example 8)

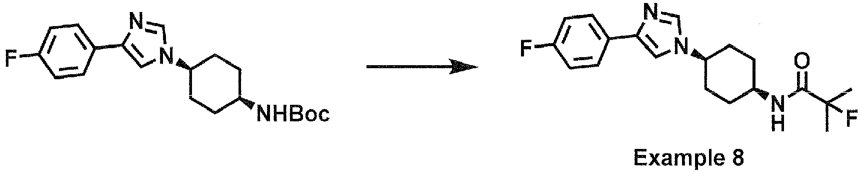

To a solution of tert-butyl{cis-4-[4-(4-fluorophenyl)-1H-imidazol-1-yl]cyclohexyl}carbamate (2.89 g) obtained in a similar manner to Reference Example 1 in methanol (20 ml) was added 4 mol/L hydrogen chloride/dioxane solution (5 ml) at room temperature, and then the mixture was stirred at room temperature for 5 hours. The reaction solution was concentrated under reduced pressure, and then to the residue were added dichloromethane (40.2 ml), diisopropylethylamine (7.00 ml), HBTU (4.57 g), and 2-fluoro-2-methylpropionic acid (1.28 g) at room temperature, and the mixture was stirred at room temperature for 3 hours. The reaction solution was concentrated under reduced pressure, and then the resulting residue was purified by aminosilica gel column chromatography (Eluting solvent; hexane:chloroform = 20:80 to 0:100) to give Example 8 (2.86 g).
¹H-NMR (400 MHz, CDCl₃) δ: 7.70 (dd, J = 9, 5.5 Hz, 2H), 7.56 (s, 1H), 7.19 (s, 1H), 7.03 (t, J = 9 Hz, 2H), 6.46 (br, 1H), 4.00-4.12 (m, 2H), 2.01-2.10 (m, 2H), 1.74-1.98 (m, 6H), 1.58 (s, 3H), 1.52 (s, 3H).

### Example 9

### cis-N-Cyclohexyl-4-(4-phenyl-1H-imidazol-1-yl)cyclohexanecarboxamide (Example 9)

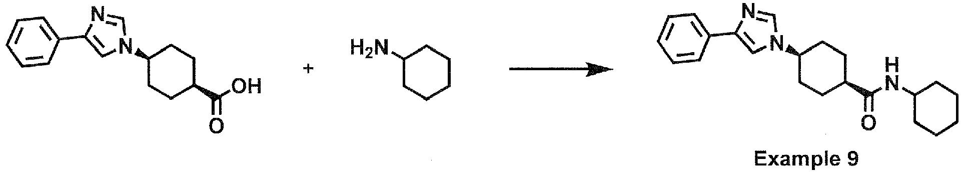

To a solution of Reference Example 6 (49 mg) in dimethylformamide (3 ml) were added cyclohexylamine (41 mg), WSC·HCl (69 mg), HOBt (49 mg), and triethylamine (0.15 ml) at room temperature, and then the mixture was stirred at room temperature for 72 hours. The mixture was concentrated under reduced pressure, and then the resulting residue was purified by silica gel column chromatography (Eluting solvent; chloroform:methanol = 100:0 to 90:10) to give Example 9 (23 mg).
¹H-NMR (300 MHz, CDCl₃) δ: 7.78-7.75 (m, 2H), 7.59-7.58 (m, 1H), 7.39-7.19 (m, 4H), 5.35-5.32 (m, 1H), 4.06-3.97 (m, 1H), 3.84-3.73 (m, 1H), 2.44-2.42 (m, 1H), 2.31-2.18 (m, 2H), 2.13-1.06 (m, 16H).

### Examples 10 to 137

Compounds listed in Table 1 were obtained by using corresponding starting compounds according to the methods of Reference Examples 1 to 5 and Examples 1 to 5 or 7.

**[Table 1]**

| | | | | | |
|---|---|---|---|---|---|
| 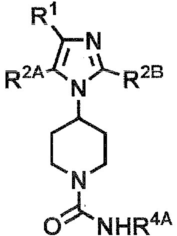 | | | | | |

| Example | R¹ | R^{2A} | R^{2B} | R^{4A} | LCMS; [M+H]⁺ / Rt (min): Measurement Condition |
|---|---|---|---|---|---|
| 10 | 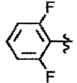 | H | H | 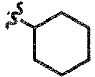 | 389 / 0.58 : A |
| 11 | 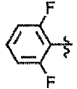 | H | H | 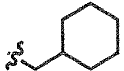 | 403 / 0.65 : A |
| 12 | 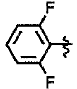 | H | H | 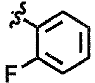 | 401 / 0.54 : A |
| 13 | 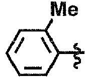 | H | H | 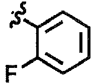 | 379 / 0.56 : A |
| 14 | 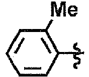 | H | H | 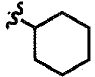 | 367 / 0.58 : A |
| 15 | 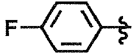 | H | H | 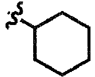 | 371 / 0.60 : A |
| 16 | 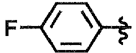 | H | H | 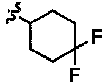 | 407 / 0.57 : A |
| 17 | 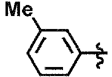 | H | H | 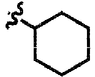 | 367 / 0.60 : A |
| 18 | 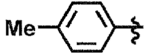 | H | H | 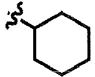 | 367 / 0.60 : A |
| 19 | 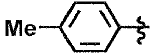 | H | H | 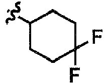 | 402 / 0.61 : A |
| 20 | 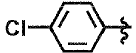 | H | H | 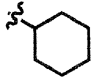 | 387 / 0.67 : A |
| 21 | 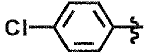 | H | H | 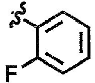 | 399 / 0.64 : A |
| 22 | 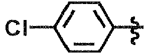 | H | H | 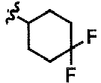 | 423 / 0.65 : A |
| 23 | 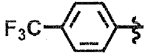 | H | H | 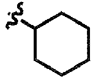 | 421 / 0.78 : A |
| 24 | 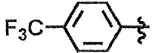 | H | H | 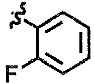 | 433 / 0.75 : A |
| 25 | 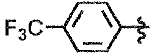 | H | H | 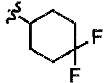 | 457 / 0.75 : A |
| 26 | 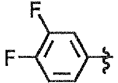 | H | H | 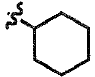 | 389 / 0.69 : A |
| 27 | 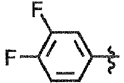 | H | H | 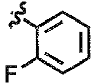 | 401 / 0.66 : A |
| 28 | 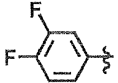 | H | H | 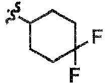 | 425 / 0.67 : A |
| 29 | 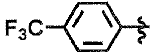 | H | H | 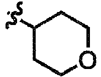 | 423 / 0.59 : A |
| 30 | 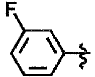 | H | H | 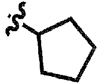 | 357 / 0.61 : A |
| 31 | 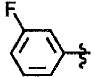 | H | H | 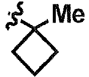 | 357 / 0.61 : A |
| 32 | 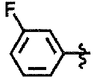 | H | H | 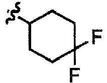 | 407 / 0.64 : A |
| 33 | 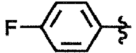 | H | H | 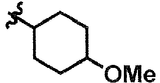 | 401 / 0.51 : A |
| 34 | 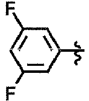 | H | H | 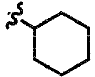 | 389 / 0.76 : A |
| 35 | 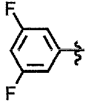 | H | H | 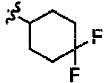 | 425 / 0.74 : A |
| 36 | 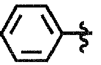 | H | H | 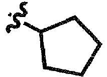 | 339 / 0.50 : A |
| 37 | 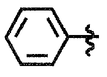 | H | H | 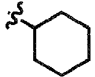 | 353 / 0.54 : A |
| 38 | 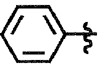 | H | H | 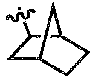 | 365 / 0.57 : A |
| 39 | 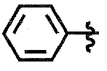 | H | H | 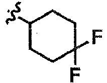 | 389 / 0.53 : A |
| 40 | 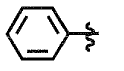 | H | H | 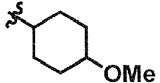 | 383 / 0.48 : A |
| 41 | 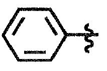 | H | H | 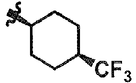 | 421 / 0.62 : A |
| 42 | 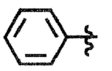 | H | H | 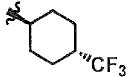 | 421 / 0.61 : A |
| 43 | 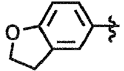 | H | H | 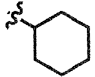 | 395 / 0.56 : A |
| 44 | 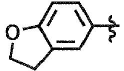 | H | H | 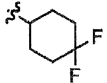 | 431 / 0.54 : A |
| 45 | 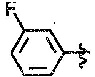 | H | H | 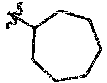 | 385 / 0.68 : A |
| 46 | 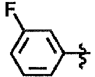 | H | H | 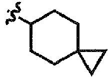 | 397 / 0.70 : A |
| 47 | 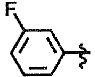 | H | H | 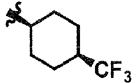 | 439 / 0.69 : A |
| 48 | 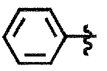 | H | H | 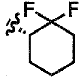 | 389 / 0.53 : A |
| 49 | 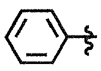 | H | H | 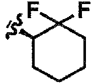 | 389 / 0.53 : A |
| 50 | 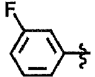 | H | H | 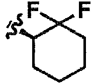 | 407 / 0.59 : A |
| 51 | 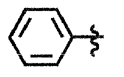 | H | H | 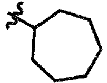 | 367 / 0.60 : A |
| 52 | 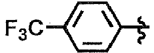 | H | H | 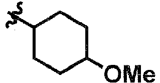 | 451 / 0.66, 0.67 : A |
| 53 | 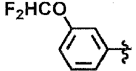 | H | H | 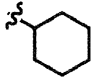 | 419 / 0.67 : A |
| 54 | 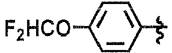 | H | H | 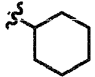 | 419/0.64:A |
| 55 | 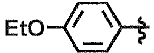 | H | H | 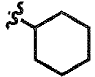 | 397 / 0.61 : A |
| 56 | 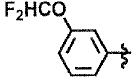 | H | H | 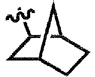 | 431 / 0.69 : A |
| 57 | 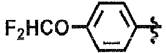 | H | H | 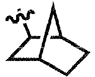 | 431 / 0.66 : A |
| 58 | 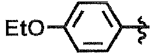 | H | H | 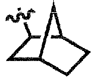 | 409 / 0.62 : A |
| 59 | 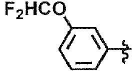 | H | H | 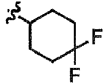 | 455 / 0.66 : A |
| 60 | 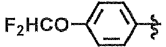 | H | H | 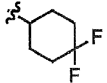 | 455 / 0.62 : A |
| 61 | 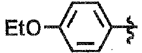 | H | H | 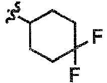 | 433 / 0.59 : A |
| 62 | 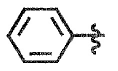 | H | H | 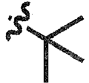 | 328 / 2.68 : C |
| 63 | 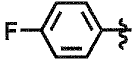 | H | H | 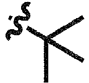 | 345 / 0.55 : B |
| 64 | 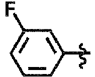 | H | H | 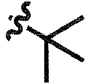 | 345 / 0.67 : B |
| 65 | 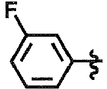 | H | H | 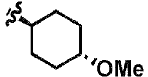 | 401 / 0.77 : B |
| 66 | 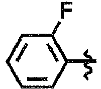 | H | H | 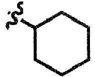 | 371 / 1.30 : B |
| 67 | 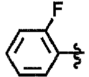 | H | H | 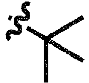 | 345 / 0.80 : B |
| 68 | 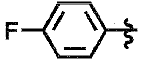 | H | H | 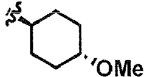 | 401 / 0.61 : B |
| 69 | 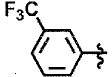 | H | H | 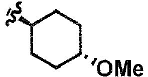 | 451 / 1.20 : B |
| 70 | 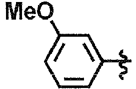 | H | H | 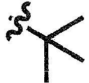 | 357 / 0.65 : A |
| 71 | 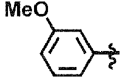 | H | H | 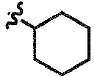 | 383 / 0.66 : A |
| 72 | 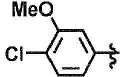 | H | H | 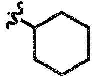 | 417 / 0.73 : A |
| 73 | 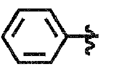 | Cl | H | 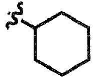 | 387 / 0.93 : A |
| 74 | 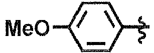 | Cl | H | 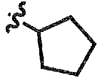 | 403 / 0.59 : A |
| 75 | 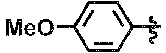 | Cl | H | 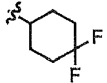 | 453 / 0.88 : A |
| 76 | 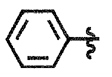 | Cl | H | 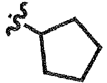 | 373 / 0.88 : A |
| 77 | 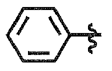 | Cl | H | 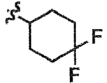 | 423 / 0.91 :A |
| 78 | 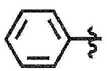 | Cl | H | 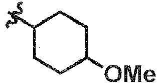 | 417 / 0.83 : A |
| 79 | 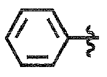 | Cl | H | 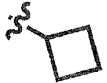 | 359 / 0.52 : A |
| 80 | 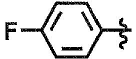 | Cl | H | 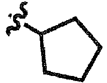 | 391 / 0.94 : A |
| 81 | 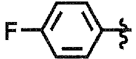 | Cl | H | 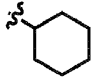 | 405 / 0.97 : A |
| 82 | 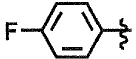 | Cl | H | 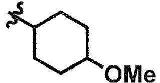 | 435 / 0.86 : A |
| 83 | 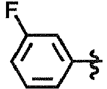 | Cl | H | 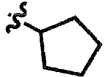 | 391 / 0.97 : A |
| 84 | 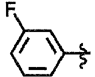 | Cl | H | 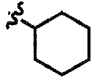 | 405 / 1.03 : A |
| 85 | 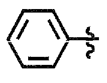 | Cl | H | 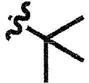 | 362 / 3.58 : C |
| 86 | 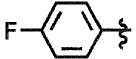 | Cl | H | 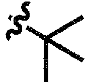 | 379 / 1.63 : B |
| 87 | 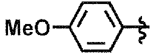 | Cl | H | 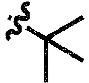 | 391 / 1.55 : B |
| 88 | 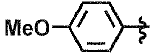 | Cl | H | 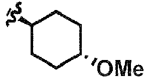 | 447 / 1.52 : B |
| 89 | 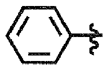 | Cl | H | 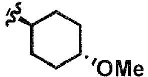 | 417 / 1.43 : B |
| 90 | 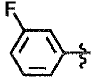 | Cl | H | 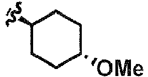 | 435 / 1.51 : B |
| 91 | 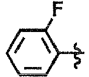 | Cl | H | 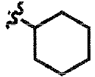 | 405 / 1.63 : B |
| 92 | 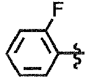 | Cl | H | 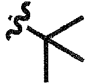 | 379 / 1.47 : B |
| 93 | 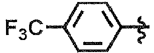 | Cl | H | 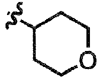 | 457 / 1.64 : B |
| 94 | 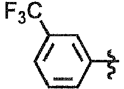 | Cl | H | 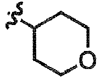 | 457 / 1.63 : B |
| 95 | 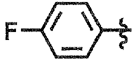 | Cl | H | 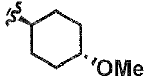 | 435 / 1.56 : B |
| 96 | 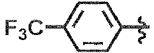 | Cl | H | 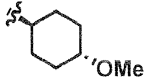 | 485 / 1.68 : B |
| 97 | 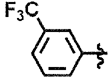 | Cl | H | 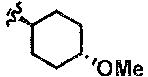 | 485 / 1.67 : B |
| 98 | 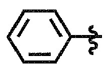 | CN | H | 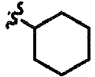 | 379 / 4.36 : C |
| 99 | 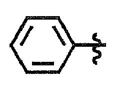 | CN | H | 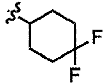 | 414 / 0.87 : A |
| 100 | 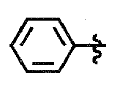 | CN | H | 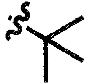 | 352 / 1.50 : C |
| 101 | 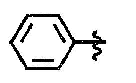 | Br | H | 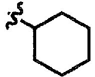 | 432 / 0.92 : A |
| 102 | 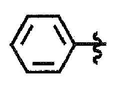 | Me | H | 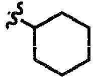 | 367 / 0.53 : A |
| 103 | 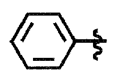 | Et | H | 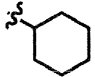 | 381 / 0.88 :A |
| 104 | 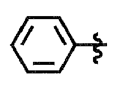 | Br | H | 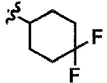 | 468 / 0.89 : A |
| 105 | 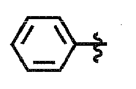 | Me | H | 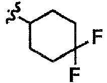 | 403 / 0.51 : A |
| 106 | 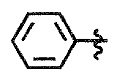 | F | H | 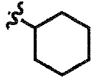 | 372 / 3.89 : C |
| 107 | 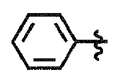 | 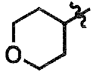 | H | 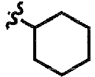 | 438 / 2.93 : C |
| 108 | 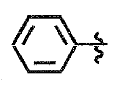 | 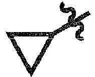 | H | 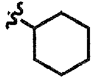 | 393 / 0.63 : B |
| 109 | 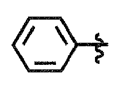 | 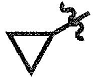 | H | 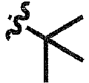 | 367 / 0.41 : B |
| 110 | 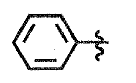 | 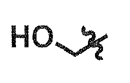 | H | 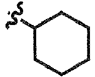 | 383 / 0.54 : B |
| 111 | 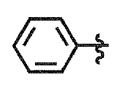 | 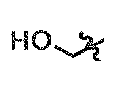 | H | 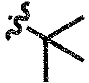 | 357 / 0.40 : B |
| 112 | 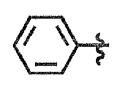 | 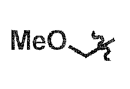 | H | 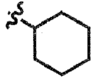 | 397 / 0.76 : B |
| 113 | 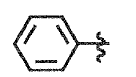 | 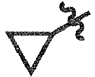 | H | 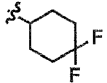 | 429 / 0.54 : B |
| 114 | 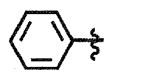 | H | Me | 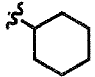 | 367 / 0.56 : A |
| 115 | 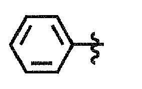 | H | F | 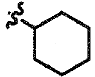 | 371 / 3.90 : C |
| 116 | 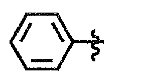 | H | Cl | 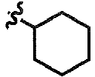 | 387 / 4.37 : C |
| 117 | 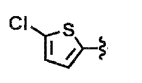 | H | H | 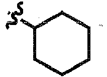 | 393 / 0.80 : A |
| 118 | 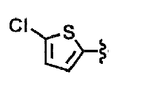 | H | H | 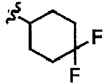 | 429 / 0.77 : A |
| 119 | 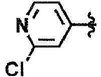 | H | H | 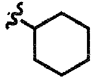 | 388 / 0.66 : A |
| 120 | 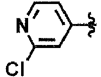 | H | H | 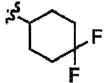 | 424 / 0.66 : A |
| 121 | 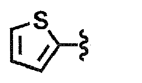 | H | H | 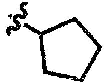 | 345 / 0.55 : A |
| 122 | 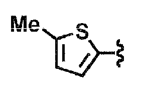 | H | H | 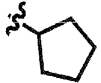 | 359 / 0.59 : A |
| 123 | 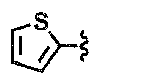 | H | H | 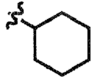 | 359 / 0.61 : A |
| 124 | 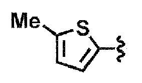 | H | H | 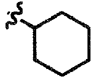 | 373 / 0.65 : A |
| 125 | 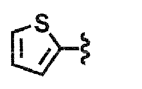 | H | H | 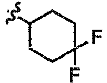 | 395 / 0.58 : A |
| 126 | 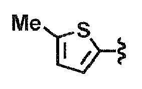 | H | H | 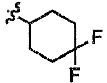 | 409 / 0.62 : A |
| 127 | 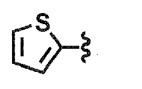 | H | H | 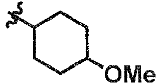 | 389 / 0.51 : A |
| 128 | 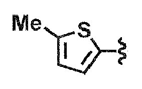 | H | H | 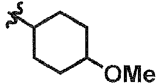 | 403 / 0.55 : A |
| 129 | 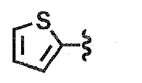 | H | H | 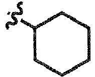 | 359 / 0.52 : A |
| 130 | 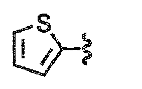 | H | H | 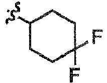 | 394 / 0.64 : A |
| 131 | 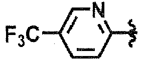 | H | H | 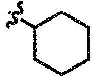 | 422 / 0.79 : A |
| 132 | 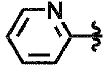 | H | H | 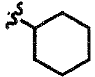 | 354 / 0.85 : B |
| 133 | 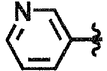 | H | H | 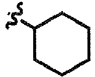 | 354 / 0.58 : B |
| 134 | 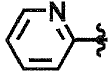 | Cl | H | 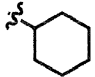 | 388 / 1.07 : B |
| 135 | 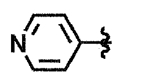 | Cl | H | 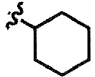 | 388 / 1.25 : B |
| 136 | 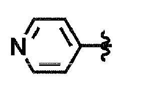 | Cl | H | 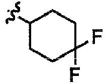 | 424 / 0.73 : B |
| 137 | 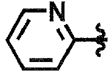 | Cl | H | 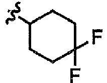 | 424 / 0.98 : B |

### Examples 138 to 160

Compounds listed in Table 2 were obtained by using corresponding starting compounds according to the methods of Reference Example 1 or 3, and Examples 5 to 7.

**[Table 2]**

| | | | | |
|---|---|---|---|---|
| 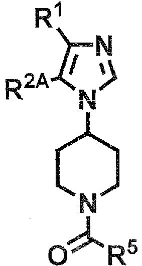 | | | | |

| Example | R¹ | R^{2A} | R⁵ | LCMS; [M+H]⁺ / Rt (min): Measurement Condition |
|---|---|---|---|---|
| 138 | 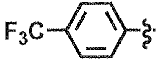 | H | 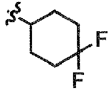 | 442 / 0.78 : A |
| 139 | 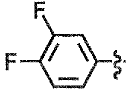 | H | 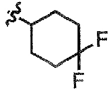 | 410 / 0.68 : A |
| 140 | 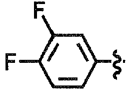 | H | 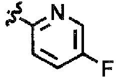 | 387 / 0.57: A |
| 141 | 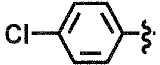 | H | 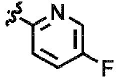 | 385 / 0.58 : A |
| 142 | 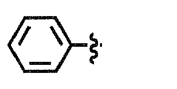 | H | 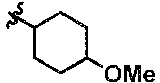 | 368 / 0.47,0.49 : A |
| 143 | 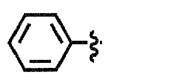 | H | 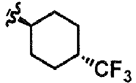 | 406 / 0.64 : A |
| 144 | 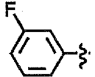 | H | 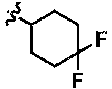 | 392 / 0.63 : A |
| 145 | 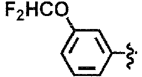 | H | 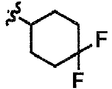 | 440 / 0.68 : A |
| 146 | 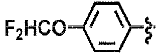 | H | 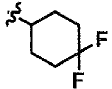 | 440 / 0.68 : A |
| 147 | 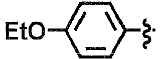 | H | 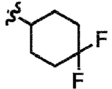 | 418 / 0.60 : A |
| 148 | 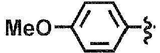 | Cl | 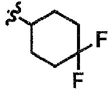 | 438 / 0.91 : A |
| 149 | 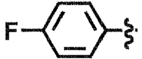 | Cl | 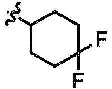 | 426 / 0.98 : A |
| 150 | 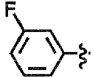 | Cl | 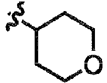 | 392 / 0.86 : A |
| 151 | 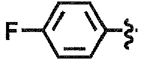 | Cl | 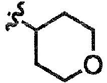 | 392 / 0.80 : A |
| 152 | 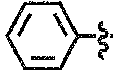 | Cl | 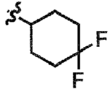 | 408 / 0.95 : A |
| 153 | 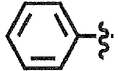 | CN | 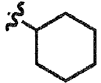 | 363 / 1.62 : B |
| 154 | 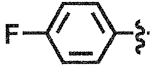 | CN | 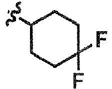 | 417 / 1.57 : B |
| 155 | 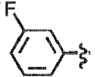 | CN | 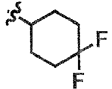 | 417 / 1.47 : B |
| 156 | 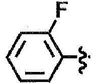 | CN | 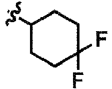 | 417 / 1.31 : B |
| 157 | 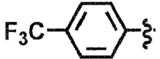 | CN | 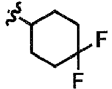 | 467 / 1.69 : B |
| 158 | 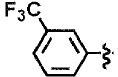 | CN | 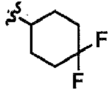 | 467 / 1.62 : B |
| 159 | 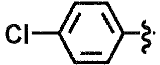 | CN | 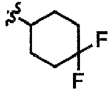 | 433 / 1.64 : B |
| 160 | 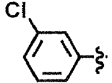 | CN | 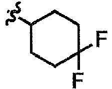 | 433 / 1.59 : B |

### Examples 161 to 203

Compounds listed in Table 3 were obtained by using corresponding starting compounds according to the methods of Reference Example 1 or 5, and Example 5, 7 or 8.

**[Table 3]**

| | | | | |
|---|---|---|---|---|
| 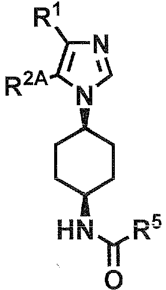 | | | | |

| Example | R¹ | R^{2A} | R⁵ | LCMS; [M+H]⁺ / Rt (min): Measurement Condition |
|---|---|---|---|---|
| 161 | 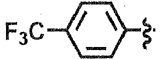 | H | 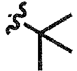 | 394 / 0.76 : A |
| 162 | 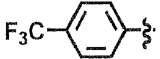 | H | 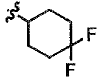 | 456 / 0.77 : A |
| 163 | 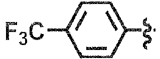 | H | 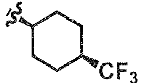 | 488 / 0.87 : A |
| 164 | 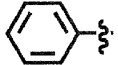 | H | 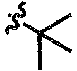 | 327 / 2.96 : C |
| 165 | 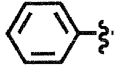 | H | 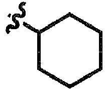 | 353 / 3.32 : C |
| 166 | 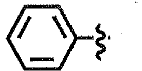 | H | 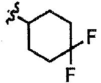 | 389 / 1.20 : C |
| 167 | 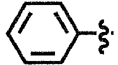 | Cl | 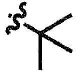 | 360 / 3.84 : C |
| 168 | 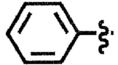 | Cl | 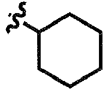 | 386 / 4.19 : C |
| 169 | 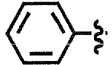 | Cl | 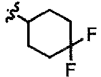 | 422 / 3.40 : C |
| 170 | 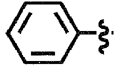 | Cl | 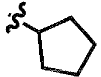 | 372 / 1.75 : B |
| 171 | 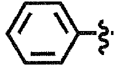 | Cl | 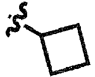 | 358 / 1.60 : B |
| 172 | 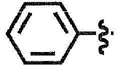 | Cl | 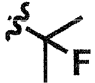 | 364 / 1.64 : B |
| 173 | 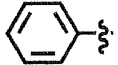 | Cl | 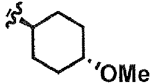 | 416 / 1.68 : B |
| 174 | 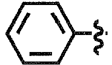 | Cl | 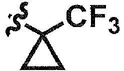 | 412 / 1.69 : B |
| 175 | 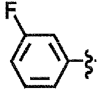 | Cl | 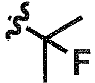 | 382 / 1.70 : B |
| 176 | 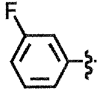 | Cl | 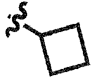 | 376 / 0.99 : A |
| 177 | 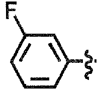 | Cl | 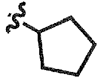 | 390 / 1.05 : A |
| 178 | 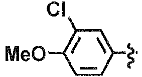 | Cl | 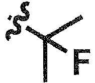 | 428 / 1.74 : B |
| 179 | 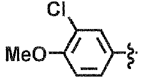 | Cl | 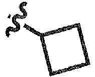 | 422 / 1.00 : A |
| 180 | 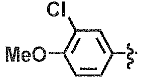 | Cl | 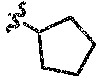 | 436 / 1.05 : A |
| 181 | 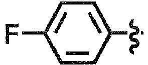 | Cl | 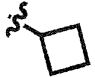 | 376 / 0.96 : A |
| 182 | 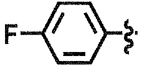 | Cl | 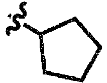 | 390 / 1.0 : A |
| 183 | 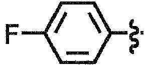 | Cl | 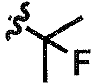 | 382 / 1.65 : B |
| 184 | 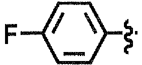 | Cl | 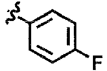 | 416 / 1.71 : B |
| 185 | 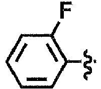 | Cl | 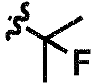 | 382 / 0.91 : A |
| 186 | 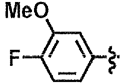 | Cl | 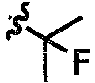 | 412 / 0.99 : A |
| 187 | 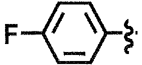 | Cl | 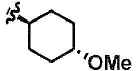 | 434 / 0.94 : A |
| 188 | 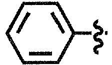 | Cl | 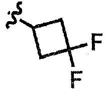 | 394 / 1.68 : B |
| 189 | 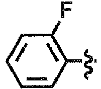 | Cl | 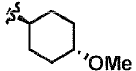 | 434 / 1.62 : B |
| 190 | 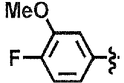 | Cl | 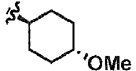 | 464 / 1.70 : B |
| 191 | 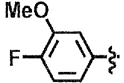 | Cl | 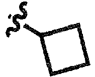 | 406 / 1.68 : B |
| 192 | 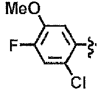 | Cl | 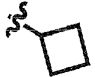 | 440 / 1.74 : B |
| 193 | 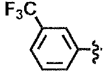 | Cl | 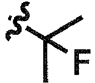 | 432 / 1.83 : B |
| 194 | 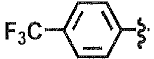 | Cl | 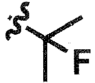 | 432 / 1.84 : B |
| 195 | 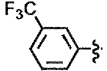 | Cl | 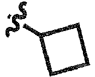 | 426 / 1.86 : B |
| 196 | 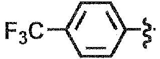 | Cl | 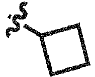 | 426 / 1.91 : B |
| 197 | 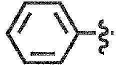 | CN | 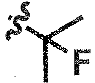 | 355 / 1.52 :B |
| 198 | 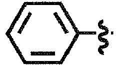 | CN | 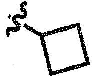 | 349 / 1.54 : B |
| 199 | 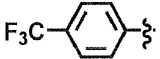 | CN | 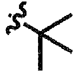 | 419 / 1.80 : B |
| 200 | 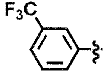 | CN | 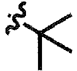 | 419 / 1.79 : B |
| 201 | 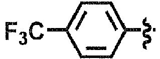 | CN | 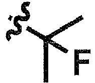 | 423 / 1.73 : B |
| 202 | 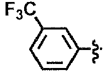 | CN | 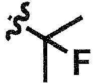 | 423 / 1.68 : B |
| 203 | 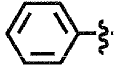 | CN | 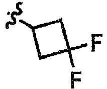 | 385 / 1.54 : B |

### Examples 204 to 207

Compounds listed in Table 4 were obtained by using corresponding starting compounds according to the methods of Reference Example 1, and Example 5 or 8.

**[Table 4]**

| | | | | |
|---|---|---|---|---|
| 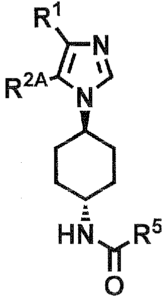 | | | | |

| Example | R¹ | R^{2A} | R⁵ | LCMS; [M+H]⁺ / Rt (min) : Measurement Condition |
|---|---|---|---|---|
| 204 | 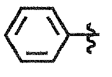 | H | 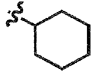 | 352 / 0.75 : B |
| 205 | 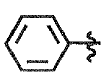 | H | 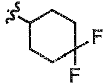 | 388 / 0.47 : B |
| 206 | 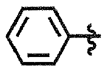 | Cl | 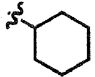 | 386 / 1.75 : B |
| 207 | 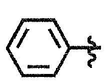 | Cl | 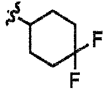 | 422 / 1.60 : B |

### Examples 208 to 230

Compounds listed in Table 5 were obtained by using corresponding starting compounds according to the methods of Reference Example 1 or 6, and Example 5, 7 or 9.

**[Table 5]**

| | | | | | |
|---|---|---|---|---|---|
| 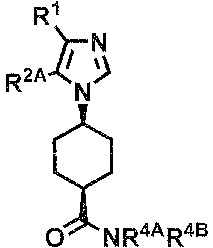 | | | | | |

| Example | R¹ | R^{2A} | R^{4A} | R^{4B} | LCMS; [M+H]⁺ / Rt (min): Measurement Condition |
|---|---|---|---|---|---|
| 208 | 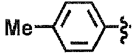 | H | 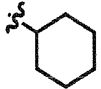 | H | 366 / 0.64 : A |
| 209 | 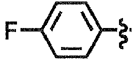 | H | 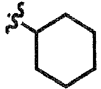 | H | 370 / 0.62 : A |
| 210 | 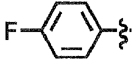 | H | 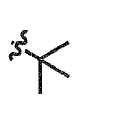 | H | 344 / 0.56 : A |
| 211 | 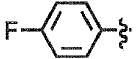 | H | 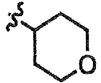 | H | 372 / 0.46 : A |
| 212 | 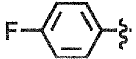 | H | 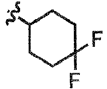 | H | 406 / 0.59 : A |
| 213 | 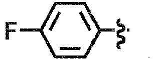 | H | 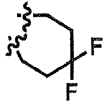 | | 392 / 0.57 : A |
| 214 | 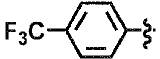 | H | 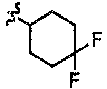 | H | 456 / 0.72 : A |
| 215 | 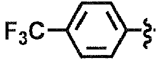 | H | 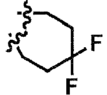 | | 442 / 0.73 : A |
| 216 | 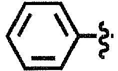 | H | 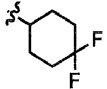 | H | 388 / 0.84 : B |
| 217 | 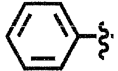 | Cl | 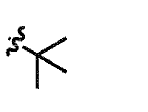 | H | 360 / 0.92 : A |
| 218 | 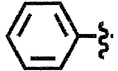 | Cl | 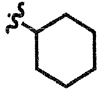 | H | 386 / 4.02 : C |
| 219 | 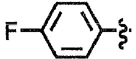 | Cl | 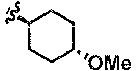 | H | 434 / 1.64 : B |
| 220 | 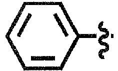 | Cl | 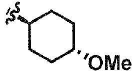 | H | 416 / 1.58 : B |
| 221 | 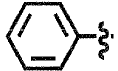 | Cl | 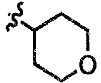 | H | 388 / 1.48 : B |
| 222 | 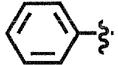 | Cl | 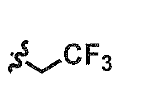 | H | 386 / 1.55 : B |
| 223 | 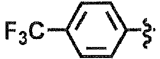 | Cl | 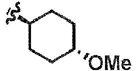 | H | 484 / 1.85 : B |
| 224 | 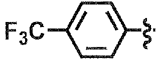 | Cl | 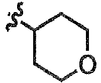 | H | 456 / 1.74 : B |
| 225 | 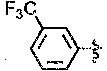 | Cl | 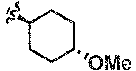 | H | 484 / 1.81 : B |
| 226 | 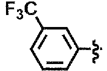 | Cl | 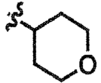 | H | 456 / 1.72 : B |
| 227 | 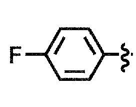 | Cl | 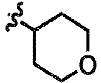 | H | 406 / 1.55 : B |
| 228 | 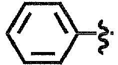 | CN | 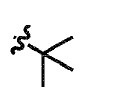 | H | 351 / 1.59 : B |
| 229 | 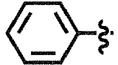 | CN | 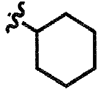 | H | 377 / 1.66 : B |
| 230 | 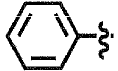 | CN | 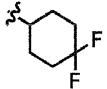 | H | 413 / 1.56 : B |

### Examples 231 to 234

Compounds listed in Table 6 were obtained by using corresponding starting compounds according to the methods of Reference Example 1 or 6, and Example 5 or 9.

**[Table 6]**

| | | | | |
|---|---|---|---|---|
| 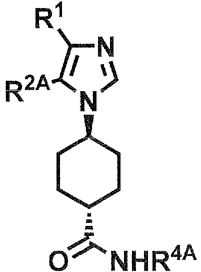 | | | | |

| Example | R¹ | R^{2A} | R^{4A} | LCMS; [M+H]⁺ / Rt (min) : Measurement Condition |
|---|---|---|---|---|
| 231 | 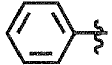 | H | 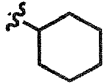 | 352 / 0.60 : B |
| 232 | 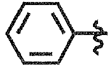 | H | 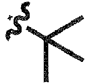 | 326 / 0.50 : B |
| 233 | 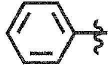 | Cl | 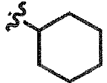 | 386 / 1.78 : B |
| 234 | 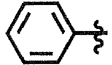 | Cl | 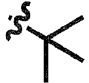 | 360 / 1.66 : B |

### Example 235

### N-[cis-4-(4-Phenyl-1H-imidazol-1-yl)cyclohexyl]-6-(trifluoromethyl)pyrimidin-4-amine (Example 235)

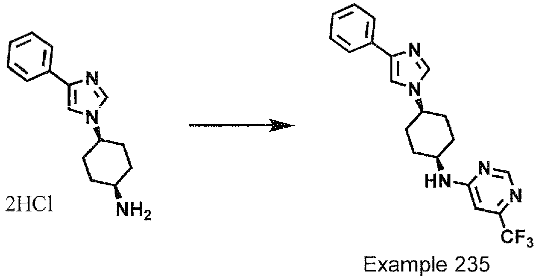

To a solution of cis-4-(4-phenyl-1H-imidazol-1-yl)cyclohexaneamine hydrochloride (55 mg) obtained in a similar manner to Reference Example 1, Example 1a) and diisopropylethylamine (122 µl) in NMP (2 ml) was added 4-chloro-6-trifluoromethylpyrimidine (35 mg), and the mixture was stirred at 50°C for 4 hours. To the reaction solution was added water (50 ml), and the mixture was extracted with ethyl acetate (80 ml × twice). The organic layer was dried over anhydrous magnesium sulfate, and then passed through a filter, and the solvent was removed under reduced pressure. The residue was purified by silica gel column chromatography (Eluting solution: hexane/ethyl acetate = 50:50 to 0:100) to give the titled compound 39 mg.
LCMS; [M+H]⁺ / Rt (min): Measurement Condition (388 / 0.62: A)
¹H-NMR (CDCl₃) δ: 1.71-2.37 (8H, m), 3.20-3.54 (1H, m), 3.85-4.42 (1H, m), 5.58-5.89 (1H, m), 6.65 (1H, s), 7.09-7.39 (3H, m), 7.53 (1H, s), 7.60-7.80 (3H, m), 8.61 (1H, s)

### Examples 236 to 239

Compounds listed in Table 7 were obtained by using corresponding starting compounds according to the method of Example 235.

**[Table 7]**

| | | | |
|---|---|---|---|
| 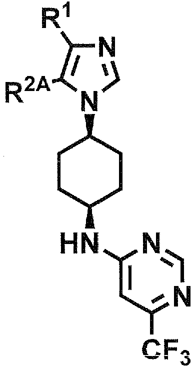 | | | |

| Example | R¹ | R^{2A} | LCMS; [M+H]⁺ / Rt (min) : Measurement Condition |
|---|---|---|---|
| 236 | 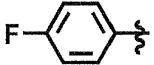 | H | 407 / 0.65 : A |
| 237 | 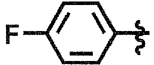 | Cl | 440 / 0.81 : A |
| 238 | 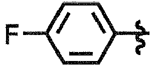 | CN | 432 / 1.12 : A |
| 239 | 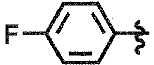 | Me | 420 / 0.74 : A |

### Examples 240 and 241

Compounds listed in Table 8 were obtained by using corresponding starting compounds according to the methods of Reference Example 1 and Examples 1 to 5.

**[Table 8]**

| | | | | |
|---|---|---|---|---|
| 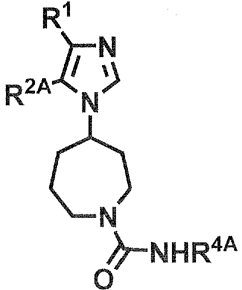 | | | | |

| Example | R¹ | R^{2A} | R^{4A} | LCMS; [M+H]⁺ / Rt (min) : Measurement Condition |
|---|---|---|---|---|
| 240 | 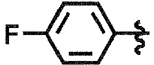 | H | 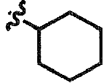 | 385 / 1.14 : B |
| 241 | 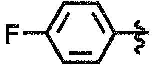 | Cl | 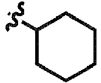 | 419 / 1.90 : B |

### Examples 242 to 243

Compounds listed in Table 9 were obtained by using corresponding starting compounds according to the methods of Reference Example 1 and Example 5 or 6.

**[Table 9]**

| | | | | |
|---|---|---|---|---|
| 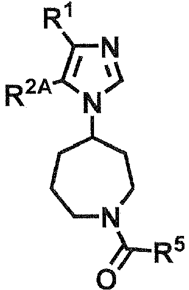 | | | | |

| Example | R¹ | R^{2A} | R⁵ | LCMS; [M+H]⁺ / Rt (min) : Measurement Condition |
|---|---|---|---|---|
| 242 | 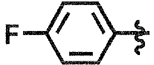 | H | 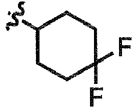 | 406 / 1.18 : B |
| 243 | 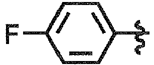 | Cl | 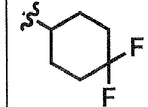 | 440 / 1.79 : B |

### Examples 244 to 259

Compounds listed in Table 10 were obtained by using corresopnding starting compounds according to the methods of Reference Examples 1 to 5 and Examples 1 to 5 or 7.

**[Table 10]**

| | | | | |
|---|---|---|---|---|
| 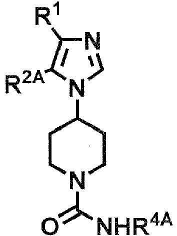 | | | | |

| Example | R¹ | R^{2A} | R^{4A} | LCMS; [M+H]⁺ / Rt (min): Measurement Condition |
|---|---|---|---|---|
| 244 | 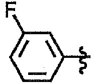 | H | 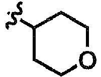 | 373 / 0.54 : B |
| 245 | 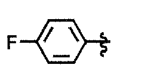 | H | 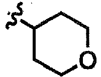 | 373 / 0.52 : B |
| 246 (TFA salt) | 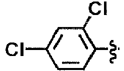 | H | 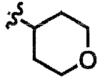 | 423 / 1.47 : B |
| 247 (TFA salt) | 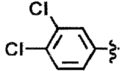 | H | 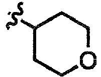 | 423 / 1.41 : B |
| 248 | 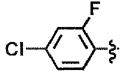 | H | 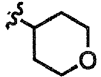 | 407 / 1.30 : B |
| 249 (TFA salt) | 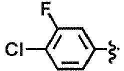 | H | 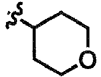 | 407 / 1.19 : B |
| 250 | 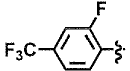 | H | 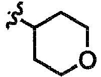 | 441 / 1.71 : B |
| 251 | 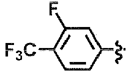 | H | 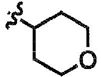 | 441 / 1.62 : B |
| 252 | 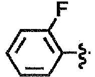 | Cl | 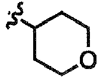 | 407 / 1.38 : B |
| 253 | 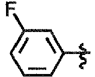 | Cl | 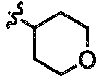 | 407 / 1.58 : B |
| 254 | 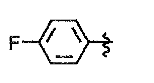 | Cl | 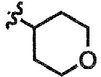 | 408 / 1.08 : B |
| 255 | 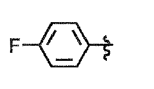 | CN | 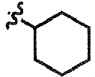 | 396 / 0.98 : A |
| 256 | 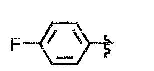 | Me | 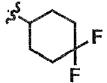 | 421 / 1.10 : B |
| 257 | 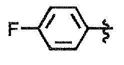 | Me | 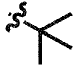 | 359 / 0.96 : B |
| 258 | 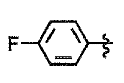 | Me | 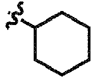 | 385 / 1.25 : B |
| 259 | 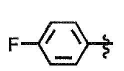 | Me | 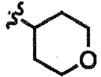 | 387 / 0.45 : B |

### Examples 260 to 276

Compounds listed in Table 11 were obtained by using corresponding starting compounds according to the methods of Reference Example 1 or 3 and Examples 5 to 7.

**[Table 11]**

| | | | | |
|---|---|---|---|---|
| 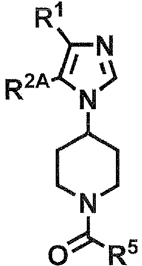 | | | | |

| Example | R¹ | R^{2A} | R⁵ | LCMS; [M+H]⁺ / Rt (min): Measurement Condition |
|---|---|---|---|---|
| 260 | 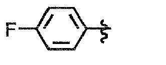 | H | 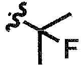 | 334 / 1.11 : B |
| 261 | 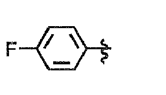 | H | 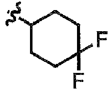 | 392 / 1.42 : B |
| 262 | 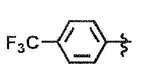 | Cl | 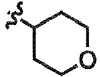 | 442 / 1.60 : B |
| 263 | 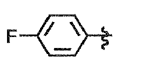 | Cl | 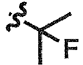 | 368 / 1.78 : B |
| 264 | 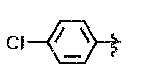 | Cl | 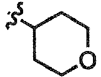 | 408 / 1.69 : B |
| 265 | 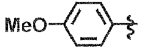 | CN | 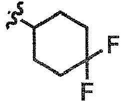 | 429 / 0.97 : A |
| 266 | 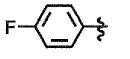 | CN | 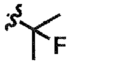 | 359 / 1.81 : B |
| 267 | 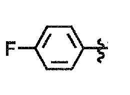 | CN | 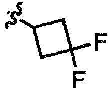 | 389 / 1.72 : B |
| 268 | 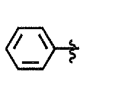 | CN | 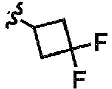 | 371 / 1.40 : B |
| 269 | 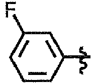 | CN | 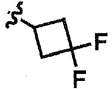 | 389 / 1.45 : B |
| 270 | 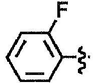 | CN | 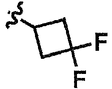 | 389 / 1.20 : B |
| 271 | 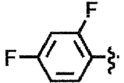 | CN | 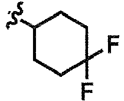 | 435 / 1.60 : B |
| 272 | 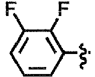 | CN | 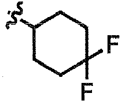 | 435 / 0.92 : B |
| 273 | 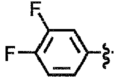 | CN | 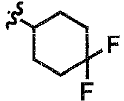 | 435 / 1.77 : B |
| 274 | 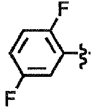 | CN | 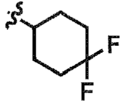 | 435 / 2.05 : B |
| 275 | 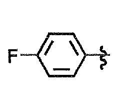 | Me | 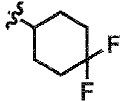 | 406 / 1.04 : B |
| 276 | 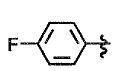 | Me | 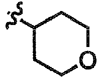 | 372 / 0.45 : B |

### Examples 277 to 354

Compounds listed in Table 12 were obtained by using corresponding starting compounds according to the methods of Reference Example 1 and Example 5, 7 or 8.

**[Table 12]**

| | | | | |
|---|---|---|---|---|
| 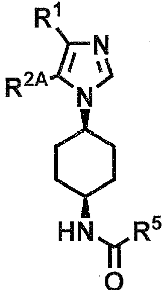 | | | | |

| Example | R¹ | R^{2A} | R⁵ | LCMS; [M+H]⁺ / Rt (min) : Measurement Condition |
|---|---|---|---|---|
| 277 | 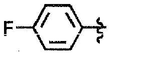 | H | 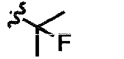 | 348 / 1.23 : B |
| 278 | 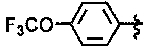 | H | 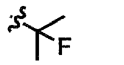 | 414 / 1.46 : B |
| 279 | 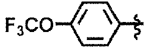 | H | 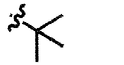 | 410 / 0.86 : B |
| 280 | 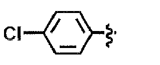 | H | 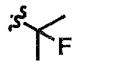 | 364 / 0.96 : B |
| 281 | 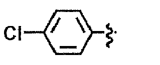 | H | 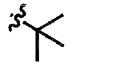 | 360 / 0.94 : B |
| 282 | 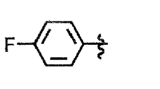 | H | 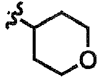 | 372 / 0.52 : B |
| 283 | 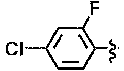 | H | 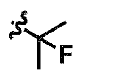 | 382 / 1.43 : B |
| 284 | 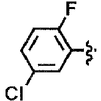 | H | 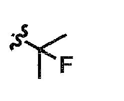 | 382 / 0.97 : B |
| 285 | 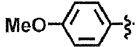 | H | 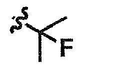 | 360 / 0.51 : B |
| 286 | 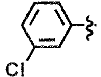 | H | 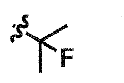 | 364 / 0.95 : B |
| 287 | 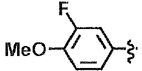 | H | 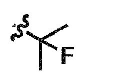 | 378 / 0.53 : B |
| 288 | 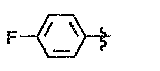 | H | 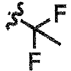 | 352 / 0.51 : B |
| 289 | 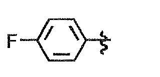 | H | 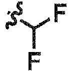 | 338 / 0.43 : B |
| 290 | 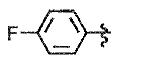 | H | 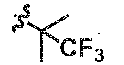 | 398 / 0.65 : B |
| 291 | 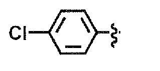 | H | 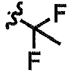 | 368 / 0.66 : B |
| 292 | 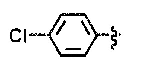 | H | 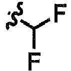 | 354 / 0.49 : B |
| 293 | 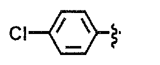 | H | 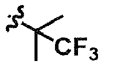 | 414 / 1.02 : B |
| 294 | 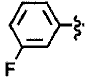 | H | 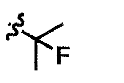 | 348 / 0.57 : B |
| 295 | 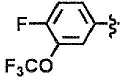 | H | 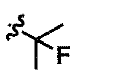 | 432 / 1.15 : B |
| 296 | 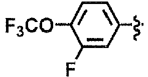 | H | 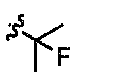 | 432 / 1.14 : B |
| 297 | 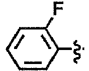 | H | 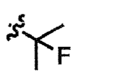 | 348 / 0.50 : B |
| 298 | 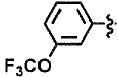 | H | 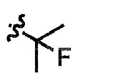 | 414 / 0.92 : B |
| 299 | 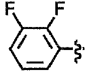 | H | 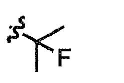 | 366 / 0.92 : B |
| 300 | 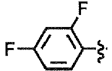 | H | 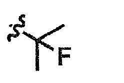 | 366 / 0.59 : B |
| 301 | 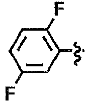 | H | 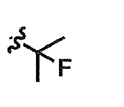 | 366 / 0.62 : B |
| 302 | 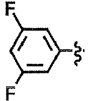 | H | 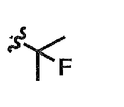 | 366 / 0.73 : B |
| 303 | 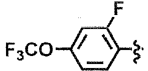 | H | 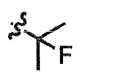 | 432 / 1.23 : B |
| 304 | 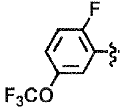 | H | 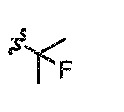 | 432 / 1.26 : B |
| 305 | 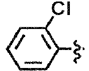 | H | 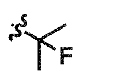 | 364 / 0.55 : B |
| 306 | 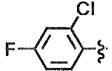 | H | 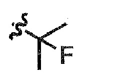 | 382 / 0.94 : B |
| 307 | 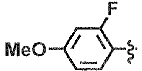 | H | 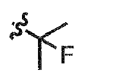 | 378 / 0.88 : B |
| 308 | 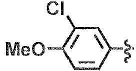 | H | 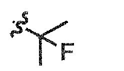 | 394 / 0.61 : B |
| 309 | 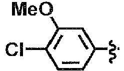 | H | 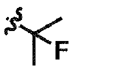 | 394 / 0.68 : B |
| 310 | 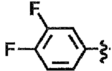 | H | 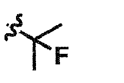 | 366 / 1.59 : D |
| 311 | 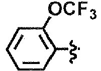 | H | 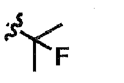 | 414 / 1.60 : D |
| 312 | 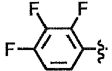 | H | 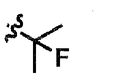 | 384 / 1.57 : D |
| 313 | 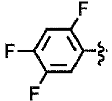 | H | 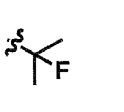 | 384 / 1.53 : D |
| 314 | 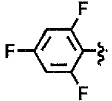 | H | 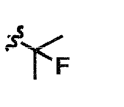 | 384 / 1.44 : D |
| 315 | 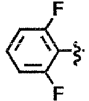 | H | 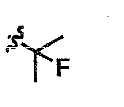 | 366 / 1.33 : D |
| 316 | 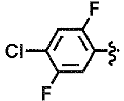 | H | 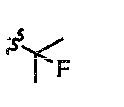 | 400 / 1.68 : D |
| 317 | 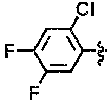 | H | 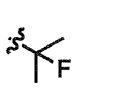 | 400 / 1.55 : D |
| 318 | 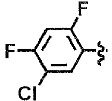 | H | 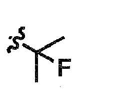 | 400 / 1.62 : D |
| 319 | 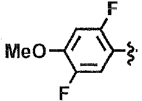 | H | 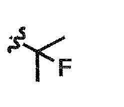 | 396 / 1.41 : D |
| 320 | 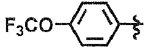 | Cl | 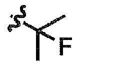 | 448 / 1.89 : B |
| 321 | 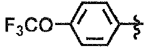 | Cl | 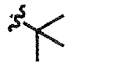 | 444 / 1.91 : B |
| 322 | 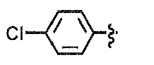 | Cl | 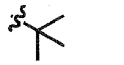 | 394 / 1.84 : B |
| 323 | 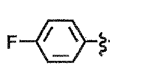 | Cl | 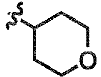 | 406 / 1.77 : B |
| 324 | 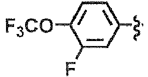 | Cl | 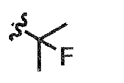 | 466 / 1.96 : B |
| 325 | 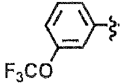 | Cl | 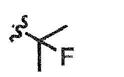 | 448 / 1.86 : B |
| 326 | 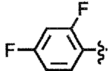 | Cl | 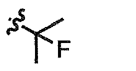 | 400 / 1.47 : B |
| 327 | 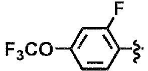 | Cl | 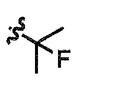 | 466 / 1.86 : B |
| 328 | 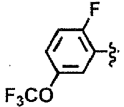 | Cl | 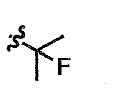 | 466 / 1.80 : B |
| 329 | 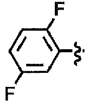 | Cl | 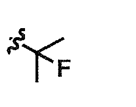 | 400 / 1.50 : B |
| 330 | 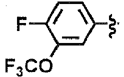 | Cl | 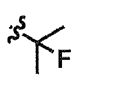 | 466 / 1.87 : E |
| 331 | 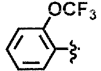 | Cl | 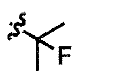 | 448 / 1.96 : D |
| 332 | 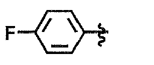 | CN | 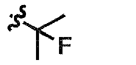 | 373 / 1.54 : B |
| 333 | 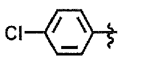 | CN | 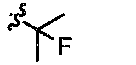 | 389 / 1.71 : B |
| 334 | 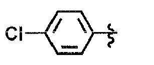 | CN | 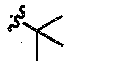 | 385 / 1.78 : B |
| 335 | 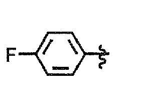 | CN | 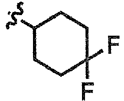 | 431 / 1.91 : B |
| 336 | 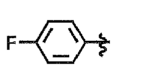 | CN | 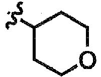 | 397 / 1.41 : B |
| 337 | 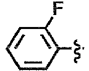 | CN | 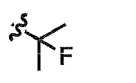 | 373 / 1.13 : E |
| 338 | 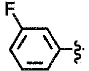 | CN | 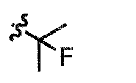 | 373 / 1.56 : E |
| 339 | 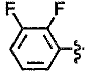 | CN | 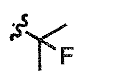 | 391 / 2.11 : D |
| 340 | 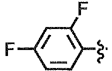 | CN | 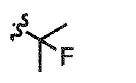 | 391 / 2.12 : D |
| 341 | 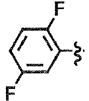 | CN | 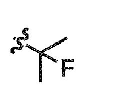 | 391 / 2.11 : D |
| 342 | 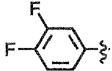 | CN | 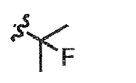 | 391 / 2.19 : D |
| 343 | 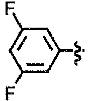 | CN | 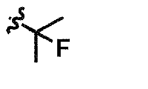 | 391 / 2.28 : D |
| 344 | 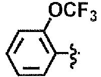 | CN | 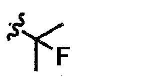 | 439 / 2.14 : D |
| 345 | 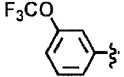 | CN | 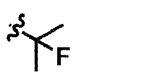 | 439 / 1.87 : E |
| 346 | 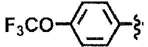 | CN | 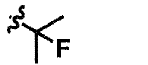 | 439 / 2.36 : D |
| 347 | 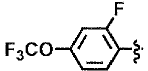 | CN | 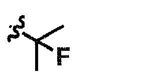 | 457 / 2.36 : D |
| 348 | 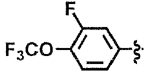 | CN | 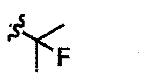 | 457 / 1.77 : E |
| 349 | 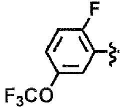 | CN | 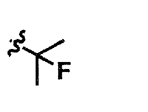 | 457 / 2.37 : D |
| 350 | 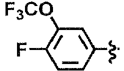 | CN | 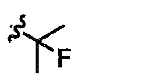 | 457 / 1.75 : E |
| 351 | 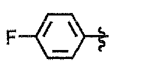 | Me | 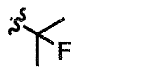 | 362 / 1.07 : B |
| 352 | 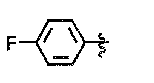 | Me | 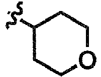 | 386 / 0.49 : B |
| 353 | 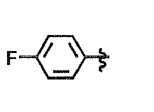 | Me | 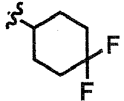 | 420 / 1.12 : B |
| 354 | 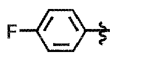 | Me | 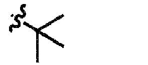 | 358 / 0.85 : B |

### Examples 355 to 381.

Compounds listed in Table 13 were obtained by using corresponding starting compounds according to the methods of Reference Example 1 or 6 and Example 5, 7 or 9.

**[Table 13]**

| | | | | |
|---|---|---|---|---|
| 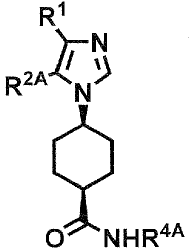 | | | | |

| Example | R¹ | R^{2A} | R^{4A} | LCMS; [M+H]⁺ / Rt (min) : Measurement Condition |
|---|---|---|---|---|
| 355 | 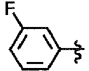 | H | 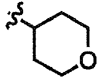 | 372 / 0.85 : B |
| 356 | 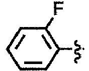 | H | 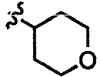 | 372 / 0.70 : B |
| 357 | 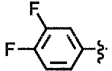 | H | 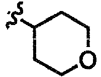 | 390 / 1.10 : B |
| 358 | 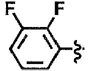 | H | 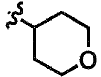 | 390 / 1.10 : B |
| 359 | 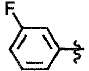 | Cl | 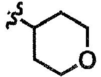 | 406 / 0.68 : B |
| 360 | 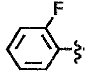 | Cl | 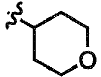 | 406 / 0.67 : B |
| 361 | 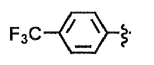 | Cl | 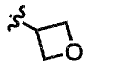 | 428 / 1.43 : B |
| 362 | 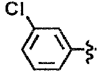 | Cl | 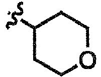 | 422 / 1.70 : B |
| 363 | 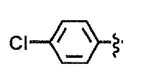 | Cl | 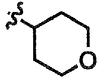 | 422 / 1.74 : B |
| 364 | 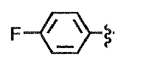 | Cl | 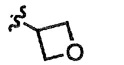 | 378 / 1.76 : B |
| 365 | 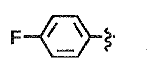 | Cl | 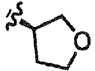 | 392 / 1.76 : B |
| 366 | 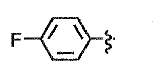 | Cl | 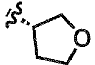 | 392 / 1.77 : B |
| 367 (TFA salt) | 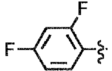 | Cl | 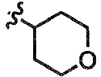 | 424 / 1.83 : B |
| 368 | 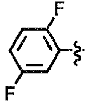 | Cl | 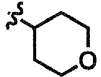 | 424 / 1.81 : B |
| 369 (TFA salt) | 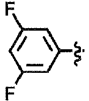 | Cl | 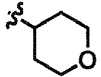 | 424 / 2.00 : B |
| 370 | 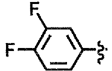 | Cl | 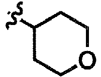 | 424 / 1.80 : B |
| 371 | 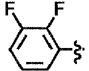 | Cl | 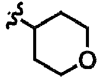 | 424 / 1.68 : B |
| 372 (TFA salt) | 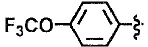 | Cl | 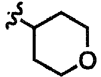 | 472 / 1.95 : B |
| 373 | 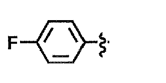 | CN | 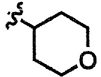 | 397 / 1.41 : B |
| 374 (TFA salt) | 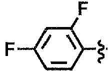 | CN | 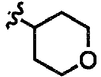 | 415 / 1.66 : B |
| 375 | 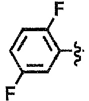 | CN | 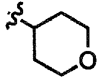 | 415 / 1.65 : B |
| 376 | 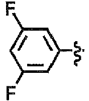 | CN | 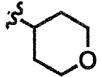 | 41 / 1.86 : B |
| 377 | 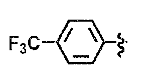 | CN | 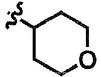 | 447 / 1.92 : B |
| 378 (TFA salt) | 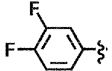 | CN | 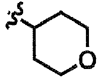 | 415 / 1.80 : B |
| 379 (TFA salt) | 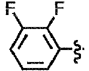 | CN | 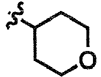 | 415 / 1.62 : B |
| 380 | 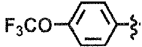 | CN | 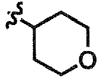 | 463 / 1.72 : B |
| 381 | 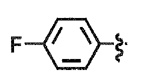 | Me | 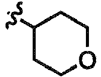 | 386 / 0.83 : B |

### Examples 382 to 404

Compounds listed in Table 14 were obtained by using corresponding starting compounds according to the methods of Reference Examples 1 to 5 and Examples 1 to 5 or 7.

**[Table 14]**

| | | | | |
|---|---|---|---|---|
| 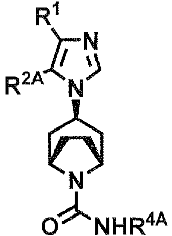 | | | | |

| Example | R¹ | R^{2A} | R^{4A} | LCMS; [M+H]⁺ / Rt (min) : Measurement Condition |
|---|---|---|---|---|
| 382 | 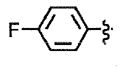 | H | 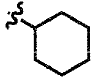 | 397 / 0.68 : A |
| 383 | 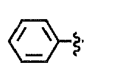 | H | 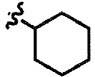 | 379 / 1.30 : B |
| 384 | 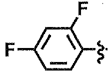 | H | 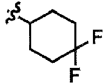 | 451 / 0.76 : B |
| 385 | 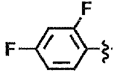 | H | 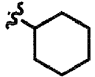 | 415 / 2.13 : F |
| 386 | 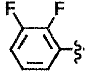 | H | 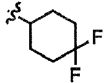 | 451 / 1.90 : G |
| 387 | 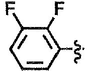 | H | 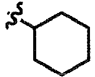 | 415 / 2.16 : F |
| 388 | 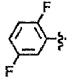 | H | 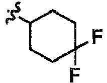 | 451 / 1.60 : D |
| 389 | 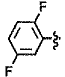 | H | 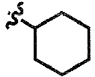 | 415 / 1.65 : D |
| 390 | 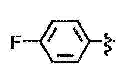 | Cl | 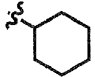 | 431 / 2.00 : B |
| 391 (TFA salt) | 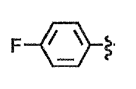 | Cl | 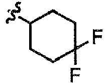 | 467 / 1.93 : B |
| 392 (TFA salt) | 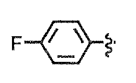 | Cl | 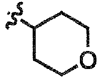 | 433 / 1.71 : B |
| 393 | 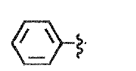 | Cl | 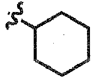 | 413 / 0.72 : B |
| 394 | 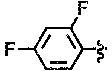 | Cl | 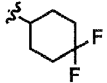 | 485 / 1.57 : B |
| 395 | 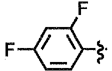 | Cl | 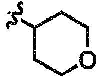 | 451 / 2.13 : F |
| 396 | 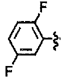 | Cl | 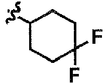 | 485 / 2.02 : D |
| 397 | 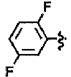 | Cl | 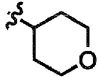 | 451 / 1.69 : D |
| 398 | 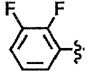 | Cl | 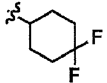 | 485 / 1.95 : D |
| 399 | 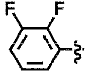 | Cl | 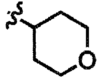 | 451 / 1.63 : G |
| 400 (TFA salt) | 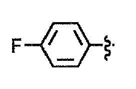 | CN | 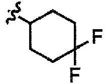 | 458 / 1.63 : B |
| 401 (TFA salt) | 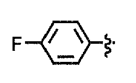 | CN | 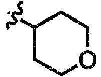 | 424 / 1.39 : B |
| 402 | 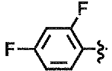 | CN | 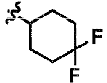 | 476 / 2.08 : D |
| 403 | 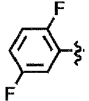 | CN | 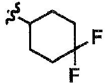 | 476 / 1.47 : E |
| 404 | 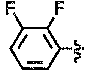 | CN | 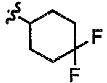 | 476 / 2.07 : D |

### Examples 405 to 424

Compounds listed in Table 15 were obtained by using corresponding starting compounds according to the methods of Reference Examples 1 to 5 and Examples 1 to 5 or 7.

**[Table 15]**

| | | | | |
|---|---|---|---|---|
| 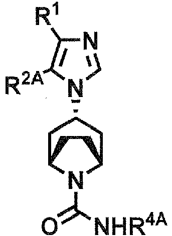 | | | | |

| Example | R¹ | R^{2A} | R^{4A} | LCMS; [M+H]⁺ / Rt (min) : Measurement Condition |
|---|---|---|---|---|
| 405 | 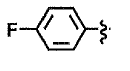 | H | 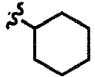 | 397 / 0.67 : A |
| 406 | 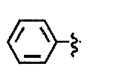 | H | 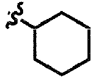 | 379 / 1.32 : A |
| 407 | 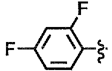 | H | 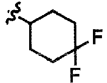 | 451 / 0.70 : B |
| 408 | 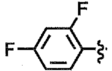 | H | 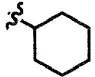 | 415 / 1.00 : B |
| 409 | 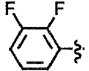 | H | 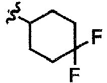 | 451 / 2.09 : B |
| 410 | 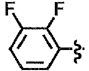 | H | 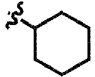 | 415 / 2.15 : B |
| 411 | 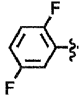 | H | 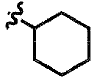 | 415 / 1.66 : D |
| 412 | 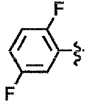 | H | 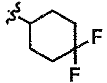 | 451 / 1.60 : D |
| 413 | 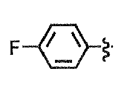 | Cl | 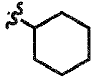 | 431 / 1.97 : B |
| 414 | 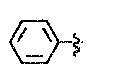 | Cl | 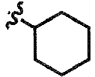 | 413 / 0.72 : B |
| 415 | 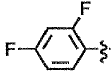 | Cl | 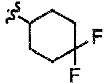 | 485 / 1.98 : D |
| 416 | 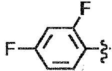 | Cl | 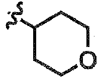 | 451 / 1.71 : D |
| 417 | 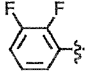 | Cl | 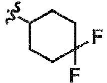 | 485 / 2.04 : D |
| 418 | 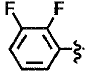 | Cl | 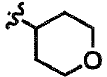 | 451 / 1.77 : D |
| 419 | 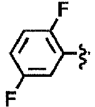 | Cl | 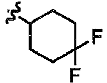 | 485 / 2.03 : D |
| 420 | 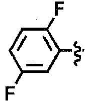 | Cl | 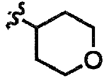 | 451 / 1.75 : D |
| 421 | 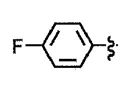 | CN | 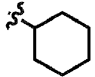 | 422 / 1.97 : B |
| 422 | 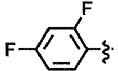 | CN | 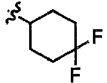 | 476 / 2.10 : D |
| 423 | 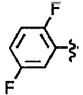 | CN | 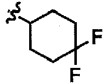 | 476 / 1.46 : E |
| 424 | 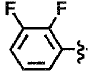 | CN | 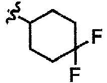 | 476 / 1.86 : G |

### Examples 425 to 445

Compounds listed in Table 16 were obtained by using corresponding starting compounds according to the methods of Reference Example 1 or 3 and Examples 5 to 7.

**[Table 16]**

| | | | | |
|---|---|---|---|---|
| 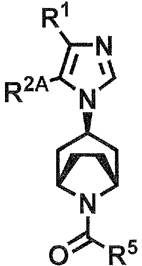 | | | | |

| Example | R¹ | R^{2A} | R⁵ | LCMS; [M+H]⁺ / Rt (min) : Measurement Condition |
|---|---|---|---|---|
| 425 | 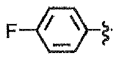 | H | 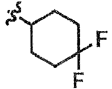 | 418 / 1.18:B |
| 426 | 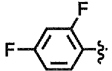 | H | 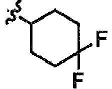 | 436 / 1.60 : B |
| 427 | 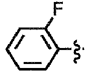 | H | 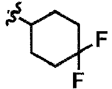 | 418 / 1.25 : B |
| 428 | 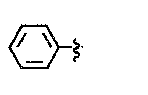 | H | 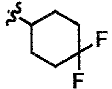 | 400 / 1.13 : B |
| 429 | 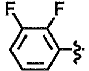 | H | 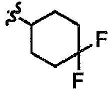 | 436 / 1.91 : G |
| 430 | 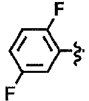 | H | 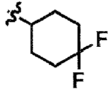 | 436 / 1.63 : D |
| 431 | 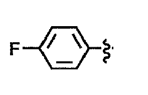 | Cl | 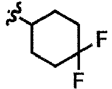 | 452 / 1.88 : B |
| 432 | 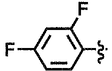 | Cl | 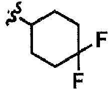 | 470 / 1.88 : B |
| 433 (TFA salt) | 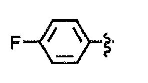 | Cl | 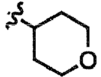 | 418 / 1.69 : B |
| 434 | 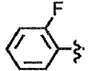 | Cl | 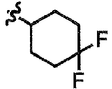 | 452 / 1.74 : B |
| 435 | 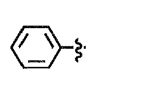 | Cl | 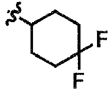 | 434 / 0.70 : B |
| 436 | 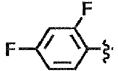 | Cl | 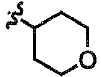 | 436 / 2.19 : F |
| 437 | 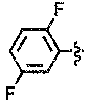 | Cl | 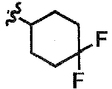 | 470 / 2.01 : D |
| 438 | 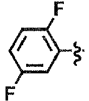 | Cl | 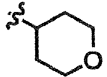 | 436 / 1.74 : D |
| 439 | 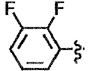 | Cl | 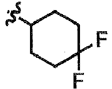 | 470 / 2.00 : D |
| 440 | 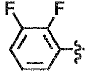 | Cl | 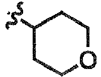 | 436 / 1.70 : D |
| 441 (TFA salt) | 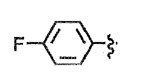 | CN | 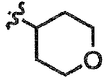 | 409 / 1.35 : B |
| 442 (TFA salt) | 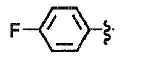 | CN | 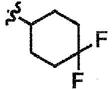 | 443 / 1.60 : B |
| 443 | 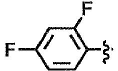 | CN | 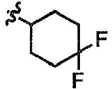 | 461 / 2.17 : D |
| 444 | 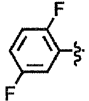 | CN | 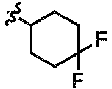 | 461 / 1.43 : E |
| 445 | 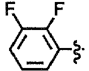 | CN | 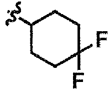 | 461 / 1.91 : G |

### Examples 446 to 464

Compounds listed in Table 17 were obtained by using corresponding starting compounds according to the methods of Reference Example 1 or 3 and Examples 5 to 7.

**[Table 17]**

| | | | | |
|---|---|---|---|---|
| 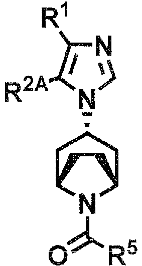 | | | | |

| Example | R¹ | R^{2A} | R⁵ | LCMS; [M+H]⁺ / Rt (min) : Measurement Condition |
|---|---|---|---|---|
| 446 | 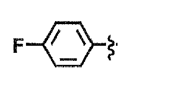 | H | 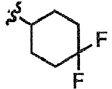 | 418 / 1.12 : B |
| 447 | 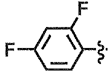 | H | 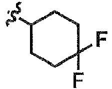 | 436 / 1.59 : B |
| 448 | 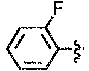 | H | 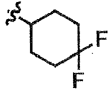 | 418 / 1.39 : B |
| 449 | 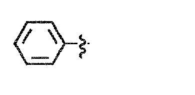 | H | 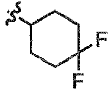 | 400 / 1.07 : B |
| 450 | 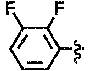 | H | 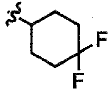 | 436 / 2.11 : B |
| 451 | 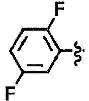 | H | 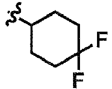 | 436 / 1.61 : D |
| 452 | 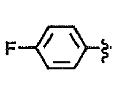 | Cl | 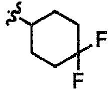 | 452 / 1.80 : B |
| 453 | 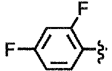 | Cl | 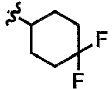 | 470 / 1.88 : B |
| 454 | 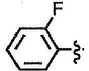 | Cl | 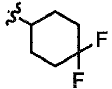 | 452 / 1.82 : B |
| 455 | 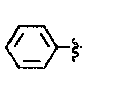 | Cl | 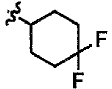 | 434 / 1.74 : B |
| 456 | 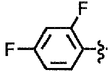 | Cl | 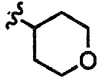 | 436 / 1.76 : D |
| 457 | 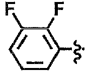 | Cl | 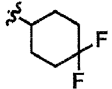 | 470 / 2.09 : D |
| 458 | 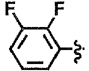 | Cl | 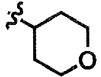 | 436 / 1.83 : D |
| 459 | 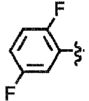 | Cl | 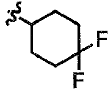 | 470 / 2.08 : D |
| 460 | 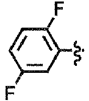 | Cl | 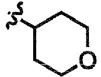 | 436 / 1.81 : D |
| 461 | 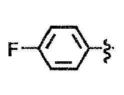 | CN | 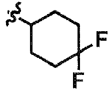 | 443 / 1.80 : B |
| 462 | 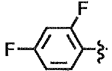 | CN | 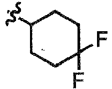 | 461 / 2.13 : D |
| 463 | 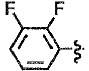 | CN | 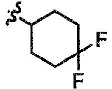 | 461 / 2.05 : D |
| 464 | 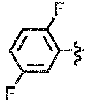 | CN | 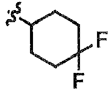 | 461 / 2.12 : D |

### Test Example

Hereinafter, pharmacological test results of the representative compounds of the present invention are demonstrated and pharmacological actions of such compounds are explained, but the present invention should not be limited thereto.

### Test Example 1. Evaluation of PAM activity with human α7 nACh receptor stably expressing cells

### (1) Human α7 nAChR stably expressing cells

Human α7 nAChR stably expressing cells were generated and cultured. In particular, GH4Cl cells derived from rat pituitary (cat#CCL-82.2, ATCC, USA) were used as a host cell. pcDNA3.1Zeo vector containing a nucleotide sequence encoding a protein GenBank BAC81731 and pcDNA3.1 vector containing human α7 nAChR gene (cat#V790-20, invitrogen, Carlsbad, CA, USA) were transfected to the cells to give aequorins and human α7 nAChR stably expressing cells. The aequorins and human α7 nAChR stably expressing cells were screened with Zeocin (cat#R25001, invitrogen, Carlsbad, CA, USA) and Geneticin (cat#10131-027, invitrogen, Carlsbad, CA, USA), respectively.

The cells were cultured in F-10 Nutrient Mixture (Ham) medium (cat#11550-043, invitrogen, Carlsbad, CA, USA) containing 2.5 % fetal bovine serum (cat#2917354, ICN Biomedicals, Inc, USA), 15 % inactivated horse serum (cat#26050-088, invitrogen, Carlsbad, CA, USA), 1 µg/mL Geneticin, and 5 µg/mL Puromycin (cat#14861-84, invitrogen, Carlsbad, CA, USA), in a Collagen Type 1-coated dish (cat#4030-010, iwaki, Tokyo, Japan). During the culture, the medium was replaced with fresh medium in every 2 to 3 days, and the cells were treated with TrypLE Express (cat# 45604-021, invitrogen, Carlsbad, CA, USA) to collect them in every 7 days. Thus, the cells were subcultured.

Seven days after subculturing, the cells were treated with TrypLE Express to collect them when they were about 80 % confluent. The cells were suspended in a reaction medium containing Hanks (cat#14065-056, invitrogen, Carlsbad, CA, USA) / 20 mmol/L Hepes (cat#15630-080, invitrogen, Carlsbad, CA, USA), Buffer (pH 7.4), F-10 Nutrient Mixture (Ham), and 0.1 mg/mL Geneticin, and the suspension was seeded in a 384-well plate (cat#781090, Greiner, Germany) at 20000 cells / 25 µL per well..

On the next day after seeding, Viviren (cat#E649X, Promega, Madison, WI, USA) was added to the medium so that the final concentration could be 4 µM (15 µL/well). The plates were centrifuged and then placed in the dark for 4 hours at room temperature.

### (2) Preparation of the test samples

Each of the test compounds was dissolved in DMSO to prepare each test sample at a concentration of 1000-fold the final concentration. To the solution was added Hanks / 20 mM HEPES / 0.2% BSA (cat#A3803, Sigma, St.Louis, MO, USA), and the concentration was adjusted to 6-fold the final concentration.

### (3) Evaluation of PAM activity

FDSS7000 (Hamamatsu Photonics) was used to detect the luminescence signal evoked by α7 nAChR stimulation. The cells and a luminescent substrate were put on a plate, and the test sample was added thereto. After 150 seconds, ACh whose concentration shows 20% (EC₂₀) of the maximal signal was added thereto. After the addition of ACh, the luminescence signal (the central wavelength: 465 nm) was measured for 138 seconds to calculate RLU (Max-Min). The ratio of the RLU (Max-Min) of the test-compound-containing wells to that of the control wells was defined as PAM activity.

Tables 18 to 25 show α7 PAM activity data of the representative compounds in the present invention.

**[Table 18]**

| Example | α7PAM (%) @10µM | Example | α7PAM (%) @10µM | Example | α7PAM (%) @10µM |
|---|---|---|---|---|---|
| 1 | 836 | 26 | 563 | 51 | 520 |
| 2 | 117 | 27 | 139 | 52 | 205 |
| 3 | 493 | 28 | 798 | 53 | 256 |
| 4 | 220 | 29 | 378 | 54 | 371 |
| 5 | 1151 | 30 | 319 | 55 | 403 |
| 6 | 292 | 31 | 300 | 56 | 169 |
| 7 | 644 | 32 | 993 | 57 | 302 |
| 8 | 476 | 33 | 126* | 58 | 318 |
| 9 | 121 | 34 | 596 | 59 | 707 |
| 10 | 597 | 35 | 878 | 60 | 616 |
| 11 | 116* | 36 | 193 | 61 | 915 |
| 12 | 283 | 37 | 366 | 62 | 146 |
| 13 | 161 | 38 | 311 | 63 | 160 |
| 14 | 351 | 39 | 823 | 64 | 149 |
| 15 | 596 | 40 | 129 | 65 | 241 |
| 16 | 1391 | 41 | 832 | 66 | 1037 |
| 17 | 1103 | 42 | 122 | 67 | 317 |
| 18 | 1864 | 43 | 250 | 68 | 143 |
| 19 | 3672 | 44 | 880 | 69 | 305 |
| 20 | 656 | 45 | 260 | 70 | 158 |
| 21 | 291 | 46 | 104* | 71 | 422 |
| 22 | 1950 | 47 | 143 | 72 | 1616 |
| 23 | 209 | 48 | 305 | 73 | 2536 |
| 24 | 228 | 49 | 445 | 74 | 779 |
| 25 | 2162 | 50 | 489 | 75 | 1034 |

**[Table 19]**

| Example | α7PAM (%) @10µM | Example | α7PAM (%) @10µM | Example | α7PAM (%) @10µM |
|---|---|---|---|---|---|
| 76 | 1297 | 101 | 1350 | 126 | 780 |
| 77 | 1198 | 102 | 641 | 127 | 170 |
| 78 | 1068 | 103 | 452 | 128 | 188 |
| 79 | 112* | 104 | 1349 | 129 | 330 |
| 80 | 1436 | 105 | 1385 | 130 | 376 |
| 81 | 1479 | 106 | 945 | 131 | 738 |
| 82 | 1133 | 107 | 144 | 132 | 151 |
| 83 | 671 | 108 | 576 | 133 | 101 |
| 84 | 747 | 109 | 457 | 134 | 171 |
| 85 | 1647 | 110 | 201 | 135 | 154 |
| 86 | 1310 | 111 | 153 | 136 | 309 |
| 87 | 1273 | 112 | 1254 | 137 | 328 |
| 88 | 701 | 113 | 882 | 138 | 597 |
| 89 | 596 | 114 | 133 | 139 | 274 |
| 90 | 297 | 115 | 1092 | 140 | 127 |
| 91 | 1498 | 116 | 131 | 141 | 253 |
| 92 | 758 | 117 | 853 | 142 | 163 |
| 93 | 1539 | 118 | 1923 | 143 | 1286 |
| 94 | 981 | 119 | 1302 | 144 | 346 |
| 95 | 761 | 120 | 208 | 145 | 363 |
| 96 | 474 | 121 | 201 | 146 | 572 |
| 97 | 197 | 122 | 459 | 147 | 484 |
| 98 | 1659 | 123 | 345 | 148 | 700 |
| 99 | 1702 | 124 | 572 | 149 | 862 |
| 100 | 708 | 125 | 466 | 150 | 137 |

**[Table 20]**

| Example | α7PAM (%) @10µM | Example | α7PAM (%) @10µM | Example | α7PAM (%) @10µM |
|---|---|---|---|---|---|
| 151 | 155 | 176 | 258* | 201 | 224 |
| 152 | 945 | 177 | 389* | 202 | 263 |
| 153 | 538 | 178 | 775 | 203 | 641 |
| 154 | 644 | 179 | 269 | 204 | 172 |
| 155 | 252 | 180 | 375 | 205 | 110 |
| 156 | 522 | 181 | 641 | 206 | 102 |
| 157 | 236 | 182 | 682* | 207 | 115 |
| 158 | 132 | 183 | 791 | 208 | 202 |
| 159 | 692 | 184 | 303* | 209 | 139 |
| 160 | 367 | 185 | 576 | 210 | 122 |
| 161 | 233* | 186 | 254 | 211 | 390 |
| 162 | 323* | 187 | 379 | 212 | 135 |
| 163 | 144 | 188 | 581 | 213 | 271 |
| 164 | 176 | 189 | 624 | 214 | 273 |
| 165 | 557 | 190 | 131 | 215 | 369 |
| 166 | 389 | 191 | 364* | 216 | 235 |
| 167 | 221 | 192 | 274* | 217 | 1207 |
| 168 | 829 | 193 | 187 | 218 | 1727 |
| 169 | 454 | 194 | 1177 | 219 | 371 |
| 170 | 530 | 195 | 142 | 220 | 120 |
| 171 | 551 | 196 | 498 | 221 | 538 |
| 172 | 480 | 197 | 637 | 222 | 592 |
| 173 | 400 | 198 | 799 | 223 | 694 |
| 174 | 224 | 199 | 230* | 224 | 1345 |
| 175 | 397 | 200 | 180* | 225 | 151 |

**[Table 21]**

| Example | α7PAM (%) @10µM | Example | α7PAM (%) @10µM | Example | α7PAM (%) @10µM |
|---|---|---|---|---|---|
| 226 | 688 | 230 | 1579 | 234 | 130* |
| 227 | 818 | 231 | 149 | 235 | 631 |
| 228 | 286 | 232 | 113 | | |
| 229 | 1065 | 233 | 102* | | |

**[Table 22]**

| Example | α7PAM (%) @10µM | Example | α7PAM (%) @10µM | Example | α7PAM (%) @10µM |
|---|---|---|---|---|---|
| 236 | 158 | 261 | 399 | 286 | 469 |
| 237 | 405 | 262 | 603 | 287 | 1082 |
| 238 | 139 | 263 | 193 | 288 | 327 |
| 239 | 207 | 264 | 781 | 289 | 125 |
| 240 | 280 | 265 | 990 | 290 | 194 |
| 241 | 314 | 266 | 149 | 291 | 420 |
| 242 | 297 | 267 | 594 | 292 | 101 |
| 243 | 290 | 268 | 725 | 293 | 211 |
| 244 | 175 | 269 | 452 | 294 | 326 |
| 245 | 197 | 270 | 569 | 295 | 444* |
| 246 | 1256 | 271 | 275 | 296 | 589* |
| 247 | 448 | 272 | 138 | 297 | 866 |
| 248 | 901 | 273 | 139 | 298 | 166 |
| 249 | 384 | 274 | 351 | 299 | 893 |
| 250 | 1002 | 275 | 912 | 300 | 1379 |
| 251 | 369 | 276 | 244 | 301 | 828 |
| 252 | 648 | 277 | 424 | 302 | 325 |
| 253 | 899 | 278 | 834 | 303 | 208 |
| 254 | 192 | 279 | 236 | 304 | 600* |
| 255 | 759 | 280 | 787 | 305 | 1285 |
| 256 | 1345 | 281 | 411 | 306 | 1145 |
| 257 | 439 | 282 | 229 | 307 | 634 |
| 258 | 1174 | 283 | 390 | 308 | 513 |
| 259 | 420 | 284 | 398 | 309 | 181* |
| 260 | 146 | 285 | 869 | 310 | 227 |

**[Table 23]**

| Example | α7PAM (%) @10µM | Example | α7PAM (%) @10µM | Example | α7PAM (%) @10µM |
|---|---|---|---|---|---|
| 311 | 290 | 336 | 557 | 361 | 794 |
| 312 | 662 | 337 | 492 | 362 | 1896 |
| 313 | 273* | 338 | 192 | 363 | 3665 |
| 314 | 591 | 339 | 351 | 364 | 537 |
| 315 | 501 | 340 | 377 | 365 | 1064 |
| 316 | 267* | 341 | 233 | 366 | 834 |
| 317 | 345* | 342 | 369 | 367 | 1380 |
| 318 | 179* | 343 | 380* | 368 | 941 |
| 319 | 328 | 344 | 220 | 369 | 404 |
| 320 | 1340 | 345 | 153 | 370 | 1309 |
| 321 | 800 | 346 | 169 | 371 | 962 |
| 322 | 314 | 347 | 130 | 372 | 569 |
| 323 | 421 | 348 | 131 | 373 | 966 |
| 324 | 860 | 349 | 290 | 374 | 820 |
| 325 | 210 | 350 | 670* | 375 | 457 |
| 326 | 1196 | 351 | 540 | 376 | 426 |
| 327 | 1479 | 352 | 164 | 377 | 988 |
| 328 | 859* | 353 | 518 | 378 | 854 |
| 329 | 643 | 354 | 230 | 379 | 685 |
| 330 | 154 | 355 | 118* | 380 | 1574 |
| 331 | 467* | 356 | 119* | 381 | 377 |
| 332 | 464 | 357 | 130* | 382 | 222 |
| 333 | 233 | 358 | 126* | 383 | 598 |
| 334 | 212* | 359 | 581 | 384 | 652* |
| 335 | 312 | 360 | 598 | 385 | 218* |

**[Table 24]**

| Example | α7PAM (%) @10µM | Example | α7PAM (%) @10µM | Example | α7PAM (%) @10µM |
|---|---|---|---|---|---|
| 386 | 880 | 411 | 229 | 436 | 300 |
| 387 | 315* | 412 | 423 | 437 | 533 |
| 388 | 335* | 413 | 1407 | 438 | 235 |
| 389 | 163* | 414 | 1019 | 439 | 813 |
| 390 | 683 | 415 | 997 | 440 | 508 |
| 391 | 1703 | 416 | 2077 | 441 | 451 |
| 392 | 771 | 417 | 1080 | 442 | 1038 |
| 393 | 1002 | 418 | 1193 | 443 | 368* |
| 394 | 455 | 419 | 913 | 444 | 208 |
| 395 | 869 | 420 | 1189 | 445 | 272 |
| 396 | 585 | 421 | 426 | 446 | 143 |
| 397 | 522 | 422 | 131 | 447 | 268 |
| 398 | 607 | 423 | 390* | 448 | 276 |
| 399 | 891 | 424 | 210 | 449 | 183 |
| 400 | 1151 | 425 | 155 | 450 | 128 |
| 401 | 879 | 426 | 308 | 451 | 143* |
| 402 | 498 | 427 | 305 | 452 | 1150 |
| 403 | 590* | 428 | 144 | 453 | 1286 |
| 404 | 292 | 429 | 111* | 454 | 1120 |
| 405 | 402 | 430 | 110* | 455 | 1052 |
| 406 | 629 | 431 | 836 | 456 | 1339 |
| 407 | 904 | 432 | 1131 | 457 | 865 |
| 408 | 140 | 433 | 345 | 458 | 434 |
| 409 | 576 | 434 | 512 | 459 | 730 |
| 410 | 197 | 435 | 1030 | 460 | 390 |

**[Table 25]**

| Example | α7PAM (%) @10µM | Example | α7PAM (%) @10µM | Example | α7PAM (%) @10µM |
|---|---|---|---|---|---|
| 461 | 162 | | | | |
| 462 | 384 | | | | |
| 463 | 181* | | | | |
| 464 | 124* | | | | |

| | | | | | |
|---|---|---|---|---|---|
| *α7PAM (%) @1 µM | | | | | |

Tables 18 to 25 demonstrate that the present compounds have PAM activity for α7 nAChR according to the evaluation test of PAM activity. In particular, Examples 18, 19, 22, 25, 72, 73, 85, 93, 98, 99, 118, 218, 230, 362, 363, 380, 391 and 416 show a stronger PAM activity than others.

### Test Example 2. Evaluation of cognitive function with mice in novel object recognition test (hereinafter, referred to as "mORT")

Slc:ddY mice (25 to 30 g, male, Japan SLC) can be used in the novel object recognition test wherein the interval between the 1st trial (training) and the 2nd trial (test) correlates with the memory loss for the objects used in the 1st trial, and a significant memory-loss is observed when the 2nd trial is performed 24 hours after the 1 st trial. According to the test mechanism, the present compounds were administered prior to the 1st trial, and the enhancement effect on memory in the 2nd trial was evaluated. The results confirmed that Examples 1, 183, 280, 370 and 452 have a significant memory enhancing effect in 3 mg/kg (oral).

### Test Example 3. Evaluation on improvement against cognitive impairment with rats in Y-shaped maze test (hereinafter, referred to as "Y-maze test")

In Y-maze test, 0.6 mg/kg scopolamine HBr (cat#S0929, Sigma Aldrich, Japan) can be subcutaneously administered to Slc:Wistar rats (280 to 300 g, male, Japan SLC) to cause cognitive impairment and decrease the percentage of spontaneous alternation behavior. According to the test mechanism, the present compounds were treated prior to the administration of scopolamine, and the improvement effect on cognitive impairment is evaluated. The results confirmed that compounds of Examples 1 and 183 have a significant improvement effect on memory disorder in 3 mg/kg (oral).

### INDUSTRIAL APPLICABILITY

As explained above, the derivative of Formula (I) or a pharmaceutically acceptable salt thereof has potent modulatory-effects on the activity of α7 nicotinic acetylcholine receptor (α7 nAChR), and is thus useful for treating, for example, diseases associated with cholinergic properties in the central nervous system (CNS) and/or peripheral nervous system (PNS), diseases associated with smooth muscle contraction, endocrine disorders, neurodegenerative disorders, diseases such as inflammation and pain, and diseases associated with withdrawal symptoms caused by addictive drug abuse.

## Claims

1. A compound of Formula (I): 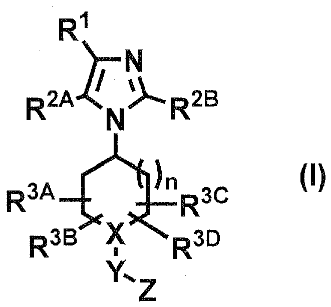
wherein X-Y-Z is N-CO-NR^{4A}R^{4B} N-COR⁵, CR⁶-CO-NR^{4A}R^{4B}, CR⁶-NR⁷-COR⁵, CR⁶-NR⁷-CONR^{4A}R^{4B} or CR⁶-NR⁷-Q,
R¹ is phenyl or monocyclic heteroaryl in which the phenyl and the monocyclic heteroaryl may be each optionally substituted with 1 to 5 substituents independently selected from the group consisting of halogen, hydroxyl group, C₁₋₆ alkyl which may be optionally substituted with 1 to 5 fluorine atoms, C₁₋₆ alkoxy which may be optionally substituted with 1 to 5 fluorine atoms, cyano, -NR⁸R⁹, -COOR⁸, -CONR⁸R⁹ and -NR⁸COR⁹,
R^{2A} and R^{2B} are the same or different and are hydrogen atom; halogen; cyano; - COOR¹⁰; -CONR¹⁰R¹¹; -NR¹⁰R¹¹; -NR¹⁰COR¹¹; C₁₋₆ alkyl which may be optionally substituted with 1 to 5 substituents independently selected from the group consisting of halogen, hydroxyl group, C₁₋₆ alkyl, C₃₋₁₀ cycloalkyl which may be optionally substituted with 1 to 5 fluorine atoms, C₁₋₆ alkoxy, 4- to 10-membered saturated heterocycle, cyano, -NR¹⁰R¹¹, -COOR¹⁰,-CONR¹⁰R¹¹ and -NR¹⁰COR¹¹; or C₃₋₁₀ cycloalkyl which may be optionally substituted with 1 to 5 substituents independently selected from the group consisting of halogen, hydroxyl group, C₁₋₆ alkyl, C₁₋₆ alkoxy, cyano, -NR¹⁰R¹¹, -COOR¹⁰, -CONR¹⁰R¹¹ and -NR¹⁰COR¹¹; provided that when X-Y-Z is N-CO-NHEt and n = 1, R^{2A} is hydrogen atom, halogen, cyano, or C₁₋₄ alkyl which may be optionally substituted with the above substituents,
R^{3A}, R^{3B}, R^{3C}, R^{3D} and R⁶ are the same or different and are hydrogen atom; fluorine atom; hydroxyl group; C₁₋₆ alkoxy which may be optionally substituted with 1 to 5 fluorine atoms; or C₁₋₆ alkyl which may be optionally substituted with 1 to 5 fluorine atoms; provided that when any two of R^{3A}, R^{3B}, R^{3C}, R^{3D} and R⁶ are independently selected from C₁₋₆ alkyl which may be optionally substituted with 1 to 5 fluorine atoms, the two alkyl groups may be combined each other together with the ring to which the alkyl groups attach to form another ring,
R^{4A}, R^{4B}, R⁵ and R⁷ are the same or different and are C₁₋₆ alkyl which may be optionally substituted with 1 to 5 substituents independently selected from the group consisting of aryl and heteroaryl (in which the aryl and the heteroaryl may be optionally substituted with 1 to 5 substituents independently selected from the group consisting of halogen, hydroxyl group, C₁₋₆ alkyl which may be optionally substituted with 1 to 5 fluorine atoms, and C₁₋₆ alkoxy which may be optionally substituted with 1 to 5 fluorine atoms), halogen, hydroxyl group, C₁₋₆ alkoxy, 4- to 10-membered saturated heterocycle, C₃₋₁₀ cycloalkyl and -NR¹²R¹³; C₃₋₁₀ cycloalkyl which may be optionally substituted with 1 to 5 substituents independently selected from the group consisting of aryl and heteroaryl (in which the aryl and the heteroaryl may be optionally substituted with 1 to 5 substituents independently selected from the group consisting of halogen, hydroxyl group, C₁₋₆ alkyl which may be optionally substituted with 1 to 5 fluorine atoms, and C₁₋₆ alkoxy which may be optionally substituted with 1 to 5 fluorine atoms), halogen, hydroxyl group, C₁₋₆ alkoxy, C₁₋₆ alkyl and -NR¹²R¹³; 4- to 10-membered saturated heterocycle which may be optionally substituted with C₁₋₆ alkyl; aryl or heteroaryl (in which the aryl and the heteroaryl may be each optionally substituted with 1 to 5 substituents independently selected from the group consisting of halogen, C₁₋₆ alkyl which may be optionally substituted with 1 to 5 fluorine atom, and C₁₋₆ alkoxy which may be optionally substituted with 1 to 5 fluorine atoms); or hydrogen atom; provided that R⁵ is not hydrogen atom, R^{4A} and R^{4B} are not concurrently hydrogen atom, and when both R^{4A} and R^{4B} are independently selected from C₁₋₆ alkyl, then they may be combined each other to form 4- to 10-membered nitrogen-containing saturated heterocycle which may be optionally substituted with 1 to 5 substituents independently selected from the group consisting of fluorine atom, C₁₋₆ alkyl and C₁₋₆ alkoxy,
Q is 6-membered heteroaryl which contains 1 or 2 nitrogen atoms [in which the heteroaryl may be optionally substituted with 1 to 3 substituents independently selected from the group consisting of C₁₋₆ alkyl which may be optionally substituted with 1 to 5 substituents independently selected from the group consisting of fluorine atom, hydroxyl group, C₁₋₆ alkoxy, C₃₋₆ cycloalkyl, -NR¹⁰R¹¹, -CONR¹⁰R¹¹ and -NR¹⁰COR¹¹; C₃₋₁₀ cycloalkyl, C₃₋₁₀ cycloalkoxy or 4- to 10-membered saturated heterocycle (in which the cycloalkyl, the cycloalkoxy and the saturated heterocycle may be each optionally substituted with 1 to 5 substituents independently selected from the group consisting of fluorine atom, hydroxyl group, C₁₋₆ alkyl, C₁₋₄ alkoxy,-NR¹⁴R¹⁵, -CONR¹⁴R¹⁵ and -NR¹⁴COR¹⁵); C₁₋₆ alkoxy which may be optionally substituted with 1 to 5 substituents independently selected from the group consisting of fluorine atom, hydroxyl group, C₁₋₆ alkoxy, -NR¹⁴R¹⁵, -CONR¹⁴R¹⁵ and -NR¹⁴COR¹⁵; halogen; cyano; -CONR¹⁴R¹⁵;-NR¹⁴COR¹⁵; or -NR¹⁴R¹⁵],
R⁸ to R¹⁵ are the same or different, and independent each other when the same substituent symbol exists plurally, and are hydrogen atom or C₁₋₆ alkyl which may be optionally substituted with 1 to 5 fluorine atoms; provided that in each combination of R⁸ and R⁹, R¹⁰ and R¹¹, R¹² and R¹³, or R¹⁴ and R¹⁵, (1) when one is hydrogen atom, the other is not hydrogen atom, and (2) when both of them are independently selected from C₁₋₆ alkyl, they may be combined each other to form 4- to 10-membered nitrogen-containing saturated heterocycle, and
n is 1 or 2, or a pharmaceutically acceptable salt thereof.

2. The compound of claim 1 wherein n is 1, or a pharmaceutically acceptable salt thereof.

3. The compound of claim 1 or 2 wherein X-Y-Z is N-CO-NR^{4A}R^{4B}, N-COR⁵, CR⁶-CO-NR^{4A}R^{4B} or CR⁶-NR⁷-COR⁵, or a pharmaceutically acceptable salt thereof.

4. The compound of any one of claims 1 to 3 wherein R¹ is phenyl or monocyclic heteroaryl in which the phenyl and the monocyclic heteroaryl may be each optionally substituted with 1 to 5 substituents independently selected from the group consisting of halogen, hydroxyl group, C₁₋₆ alkyl which may be optionally substituted with 1 to 5 fluorine atoms, C₁₋₆ alkoxy which may be optionally substituted with 1 to 5 fluorine atoms, and cyano, or a pharmaceutically acceptable salt thereof.

5. The compound of any one of claims 1 to 4 wherein R^{2A} and R^{2B} are the same or different and are hydrogen atom; halogen; cyano; or C₁₋₆ alkyl which may be optionally substituted with 1 to 5 substituents independently selected from the group consisting of halogen, hydroxyl group, C₁₋₆ alkyl, C₃₋₁₀ cycloalkyl which may be optionally substituted with 1 to 5 fluorine atoms, C₁₋₆ alkoxy, and 4- to 10-membered saturated heterocycle; provided that when X-Y-Z is N-CO-NHEt and n = 1, R^{2A} is hydrogen atom, halogen, cyano, or C₁₋₄ alkyl which may be optionally substituted with the above substituents, or a pharmaceutically acceptable salt thereof.

6. The compound of any one of claims 1 to 5 wherein R^{3A}, R^{3B}, R^{3C}, R^{3D} and R⁶ are the same or different and are hydrogen atom, or C₁₋₆ alkyl; provided that when any two of R^{3A}, R^{3B}, R^{3C} and R^{3D} are independently selected from C₁₋₆ alkyl, the two alkyl groups may be combined each other together with the carbon atoms to which the alkyl groups attach or the ring containing the carbon atoms to form another ring, or a pharmaceutically acceptable salt thereof.

7. The compound of any one of claims 1 to 6 wherein R^{4A}, R^{4B}, R⁵ and R⁷ are the same or different and are C₁₋₆ alkyl which may be optionally substituted with 1 to 5 substituents independently selected from the group consisting of halogen, hydroxyl group, C₁₋₆ alkoxy, 4-to 10-membered saturated heterocycle, C₃₋₁₀ cycloalkyl and -NR¹²R¹³; C₃₋₁₀ cycloalkyl which may be optionally substituted with 1 to 5 substituents independently selected from the group consisting of halogen, hydroxyl group, C₁₋₆ alkoxy, C₁₋₆ alkyl and -NR¹²R¹³; aryl or heteroaryl in which the aryl and the heteroaryl may be each optionally substituted with 1 to 5 substituents independently selected from the group consisting of halogen, C₁₋₆ alkyl which may be optionally substituted with 1 to 5 fluorine atoms, and C₁₋₆ alkoxy which may be optionally substituted with 1 to 5 fluorine atoms; or hydrogen atom; provided that R⁵ is not hydrogen atom, or a pharmaceutically acceptable salt thereof.

8. The compound of any one of claims 1 to 7 wherein R^{4B} and R⁷ are hydrogen atom, or a pharmaceutically acceptable salt thereof.

9. The compound of any one of claims 1 to 8 wherein X-Y-Z is N-CO-NR^{4A}R^{4B}, or a pharmaceutically acceptable salt thereof.

10. The compound of any one of claims 1 to 8 wherein X-Y-Z is N-COR⁵, or a pharmaceutically acceptable salt thereof.

11. The compound of any one of claims 1 to 8 wherein X-Y-Z is CR⁶-CO-NR^{4A}R^{4B}, or a pharmaceutically acceptable salt thereof.

12. The compound of claim 1 selected from the following compounds:
N-cyclohexyl-4-[4-(3-fluorophenyl)-1H-imidazol-1-yl]piperidine-1-carboxamide (Example 1),
1-{1-[(4,4-difluorocyclohexyl)carbonyl]piperidin-4-yl} -4-phenyl-1 H-imidazole-5 -carbonitrile (Example 7),
N-cyclohexyl-4-[4-(2-fluorophenyl)-1H-imidazol-1-yl]piperidine-1-carboxamide (Example 66), 4-{5-chloro-4-[3-(trifluoromethyl)phenyl]-1H-imidazol-1-yl}-N-(tetrahydro-2H-pyran-4-yl)piperidine-1-carboxamide (Example 94),
N-(4,4-difluorocyclohexyl)-4-(5-methyl-4-phenyl-1H-imidazol-1-yl)piperidine-1-carboxamide (Example 105),
1- {1-[(4,4-difluorocyclohexyl)carbonyl]piperidin-4-yl}-4-(4-fluorophenyl)-1H-imidazole-5-carbonitrile (Example 154),
1-{1-[(4,4-difluorocyclohexyl)carbonyl]piperidin-4-yl}-4-(2-fluorophenyl)-1H-imidazole-5-carbonitrile (Example 156),
N-{cis-4-[5-chloro-4-(4-fluorophenyl)-1H-imidazol-1-yl]cyclohexyl}-2-fluoro-2-methylpropanamide (Example 183),
N-[cis-4-(5-cyano-4-phenyl-1H-imidazol-1-yl)cyclohexyl]-2-fluoro-2-methylpropanamide (Example 197),
N-(cis-4-{5-cyano-4-[4-(trifluoromethyl)phenyl]-1H-imidazol-1-yl}cyclohexyl)-2-fluoro-2-methylpropanamide (Example 201),
cis-4-{5-chloro-4-[4-(trifluoromethyl)phenyl]-1H-imidazol-1-yl}-N-(tetrahydro-2H-pyran-4-yl)cyclohexanecarboxamide (Example 224),
N- {cis-4-[4-(4-chlorophenyl)-1H-imidazol-1-yl]cyclohexyl}-2-fluoro-2-methylpropanamide (Example 280),
N-{cis-4-[4-(4-chloro-2-fluorophenyl)-1H-imidazol-1-yl]cyclohexyl}-2-fluoro-2-methylpropanamide (Example 283),
N-{cis-4-[4-(2,4-difluorophenyl)-1H-imidazol-1-yl]cyclohexyl}-2-fluoro-2-methylpropanamide (Example 300),
N-{cis-4-[5-cyano-4-(4-fluorophenyl)-1H-imidazol-1-yl]cyclohexyl}-2-fluoro-2-methylpropanamide (Example 332),
cis-4-[5-chloro-4-(3,4-difluorophenyl)-1H-imidazol-1-yl]-N-(tetrahydro-2H-pyran-4-yl)cyclohexanecarboxamide (Example 370), and
{(3-exo)-3-[5-chloro-4-(4-fluorophenyl)-1H-imidazol-1-yl]-8-azabicyclo[3.2.1]oct-8-yl}(4,4-difluorocyclohexyl)methanone (Example 452),
or a pharmaceutically acceptable salt thereof.

13. The compound of claim 1 selected from the following compounds:
N-cyclohexyl-4-[4-(3-fluorophenyl)-1H-imidazol-1-yl]piperidine-1-carboxamide (Example 1), 1-{1-[(4,4-difluorocyclohexyl)carbonyl]piperidin-4-yl}-4-phenyl-1H-imidazole-5-carbonitrile (Example 7),
N-cyclohexyl-4-[4-(2-fluorophenyl)-1H-imidazol-1-yl]piperidine-1-carboxamide (Example 66), 1-{1-[(4,4-difluorocyclohexyl)carbonyl]piperidin-4-yl}-4-(4-fluorophenyl)-1H-imidazole-5-carbonitrile (Example 154),
N-{cis-4-[5-chloro-4-(4-fluorophenyl)-1H-imidazol-1-yl]cyclohexyl}-2-fluoro-2-methylpropanamide (Example 183),
cis-4-[5-chloro-4-(3,4-difluorophenyl)-1H-imidazol-1-yl]-N-(tetrahydro-2H-pyran-4-yl)cyclohexanecarboxamide (Example 370), and
{(3-exo)-3-[5-chloro-4-(4-fluorophenyl)-1H-imidazol-1-yl]-8-azabicyclo[3.2.1]oct-8-yl}(4,4-difluorocyclohexyl)methanone (Example 452),
or a pharmaceutically acceptable salt thereof.

14. A pharmaceutical composition comprising the compound of any one of claims 1 to 13 or a pharmaceutically acceptable salt thereof.

15. A medicament for treating a disease due to an abnormality of the intracellular signaling mediated by acetylcholine, comprising as an active ingredient the compound of any one of claims 1 to 13 or a pharmaceutically acceptable salt thereof.

16. The medicament of claim 15 wherein the disease due to an abnormality of the intracellular signaling mediated by acetylcholine is CIAS (cognitive impairment associated with schizophrenia), Alzheimer's disease, Down's syndrome, cognitive disorder, mild cognitive disorder, memory disorder/learning disorder, attention deficit/hyperactivity disorder or cerebral angiopathy.

17. A drug comprising the combination use of the compound of any one of claims 1 to 13 or a pharmaceutically acceptable salt thereof and at least one agent selected from atypical antipsychotics.

18. A method for treating a disease due to an abnormality of the intracellular signaling mediated by acetylcholine, comprising administering a therapeutically effective amount of the compound of any one of claims 1 to 13 or a pharmaceutically acceptable salt thereof to a patient in need thereof.

19. Use of the compound of any one of claims 1 to 13 or a pharmaceutically acceptable salt thereof in the manufacture of a medicament for treating a disease due to an abnormality of the intracellular signaling mediated by acetylcholine.

20. A pharmaceutical composition comprising the compound of any one of claims 1 to 13 or a pharmaceutically acceptable salt thereof for use in the treatment of a disease due to an abnormality of the intracellular signaling mediated by acetylcholine.


SEQUENCE LISTING

<110>  Dainippon Sumitomo Pharma Co., Ltd.

<120>  IMIDAZOLE DERIVATIVES

<130>  671727

<150> JP 2012-219934
<151> 2012-10-02

<160>  1

<170>  PatentIn version 3.5

<210>  1
<211>  1514
<212>  DNA
<213>  homo sapience

<220>
<223>  Human alpha7 nAChR gene

<400>  1
ccaccatgcg ctgctcgccg ggaggcgtct ggctggcgct ggccgcgtcg ctcctgcacg      60

tgtccctgca aggcgagttc cagaggaagc tttacaagga gctggtcaag aactacaatc     120

ccttggagag gcccgtggcc aatgactcgc aaccactcac cgtctacttc tccctgagcc     180

tcctgcagat catggacgtg gatgagaaga accaagtttt aaccaccaac atttggctgc     240

aaatgtcttg gacagatcac tatttacagt ggaatgtgtc agaatatcca ggggtgaaga     300

ctgttcgttt cccagatggc cagatttgga aaccagacat tcttctctat aacagtgctg     360

atgagcgctt tgacgccaca ttccacacta acgtgttggt gaattcttct gggcattgcc     420

agtacctgcc tccaggcata ttcaagagtt cctgctacat cgatgtacgc tggtttccct     480

ttgatgtgca gcactgcaaa ctgaagtttg ggtcctggtc ttacggaggc tggtccttgg     540

atctgcagat gcaggaggca gatatcagtg gctatatccc caatggagaa tgggacctag     600

tgggaatccc cggcaagagg agtgaaaggt tctatgagtg ctgcaaagag ccctaccccg     660

atgtcacctt cacagtgacc atgcgccgca ggacgctcta ctatggcctc aacctgctga     720

tcccctgtgt gctcatctcc gccctcgccc tgctggtgtt cctgcttcct gcagattccg     780

gggagaagat ttccctgggg ataacagtct tactctctct taccgtcttc atgctgctcg     840

tggctgagat catgcccgca acatccgatt cggtaccatt gatagcccag tacttcgcca     900

gcaccatgat catcgtgggc ctctcggtgg tggtgacagt gatcgtgctg cagtaccacc     960

accacgaccc cgacgggggc aagatgccca agtggaccag agtcatcctt ctgaactggt    1020

gcgcgtggtt cctgcgaatg aagaggcccg gggaggacaa ggtgcgcccg gcctgccagc 1080

acaagcagcg gcgctgcagc ctggccagtg tggagatgag cgccgtggcg ccgccgcccg 1140

ccagcaacgg gaacctgctg tacatcggct tccgcggcct ggacggcgtg cactgtgtcc 1200

cgacccccga ctctggggta gtgtgtggcc gcatggcctg ctcccccacg cacgatgagc 1260

acctcctgca cggcgggcaa ccccccgagg gggacccgga cttggccaag atcctggagg 1320

aggtccgcta cattgccaac cgcttccgct gccaggacga aagcgaggcg gtctgcagcg 1380

agtggaagtt cgccgcctgt gtggtggacc gcctgtgcct catggccttc tcggtcttca 1440

ccatcatctg caccatcggc atcctgatgt cggctcccaa cttcgtggag gccgtgtcca 1500

aagactttgc gtaa 1514